# EUROPÄISCHE PATENTSCHRIFT

(11) **EP 0 842 927 B1**
(45) Veröffentlichungstag und Bekanntmachung des Hinweises auf die Patenterteilung: **25.02.2004**
(21) Anmeldenummer: 97119637.3
(22) Anmeldetag: 10.11.1997
(51) Int. Cl.: C07D 311/70, A61K 31/35, C07D 311/58, C07D 405/12

(54) **3-Amido-chromanylsulfonyl(thio)harnstoffe, Verfahren zu ihrer Herstellung, ihre Verwendung und sie enthaltende pharmazeutische Präparate**
3-Amido-chromanylsulfonyl(thio)ureas, process for their preparation, their use and medicaments containing them
3-Amido-chromanylsulfonyl(thio)urées, procédé pour leur préparation, leur utilisation et médicaments les contenant

(30) Priorität: 14.11.1996 DE 19647000
(43) Veröffentlichungstag der Anmeldung: 20.05.1998
(73) Patentinhaber: Aventis Pharma Deutschland GmbH, 65929 Frankfurt am Main (DE)
(72) Erfinder: Englert, Heinrich Christian, Dr., 65719 Hofheim (DE); Gerlach, Uwe, Dr., 65795 Hattersheim (DE); Mania, Dieter, Dr., 61462 Königstein (DE); Linz, Wolfgang, Dr., 55129 Mainz (DE); Gögelein, Heinz, Prof. Dr., 60528 Frankurt (DE); Klaus, Erik, Dr., 65779 Kelkheim (DE)

(56) Entgegenhaltungen:
- EP-A- 0 587 180
- EP-A- 0 612 724
- EP-A- 0 727 416
- EP-A- 0 779 288
- SMITS P ET AL: "CARDIOVASCULAR EFFECTS OF SULPHONYLUREA DERIVATIVES IMPLICATIONS FOR THE TREATMENT OF NIDDM?" DIABETOLOGIA, Bd. 38, Nr. 1, 1.Januar 1995, Seiten 116-121, XP000571858

## Beschreibung

3-Amido-chromanylsulfonyl(thio)harnstoffe, Verfahren zu ihrer Herstellung, ihre Verwendung und sie enthaltende pharmazeutische Präparate

Die vorliegende Erfindung betrifft 3-Amido-chromanylsulfonyl(thio)harnstoffe der Formel I, 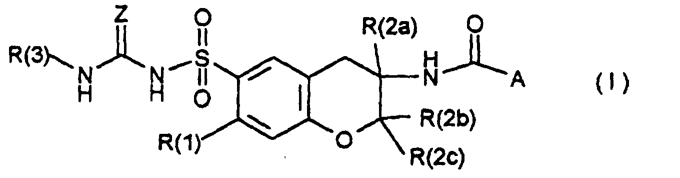 die wertvolle Arzneimittelwirkstoffe zur Behandlung von Störungen des Herz-Kreislaufsystems, insbesondere zur Behandlung von Arrhythmien, zur Verhinderung des plötzlichen Herztodes oder zur Beeinflussung einer verminderten Kontraktilität des Herzens sind, sowie Verfahren zu ihrer Herstellung, ihre Verwendung und sie enthaltende pharmazeutische Präparate.

Für bestimmte Benzolsulfonylharnstoffe ist eine blutzuckersenkende Wirkung beschrieben. Als Prototyp solcher blutzuckersenkenden Sulfonylharnstoffe gilt das Glibenclamid, das als Mittel zur Behandlung des Diabetes mellitus therapeutisch verwendet wird und in der Forschung als vielbeachtetes Werkzeug zur Erforschung sogenannter ATP-sensitiver Kaliumkanäle dient. Neben seiner blutzuckersenkenden Wirkung besitzt das Glibenclamid noch andere Wirkungen, die bislang therapeutisch noch nicht eingesetzt werden können, die aber allesamt auf die Blockade eben dieser ATP-sensitiven Kaliumkanäle zurückgeführt werden. Dazu gehört insbesondere eine antifibrillatorische Wirkung am Herzen. Bei der Behandlung des Kammerflimmerns oder seiner Vorstufen wäre jedoch eine gleichzeitige Blutzuckersenkung unerwünscht oder gar gefährlich, da sie den Zustand des Patienten weiter verschlechtern kann. Aus der EP-A-612 724 sind Benzolsulfonylharnstoffe bekannt, welche Wirkungen auf das Herz-Kreislauf-System haben. In der deutschen Patentanmeldung 19546736.1 bzw. der EP-A-779 288 werden Chromanylsulfonyl(thio)harnstoffe mit einer Wirkung auf das Herz-Kreislauf-System beschrieben, in denen eine Amidogruppe über eine Methylen- oder Ethylengruppe mit der 4-Position des Chromansystems verknüpft ist. Die Eigenschaften dieser Verbindungen sind jedoch in verschiedener Hinsicht noch nicht befriedigend, und es besteht weiterhin Bedarf an Verbindungen mit einem günstigen Eigenschaftsprofil, die insbesondere zur Behandlung arrhythmischer Störungen des Herzens und ihrer Folgen geeignet sind.

In der EP-A-325 964 werden Chroman-Verbindungen als α₂-adrenerge Antagonisten mit Wirkung gegen Depressionen, metabolische Störungen, Glaukom, Migräne und Bluthochdruck beschrieben. Es werden jedoch keine Verbindungen mit einer Substitution durch Sulfonylharnstoff- oder Sulfonylthioharnstoff-Gruppierungen beschrieben, und solche Verbindungen werden auch nicht nahegelegt.

Überraschend wurde nun gefunden, daß Chromanylsulfonyl(thio)harnstoffe der Formel I mit einer Amidogruppe in der 3-Position des Chromansystems eine ausgeprägte Wirkung auf das Herz-Kreislauf-System haben. Gegenstand der vorliegenden Erfindung sind somit Verbindungen der Formel I, 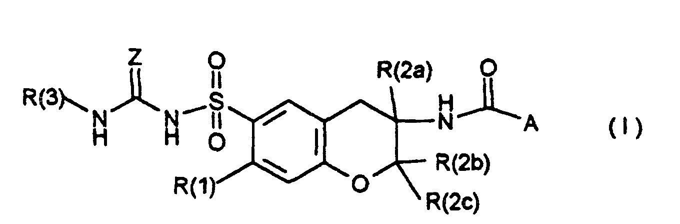 in der
- R(1): Wasserstoff, Alkyl mit 1, 2, 3 oder 4 C-Atomen, Alkoxy mit 1, 2, 3 oder 4 C-Atomen, Alkoxyalkoxy mit unabhängig voneinander 1,2,3 oder 4 C-Atomen in jeder der beiden Alkoxyeinheiten, Alkylmercapto mit 1, 2, 3 oder 4 C-Atomen, Fluor, Chlor, Brom, Iod oder Trifluormethyl bedeutet;
- R(2a), R(2b) und R(2c),: die gleich oder verschieden sind, Wasserstoff oder Alkyl mit 1 oder 2 C-Atomen bedeuten;
- R(3): Wasserstoff oder Alkyl mit 1, 2, 3 oder 4 C-Atomen bedeutet;
- Z: Schwefel oder Sauerstoff bedeutet;
- A: Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist,
oder
- A: den Rest eines gesättigten oder ungesättigten Lactams der Formel 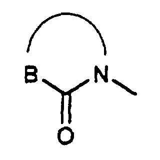 bedeutet, in der B Alkenylen oder Alkylen mit 3, 4, 5 oder 6 C-Atomen bedeutet, das unsubstituiert ist oder durch bis zu drei gleiche oder verschiedene Alkylgruppen mit 1, 2, 3 oder 4 C-Atomen substituiert ist,
oder
- A: den Rest eines bicyclischen Systems der Formeln 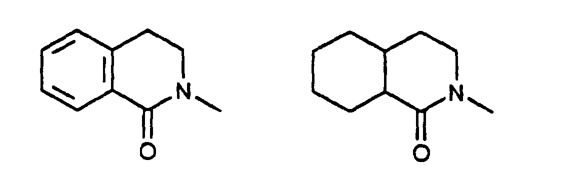 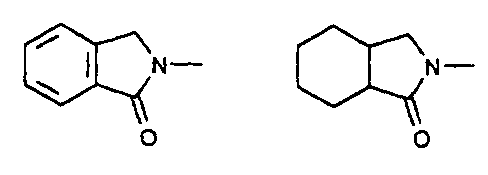 bedeutet:
in allen ihren stereoisomeren Formen und Gemische davon in allen Verhältnissen; sowie ihre physiologisch unbedenklichen Salze.

Der Begriff Alkyl bedeutet, sofern nicht anders angegeben, geradkettige oder verzweigte gesättigte Kohlenwasserstoffreste. Dies gilt auch für Alkylreste, die in einem Alkoxyrest enthalten sind, also in dem Rest Alkyl-O-, die in einem Alkoxyalkoxyrest enthalten sind, also in dem Rest Alkyl-O-alkyl-O-, oder die in einem Alkylmercaptorest enthalten sind, also in dem Rest Alkyl-S-. Beispiele für Alkylreste sind Methyl, Ethyl, n-Propyl, Isopropyl, n-Butyl, Isobutyl, sec-Butyl und tert-Butyl. Beispiele für Alkoxyreste sind Methoxy, Ethoxy, n-Propoxy, Isopropoxy, n-Butoxy, Isobutoxy, sec-Butoxy und tert-Butoxy. Beispiele für Alkoxyalkoxy sind Methoxymethoxy, Ethoxymethoxy, n-Butoxymethoxy, 2-Methoxyethoxy, 2-Ethoxyethoxy, 2-Isopropoxyethoxy, 2-(n-Propoxy)ethoxy, 2-(n-Butoxy)ethoxy, 2-Isobutoxyethoxy, 2-(tert-Butoxy)ethoxy, 3-Methoxypropoxy, 3-Ethoxypropoxy, 2-Methoxypropoxy, 2-Ethoxypropoxy, 4-Methoxybutoxy, 4-Ethoxybutoxy oder 3-Methoxybutoxy.

Beispiele für Alkylen- und Alkenylenreste, die für die Gruppe B stehen, sind 1,3-Propylen, 1,4-Butylen, 1,5-Pentylen, 1,6-Hexylen, 1,3-Prop-1-enylen, 1,3-Prop-2-enylen, 1,4-But-1-enylen, 1,4-But-2-enylen, 1,4-But-3-enylen, 1,5-Pent-1-enylen, 1,5-Pent-2-enylen, 1,5-Pent-3-enylen und 1,5-Pent-4-enylen.

In substituierten Phenylresten, die insbesondere einfach, zweifach oder dreifach substituiert sein können, können sich die Substituenten in beliebigen Positionen befinden, bei Monosubstitution z.B. in der ortho-, meta- oder para-Position, bei Disubstitution in 2,3-, 2,4-, 2,5-, 2,6-, 3,4-, 3,5-Stellung, bei Trisubstitution z. B. in 2,3,4-, 2,3,5-, 2,3,6-, 2,4,5-, 2,4,6- oder 3,4,5-Stellung.

Halogen bedeutet, sofern nicht anders angegeben, Fluor, Chlor, Brom und Iod, vorzugsweise Fluor und Chlor.

Verbindungen der Formel I können ein oder mehrere Chiralitätszentren besitzen, etwa an den C-Atomen 2 oder 3 des Chromansystems bei entsprechender Substitution, und können in stereoisomeren Formen vorliegen. Vorhandene chirale Zentren können unabhängig voneinander R- oder S-Konfiguration haben. Zur Erfindung gehören alle möglichen Stereoisomeren, z. B. Enantiomere oder Diastereomere, und Mischungen von zwei oder mehr Stereoisomeren in beliebigen Verhältnissen. Enantiomere z. B. sind in Form der reinen Enantiomeren, sowohl als links- als auch rechtsdrehende Antipoden, in Form von Racematen und in Form von Mischungen der beiden Enantiomeren in beliebigen Verhältnissen Gegenstand der Erfindung.

Die erfindungsgemäßen Verbindungen der Formel I enthalten bewegliche Wasserstoffatome und können in verschiedenen tautomeren Formen vorliegen. Auch alle diese Tautomeren sind Gegenstand der vorliegenden Erfindung.

Physiologisch verträgliche Salze der Verbindungen der Formel I sind insbesondere pharmazeutisch verwendbare Salze oder nicht toxische Salze. Solche Salze lassen sich beispielsweise aus Verbindungen der Formel I mit aciden Wasserstoffatomen und nicht toxischen anorganischen oder organischen Basen herstellen, beispielsweise geeigneten Alkali- oder Erdalkalimetallverbindungen, wie Natriumoder Kaliumhydroxid, oder Ammoniak oder organischen Aminoverbindungen oder Ammoniumhydroxiden. Umsetzungen von Verbindungen der Formel I mit Basen zur Herstellung der Salze werden im allgemeinen gemäß üblichen Vorgehensweisen in einem Lösungs- oder Verdünnungsmittel durchgeführt. Bevorzugt sind physiologisch unbedenkliche Salze der Verbindungen der Formel I, in denen als Kationen Alkali- und Erdalkalimetallionen wie Natrium-, Kalium-, Rubidium-, Magnesium- und Calciumionen, das unsubstituierte Ammoniumion oder Ammoniumionen mit einem oder mehreren organischen Resten vorliegen, sowie Additionsprodukte aus Verbindungen der Formel I und Aminosäuren, insbesondere basischen Aminosäuren wie z. B. Lysin oder Arginin. Eine Salzbildung an dem durch die Sulfonylgruppe substituierten Stickstoffatom der Harnstoffgruppe führt zu Verbindungen der Formel II, 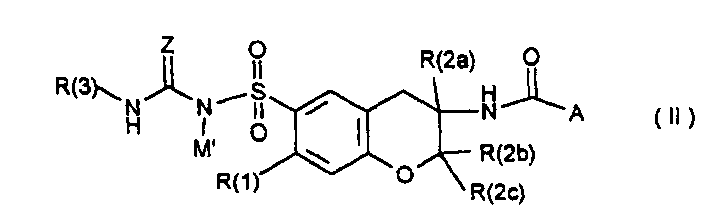 in der R(1), R(2a), R(2b), R(2c), R(3), A und Z die oben angegebenen Bedeutungen haben und das Kation M' beispielsweise für ein Alkalimetallion oder ein Äquivalent eines Erdalkalimetallions, z. B. das Natrium-, Kalium-, Rubidium-, Magnesium- oder Calciumion, für das unsubstituierte Ammoniumion oder für ein Ammoniumion mit einem oder mehreren organischen Resten steht, beispielsweise für das aus einer Aminosäure, insbesondere einer basischen Aminosäure wie z. B. Lysin oder Arginin, durch Protonierung erhaltene Kation.

R(1) bedeutet bevorzugt Wasserstoff, Alkyl mit 1 oder 2 C-Atomen, Alkoxy mit 1, 2, 3 oder 4 C-Atomen, Alkylmercapto mit 1, 2, 3 oder 4 C-Atomen, Fluor, Chlor, Brom, Iod oder Trifluormethyl, besonders bevorzugt Wasserstoff, Alkyl mit 1 oder 2 C-Atomen, Alkoxy mit 1 oder 2 C-Atomen, Alkylmercapto mit 1 oder 2 C-Atomen, Fluor, Chlor, Brom, Iod oder Triflourmethyl, ganz besonders bevorzugt Wasserstoff, Alkyl mit 1 oder 2 C-Atomen, Alkoxy mit 1 oder 2 C-Atomen oder Alkylmercapto mit 1 oder 2 C-Atomen.

R(2a) bedeutet bevorzugt Wasserstoff. R(2b) und R(2c) bedeuten bevorzugt unabhängig voneinander Wasserstoff oder Methyl, besonders bevorzugt Wasserstoff.

R(3) bedeutet bevorzugt Wasserstoff, Methyl oder Ethyl.

Bevorzugt sind Verbindungen der Formel I, in der
- R(1): Wasserstoff, Alkyl mit 1 oder 2 C-Atomen, Alkoxy mit 1, 2, 3 oder 4 C-Atomen, Alkylmercapto mit 1, 2, 3 oder 4 C-Atomen, Fluor, Chlor, Brom, Iod oder Trifluormethyl bedeutet;
- R(2a), R(2b) und R(2c),: die gleich oder verschieden sind, Wasserstoff oder Alkyl mit 1 oder 2 C-Atomen bedeuten;
- R(3): Wasserstoff oder Alkyl mit 1, 2, 3 oder 4 C-Atomen bedeutet;
- Z: Schwefel oder Sauerstoff bedeutet;
- A: Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist,
oder
- A: den Rest eines gesättigten oder ungesättigten Lactams der Formel 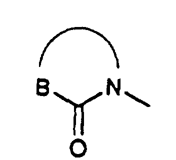 bedeutet, in der B Alkenylen oder Alkylen mit 3, 4, 5 oder 6 C-Atomen bedeutet, das unsubstituiert ist oder durch bis zu drei gleiche oder verschiedene Alkylgruppen mit 1, 2, 3 oder 4 C-Atomen substituiert ist,
oder
- A: den Rest eines bicyclischen Systems der Formeln 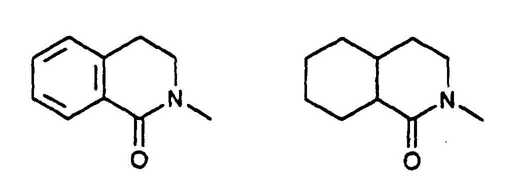 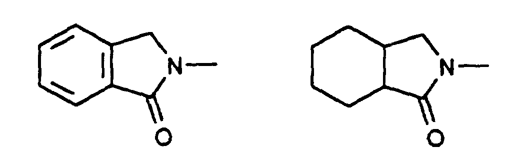 bedeutet.

Besonders bevorzugt sind Verbindungen der Formel I, in der
- R(1): Wasserstoff, Alkyl mit 1 oder 2 C-Atomen, Alkoxy mit 1 oder 2 C-Atomen, Alkylmercapto mit 1 oder 2 C-Atomen, Fluor, Chlor, Brom, Iod oder Trifluormethyl bedeutet;
- R(2a): Wasserstoff bedeutet und R(2b) und R(2c) Wasserstoff oder Methyl bedeuten;
- R(3): Wasserstoff oder Alkyl mit 1, 2, 3 oder 4 C-Atomen bedeutet;
- Z: Schwefel oder Sauerstoff bedeutet;
- A: Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist,
oder
- A: den Rest eines gesättigten oder ungesättigten Lactams der Formel 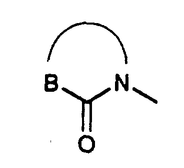 bedeutet, in der B Alkenylen oder Alkylen mit 3, 4, 5 oder 6 C-Atomen bedeutet, das unsubstituiert ist oder durch bis zu drei gleiche oder verschiedene Alkylgruppen mit 1, 2, 3 oder 4 C-Atomen substituiert ist,
oder
- A: den Rest eines bicyclischen Systems der Formeln 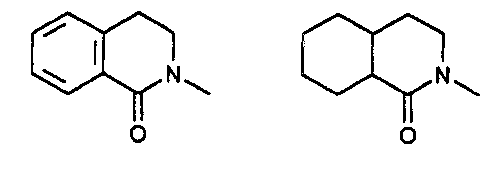 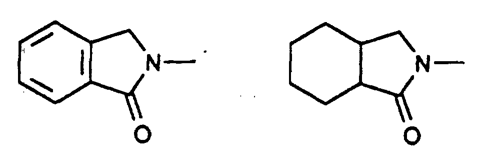 bedeutet.

Eine Reihe ganz besonders bevorzugter Verbindungen bilden solche Verbindungen der Formel I, in der
- R(1): Wasserstoff, Alkyl mit 1 oder 2 C-Atomen, Alkoxy mit 1 oder 2 C-Atomen oder Alkylmercapto mit 1 oder 2 C-Atomen bedeutet;
- R(2a), R(2b) und R(2c): Wasserstoff bedeuten;
- R(3): Wasserstoff oder Alkyl mit 1, 2, 3 oder 4 C-Atomen bedeutet;
- Z: Schwefel bedeutet;
- A: Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atome substituiert ist,
oder
- A: den Rest eines gesättigten oder ungesättigten Lactams der Formel 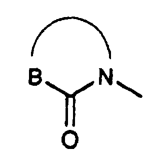 bedeutet, in der B Alkenylen oder Alkylen mit 3, 4, 5 oder 6 C-Atomen bedeutet, das unsubstituiert ist oder durch bis zu drei gleiche oder verschiedene Alkylgruppen mit 1, 2, 3 oder 4 C-Atomen substituiert ist,
oder
- A: den Rest eines bicyclischen Systems der Formeln 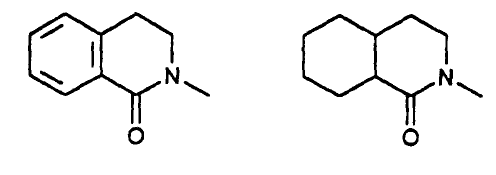 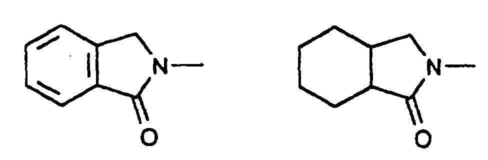 bedeutet.

In dieser Reihe sind darüber hinaus bevorzugt Verbindungen der Formel I, in der
- R(1): Wasserstoff, Alkyl mit 1 oder 2 C-Atomen, Alkoxy mit 1 oder 2 C-Atomen oder Alkylmercapto mit 1 oder 2 C-Atomen bedeutet;
- R(2a), R(2b) und R(2c): Wasserstoff bedeuten;
- R(3): Wasserstoff, Methyl oder Ethyl bedeutet;
- Z: Schwefel bedeutet;
- A: Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist,
oder
- A: den Rest eines gesättigten oder ungesättigten Lactams der Formel 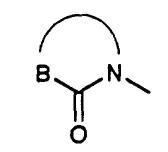 bedeutet, in der B Alkenylen oder Alkylen mit 3, 4, 5 oder 6 C-Atomen bedeutet, das unsubstituiert ist oder durch bis zu drei gleiche oder verschiedene Alkylgruppen mit 1, 2, 3 oder 4 C-Atomen substituiert ist.

In dieser Reihe sind speziell bevorzugt Verbindungen der Formel I, in der
- R(1): Wasserstoff, Alkyl mit 1 oder 2 C-Atomen, Alkoxy mit 1 oder 2 C Atomen oder Alkylmercapto mit 1 oder 2 C-Atomen bedeutet;
- R(2a), R(2b) und R(2c): Wasserstoff bedeuten;
- R(3): Wasserstoff, Methyl oder Ethyl bedeutet;
- Z: Schwefel bedeutet;
- A: Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist.

Eine weitere Reihe ganz besonders bevorzugter Verbindungen bilden solche Verbindungen der Formel I, in der
- R(1): Wasserstoff, Alkyl mit 1 oder 2 C-Atomen, Alkoxy mit 1 oder 2 C-Atomen oder Alkylmercapto mit 1 oder 2 C-Atomen bedeutet;
- R(2a), R(2b) und R(2c): Wasserstoff bedeuten;
- R(3): Wasserstoff oder Alkyl mit 1, 2, 3 oder 4 C-Atomen bedeutet;
- Z: Sauerstoff bedeutet;
- A: Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist,
oder
- A: den Rest eines gesättigten oder ungesättigten Lactams der Formel 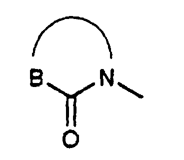 bedeutet, in der B Alkenylen oder Alkylen mit 3, 4, 5 oder 6 C-Atomen bedeutet, das unsubstituiert ist oder durch bis zu drei gleiche oder verschiedene Alkylgruppen mit 1, 2, 3 oder 4 C-Atomen substituiert ist,
oder
- A: den Rest eines bicyclischen Systems der Formeln 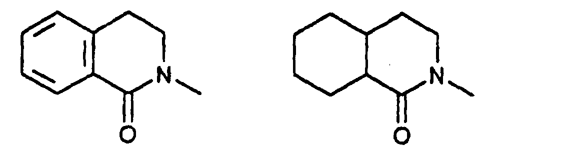 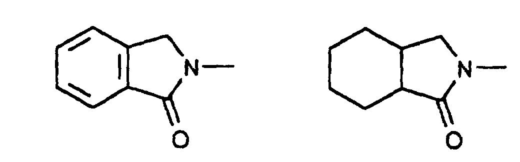 bedeutet.

In dieser weiteren Reihe sind darüber hinaus bevorzugt Verbindungen der Formel I, in der
- R(1): Wasserstoff, Alkyl mit 1 oder 2 C-Atomen, Alkoxy mit 1 oder 2 C-Atomen oder Alkylmercapto mit 1 oder 2 C-Atomen bedeutet;
- R(2a), R(2b) und R(2c): Wasserstoff bedeuten;
- R(3): Wasserstoff, Methyl oder Ethyl bedeutet;
- Z: Sauerstoff bedeutet;
- A: Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist,
oder
- A: den Rest eines gesättigten oder ungesättigten Lactams der Formel 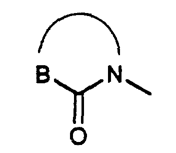 bedeutet, in der B Alkenylen oder Alkylen mit 3, 4, 5 oder 6 C-Atomen bedeutet, das unsubstituiert ist oder durch bis zu drei gleiche oder verschiedene Alkylgruppen mit 1, 2, 3 oder 4 C-Atomen substituiert ist.

In dieser weiteren Reihe sind speziell bevorzugt Verbindungen der Formel I, in der
- R(1): Wasserstoff, Alkyl mit 1 oder 2 C-Atomen, Alkoxy mit 1 oder 2 C-Atomen oder Alkylmercapto mit 1 oder 2 C-Atomen bedeutet;
- R(2a), R(2b) und R(2c): Wasserstoff bedeuten;
- R(3): Wasserstoff, Methyl oder Ethyl bedeutet;
- Z: Sauerstoff bedeutet;
- A: Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist.

Auch von allen bevorzugten Verbindungen der Formel I sind alle ihre stereoisomeren Formen und Gemische davon in allen Verhältnissen sowie ihre physiologisch unbedenklichen Salze Gegenstand der vorliegenden Erfindung.

Die Erfindung betrifft weiterhin Verfahren zur Herstellung der Verbindungen der Formel I, die durch die im folgenden wiedergegebenen Reaktionsschritte gekennzeichnet sind.
(a) 3-Amido-chromanylsulfonyl(thio)harnstoffe der Formel I, in der R(3) eine andere Bedeutung als Wasserstoff hat, können hergestellt werden, indem man Chromanylsulfonamide der Formel III 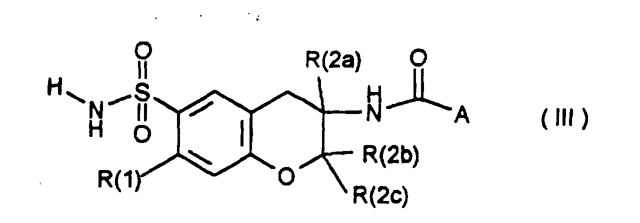 oder deren Salze der Formel IV 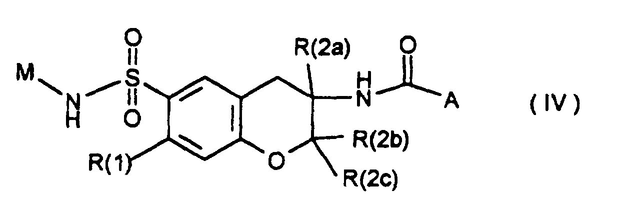 mit R(3)-substituierten Isocyanaten der Formel V bzw. R(3)-substituierten Isothiocyanaten der Formel VI

   R(3)-N=C=O (V) R(3)-N=C=S (VI)

   zu substituierten Chromanylsulfonylharnstoffen der Formel Ia 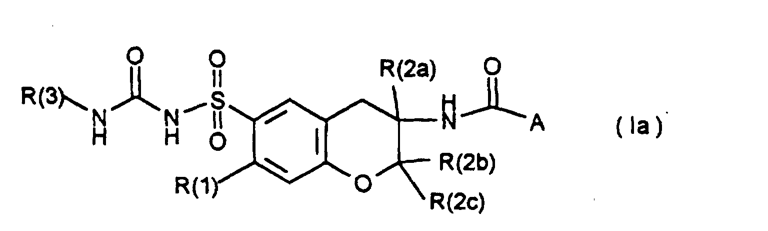 bzw. substituierten Chromanylsulfonylthioharnstoffen der Formel Ib 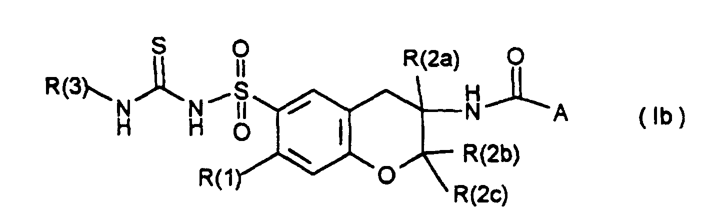 umsetzt. Die Reste R(1), R(2a), R(2b), R(2c) und A in den Formeln Ia, Ib, III und IV haben dabei die eingangs angegebenen Bedeutungen, R(3) steht hier in den Formeln Ia und Ib und in den Formeln V und VI für Alkyl mit 1, 2, 3 oder 4 C-Atomen. Als Kationen M in den Salzen der Formel IV kommen beispielsweise Alkalimetall- oder Erdalkalimetallionen, z. B. das Natrium- oder das Kaliumion, oder geeignete, nicht in unerwünschter Weise mit den Reaktionspartnern reagierende Ammoniumionen, insbesondere z. B. Tetraalkylammoniumionen oder Trialkylbenzylammoniumionen, in Betracht.
   Äquivalent zu den R(3)-substituierten Isocyanaten der Formel V kann man R(3)-substituierte Carbamidsäureester, R(3)-substituierte Carbamidsäurehalogenide oder R(3)-substituierte Harnstoffe einsetzen.
(b) Chromanylsulfonylharnstoffe der Formel Ia lassen sich aus Chromanylsulfonamiden der Formel III oder deren Salzen der Formel IV mit R(3)-substituierten Trichloracetamiden der Formel VII, 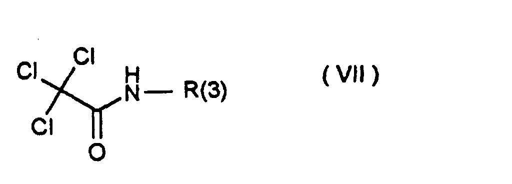 in der R(3) für Alkyl mit 1, 2, 3 oder 4 C-Atomen steht, in Gegenwart einer Base in einem inerten Lösungsmittel nach Synthesis 1987, 734-735 bei Temperaturen von 25 bis 150 °C herstellen.
   Als Basen eignen sich zum Beispiel Alkalimetall- oder Erdalkalimetallhydroxide, -hydride, -amide oder -alkoholate, wie Natriumhydroxid, Kaliumhydroxid, Calciumhydroxid, Natriumhydrid, Kaliumhydrid, Calciumhydrid, Natriumamid, Kaliumamid, Natriummethylat, Natriumethylat, Kaliummethylat oder Kaliumethylat. Als inerte Lösungsmittel eignen sich Ether wie Tetrahydrofuran (THF), Dioxan, Ethylenglykoldimethylether oder Diglyme, Ketone wie Aceton oder Butanon, Nitrile wie Acetonitril, Nitroverbindungen wie Nitromethan, Ester wie Ethylacetat, Amide wie Dimethylformamid (DMF) oder N-Methylpyrrolidon (NMP), Hexamethylphosphorsäuretriamid, Sulfoxide wie Dimethylsulfoxid (DMSO), Sulfone wie Sulfolan, Kohlenwasserstoffe wie Benzol, Toluol, Xylole. Weiterhin eignen sich auch Gemische dieser Lösungsmittel untereinander.
(c) Chromanylsulfonyl(thio)harnstoffe der Formel I, in der R(3) für Wasserstoff steht, kann man durch Umsetzung von Chromanylsulfonamiden der Formel III oder von deren Salzen der Formel IV mit Trialkylsilyliso(thio)cyanaten, z. B. Trimethylsilyliso(thio)cyanat, oder mit Siliciumtetraiso(thio)cyanat und Spaltung (z.B. Hydrolyse) der primären Silicium-substituierten Chromanylsulfonyl(thio)harnstoffe herstellen. Mit Trialkylsilylisocyanaten oder mit Siliciumtetraisocyanat gelangt man so zu Verbindungen der Formel Ic, 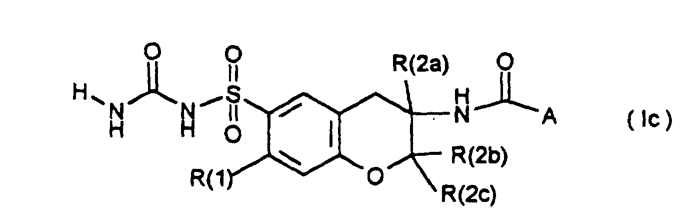 mit Trialkylsilylisothiocyanaten oder mit Siliciumtetraisothiocyanat zu Verbindungen der Formel Id, 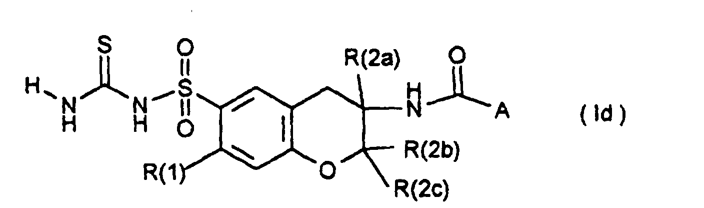 wobei in den Formeln Ic und Id die Reste R(1), R(2a), R(2b), R(2c) und A die eingangs angegebenen Bedeutungen haben.
   Weiterhin ist es möglich, Chromanylsulfonamide der Formel III oder deren Salze der Formel IV durch Umsetzung mit Halogencyanen und Hydrolyse der primär entstehenden N-Cyanosulfonamide mit Mineralsäuren bei Temperaturen von 0 bis 100 °C in Chromanylsulfonylharnstoffe der Formel Ic umzuwandeln.
   Chromanylsulfonylthioharnstoffe der Formel Id lassen sich auch durch Umsetzung von Chromanylsulfonamiden der Formel III oder deren Salzen der Formel IV mit Benzoylisothiocyanat und Umsetzung der intermediären benzoylsubstituierten Chromanylsulfonylthioharnstoffe mit einer wäßrigen Mineralsäure erhalten. Ähnliche Verfahren sind in J. Med. Chem. 35 (1992), 1137-1144 beschrieben. Eine weitere Variante zur Herstellung der Verbindungen der Formel Id besteht darin, die vorstehend erwähnten N-Cyanosulfonamide mit Schwefelwasserstoff umzusetzen.
(d) Chromanylsulfonylharnstoffe der Formel I, in der Z für Sauerstoff steht, können aus Chromanylsulfonylhalogeniden z. B. der Formel VIII, 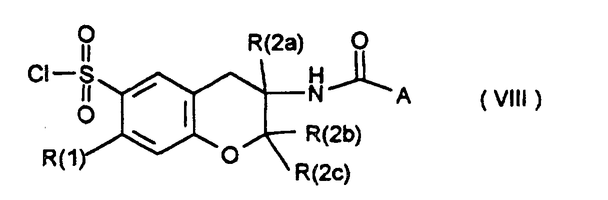 in der R(1), R(2a), R(2b), R(2c) und A die eingangs angegebenen Bedeutungen haben, mit R(3)-substituierten Harnstoffen oder R(3)-substituierten Bis(trialkylsilyl)harnstoffen hergestellt werden. Weiterhin kann man Sulfonsäurechloride der Formel VIII mit Parabansäure zu Chromanylsulfonylparabansäuren umsetzen, deren Hydrolyse mit Mineralsäuren entsprechende Chromanylsulfonylharnstoffe der Formel I liefert, in der Z für Sauerstoff steht.
(e) Chromanylsulfonyl(thio)harnstoffe der Formel I lassen sich auch durch Umsetzung von Aminen der Formel R(3)-NH₂, in der R(3) die eingangs angegebenen Bedeutungen hat, mit Chromanylsulfonylisocyanaten der Formel IX 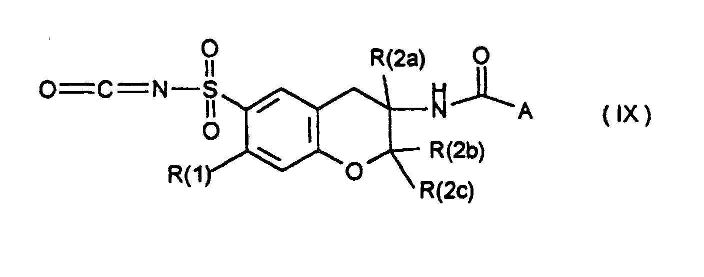 bzw. Chromanylsulfonylisothiocyanaten der Formel X 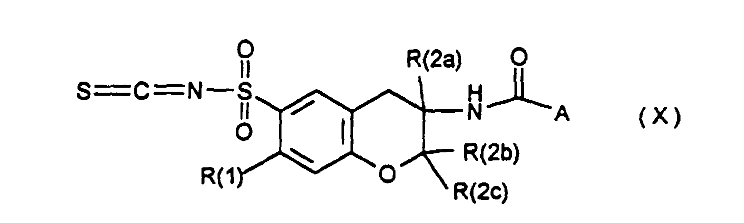 herstellen, wobei R(1 ), R(2a), R(2b), R(2c) und A in den Formeln IX und X die eingangs angegebenen Bedeutungen haben. Ebenso wie mit den Iso(thio)cyanaten der Formeln IX und X kann ein Amin der Formel R(3)-NH₂ mit einem Chromanylsulfonylcarbamidsäureester, einem -carbamidsäurehalogenid oder einem Chromanylsulfonylharnstoff der Formel Ia, in der R(3) hier für Wasserstoff steht, zu einer Verbindung der Formel I, in der Z für Sauerstoff steht, umgesetzt werden. Desgleichen kann ein Amin der Formel R(3)-NH₂ mit einem Chromanylsulfonylcarbamidsäurethioester oder einem -carbamidsäurethiohalogenid zu einer Verbindung der Formel I umgesetzt werden, in Z für Schwefel steht.
   Die Sulfonylisocyanate der Formel IX lassen sich aus den Sulfamoylchromanen der Formel III nach üblichen Methoden, beispielsweise mit Phosgen, erhalten. Die Herstellung der Sulfonylisothiocyanate der Formel X kann durch Umsetzung eines entsprechenden Sulfonsäureamids der Formel III mit Alkalihydroxid und Schwefelkohlenstoff in einem organischen Lösungsmittel, wie DMF, DMSO, N-Methylpyrrolidon, erfolgen. Das dabei erhaltene Di-alkalimetallsalz der Sulfonyldithiocarbaminsäure kann in einem inertem Lösungsmittel mit einem leichten Überschuß von Phosgen bzw. von einem Phosgenersatzstoff wie Triphosgen, mit einem Chlorameisensäureester (2 Äquivalente) oder mit Thionylchlorid umgesetzt werden. Die so erhaltene Lösung des Sulfonylisothiocyanats kann direkt mit den entsprechenden Aminen oder Ammoniak umgesetzt werden.
(f) Substituierte Chromanylsulfonylharnstoffe der Formel I, in der Z für Sauerstoff steht, kann man durch eine Umwandlungsreaktion aus Chromanylsulfonylthioharnstoffen der Formel I, in der Z für Schwefel steht, herstellen. Die Entschwefelung, also der Ersatz des Schwefelatoms im entsprechend substituierten Chromanylsulfonylthioharnstoff durch ein Sauerstoffatom, kann beispielsweise mit Hilfe von Oxiden oder Salzen von Schwermetallen oder auch durch Anwendung von Oxidationsmitteln wie Wasserstoffperoxid, Natriumperoxid oder salpetriger Säure, ausgeführt werden. Ein Thioharnstoff kann auch durch Behandlung mit Chlorierungsmitteln wie Phosgen oder Phosphorpentachlorid entschwefelt werden. Als Zwischenverbindungen werden Chlorameisensäureamidine bzw. Carbodiimide erhalten, die zum Beispiel durch Verseifen oder Anlagerung von Wasser in die entsprechenden substituierten Chromanylsulfonylharnstoffe überführt werden.
(g) Entsprechend substituierte Chromanylsulfenyl- oder -sulfinylharnstoffe lassen sich mit einem Oxidationsmittel, wie Wasserstoffperoxid, Natriumperoxid oder salpetriger Säure, zu Chromanylsulfonylharnstoffen der Formel I, in der Z für Sauerstoff steht, oxidieren.

Die Ausgangsverbindungen für die erwähnten Syntheseverfahren der Chromanylsulfonyl(thio)harnstoffe der Formel I können nach an sich bekannten Methoden hergestellt werden, wie sie in der Literatur (zum Beispiel in den Standardwerken wie Houben-Weyl, Methoden der Organischen Chemie, Georg Thieme Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier aber nicht näher erwähnten Varianten Gebrauch machen. Die Ausgangsstoffe können gewünschtenfalls auch in situ gebildet werden, derart, daß man sie aus dem Reaktionsgemisch nicht isoliert, sondern sofort weiter umsetzt.

3-Amido-chromane der Formel XVI können beispielsweise nach dem im Schema I wiedergegebenen Syntheseverfahren hergestellt werden, in dem die Substituenten die eingangs angegebenen bzw. nachfolgend erläuterten Bedeutungen haben.

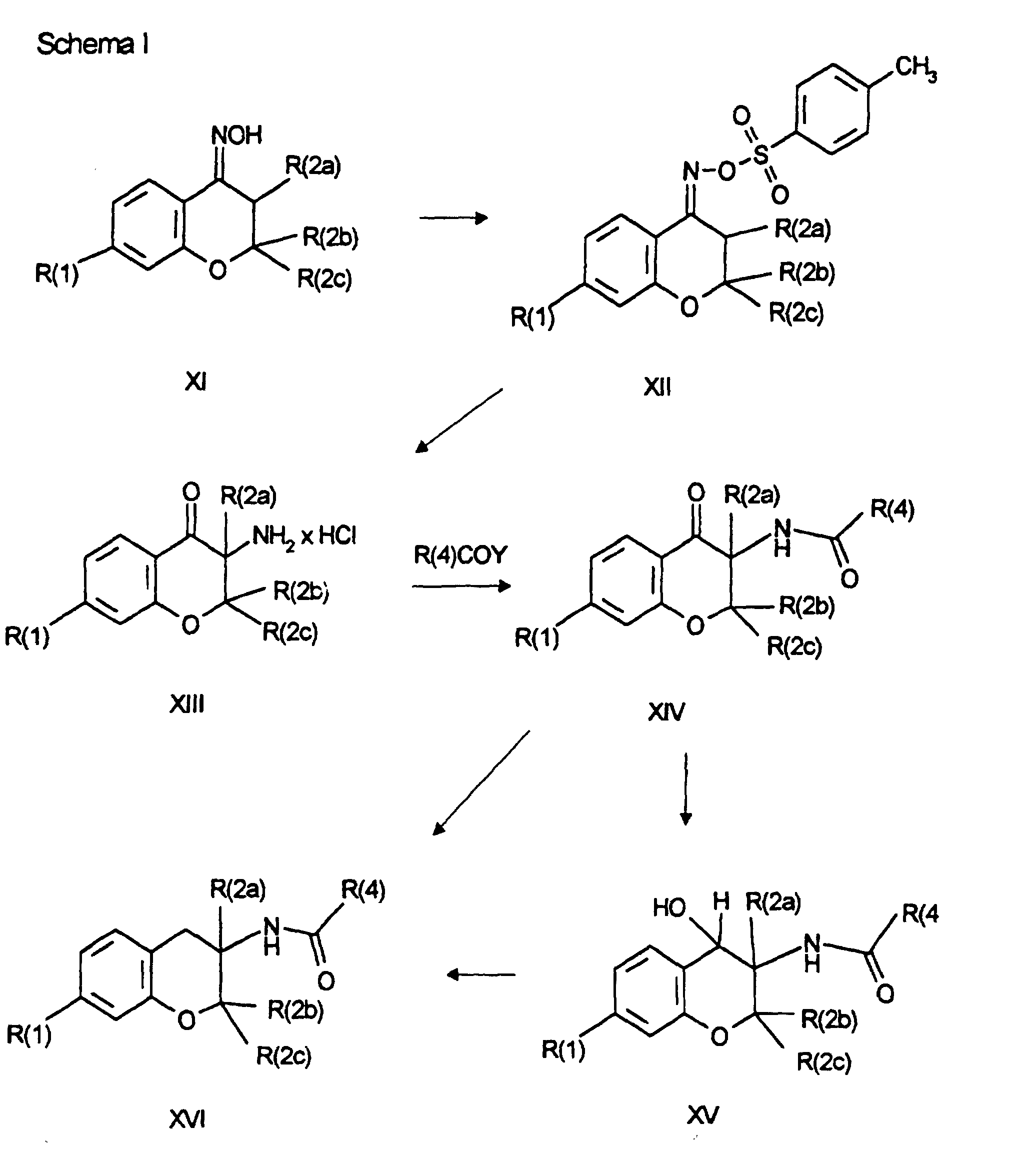

R(4) bedeutet Phenyl, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist, oder Alkyl mit 1 bis 4 C-Atomen oder Trihalogenmethyl

Die literaturbekannten Oxime der Formel XI, die beispielsweise in Heterocycles 38 (1994), 305-318 beschrieben sind, können mit Sulfonsäurechloriden, z. B. p-Toluolsulfonsäurechlorid, unter Zusatz von tertiären Basen, wie zum Beispiel Pyridin oder einem Trialkylamin, in Gegenwart oder in Abwesenheit eines inerten Lösungsmittels bei Temperaturen von 0 bis 100 °C, vorzugsweise 0 bis 10 °C, zu Oximsulfonaten umgesetzt werden, z. B. zu den Oximtosylaten der Formel XII. Als inerte Lösungsmittel eignen sich hierbei z. B. Ether wie Tetrahydrofuran, Dioxan, Glykolether, Ketone wie Aceton oder Butanon, Nitrile wie Acetonitril, Amide wie Dimethylformamid oder N-Methylpyrrolidon, Hexamethylphosphorsäuretriamid, Sulfoxide wie DMSO, chlorierte Kohlenwasserstoffe wie Dichlormethan, Chloroform, Trichlorethylen, 1,2-Dichlorethan oder Kohlenstofftetrachlorid, Kohlenwasserstoffe wie Benzol, Toluol, Xylole. Weiterhin eignen sich auch Gemische dieser

Lösungsmittel untereinander. Die Oximsulfonate z. B. der Formel XII lassen sich durch Einwirkung von Basen in einem Lösungsmittel in Aminoketone umlagern, d. h. in die 3-Amino-4-chromanone. Diese werden im allgemeinen in Form von Säureadditionssalzen isoliert, z. B. in Form der Hydrochloride der Formel XIII (J. Med. Chem. 12 (1969), 277). Geeignete Basen für diese Umlagerung sind z. B. die Alkalimetallsalze der Alkohole wie zum Beispiel Natriummethylat, Natriumethylat, Natriumisopropylat, Kaliummethylat, Kaliumethylat oder Kalium-tert-butylat, aber auch tertiäre Amin-Basen wie Pyridin oder Trialkylamine. Als Lösungsmitteln kommen zum Beispiel Alkohole wie Methanol, Ethanol, Isopropanol, tert-Butanol, Ether wie Tetrahydrofuran, Dioxan, Kohlenwasserstoffe wie Benzol, Toluol, Xylol, in Betracht. Die Umlagerung wird im allgemeinen bei Temperaturen von 10 bis 100 °C, vorzugsweise bei 20 bis 60 °C durchgeführt.

Die Aminoketone lassen sich nach Überführung der Säureadditionssalze, also z. B. der Hydrochloride der Formel XIII, mit Basen in die freien Amine zu den Amiden der Formel XIV acylieren, in denen R(4) für den in der eingangs angegebenen Definition von A bezeichneten Phenylrest stehen kann und die Gruppe R(4)-C(=O) dann im Molekül verbleiben kann, oder in denen die Gruppe R(4)-C(=O) die Funktion einer Schutzgruppe hat, die im weiteren Verlauf der Synthese wieder abgespalten wird.

Liegt der letztere Fall vor, so steht R(4) beispielsweise für Alkyl mit 1 bis 4 C-Atomen oder Trihalogenmethyl, z. B. Trifluormethyl. Als acylierende Mittel für die Aminogruppen eignen sich dabei z. B. die Alkylester, die Halogenide (wie z.B. Chloride oder Bromide) oder die Anhydride von Carbonsäuren. Insbesondere kann die Acylierung mit Verbindungen der Formel R(4)-C(=O)-Y durchgeführt werden, in der - ebenso wie in Schema I angegeben - der Rest R(4) z. B. für einen Alkylrest mit 1 bis 4 C-Atomen oder einen Trihalogenmethylrest steht oder für Phenyl steht, das unsubstituiert ist oder durch bis zu drei gleiche oder verschiedene Substituenten, ausgewählt aus der Gruppe bestehend aus Halogen, Alkyl mit 1 und 2 C-Atomen und Alkoxy mit 1 oder 2 C-Atomen, substituiert ist, und Y für eine Fluchtgruppe steht wie z. B. Halogen, (C₁-C₄)-Alkoxy, Trihalogenacetoxy oder (C₁-C₄)-Alkylcarbonyloxy.

Die Synthesen der Verbindungen der Formel XIV können unter Zusatz von tertiären Basen wie zum Beispiel Pyridin oder Trialkylaminen und in Gegenwart oder Abwesenheit eines inerten Lösungsmittels durchgeführt werden, wobei auch ein Katalysator wie zum Beispiel Dimethylaminopyridin zugegen sein kann. Die Umsetzung wird im allgemeinen bei Temperaturen von etwa 0 bis 160 °C, vorzugsweise von 20 bis 100 °C, durchgeführt. Als inerte Lösungsmittel eignen sich z. B. Ether wie Tetrahydrofuran, Dioxan, Glykolether wie Ethylenglykolmonomethyloder Ethylenglykolmonoethylether (Methylglykol oder Ethylglykol), Ethylenglykoldimethylether oder Diglyme, Ketone wie Aceton oder Butanon, Nitrile wie Acetonitril, Amide wie Dimethylformamid oder N-Methylpyrrolidon, Hexamethylphosphorsäuretriamid, Sulfoxide wie DMSO, chlorierte Kohlenwasserstoffe wie Dichlormethan, Chloroform, Trichlorethylen, 1,2-Dichlorethan oder Kohlenstofftetrachlorid, Kohlenwasserstoffe wie Benzol, Toluol, Xylole. Weiterhin eignen sich auch Gemische dieser Lösungsmittel untereinander.

Die Chromanone der Formel XIV lassen sich nach an sich bekannten Methoden zu den entsprechenden Chromanolen der Formel XV reduzieren, beispielsweise mit Alkaliborhydriden wie Natrium- oder Kaliumborhydrid in Alkoholen wie Methanol oder Ethanol (Bull. Soc. Chim. Fr. 1972, 3183).

Die Chromanone der Formel XIV wie auch die Chromanole der Formel XV lassen sich z. B. durch katalytische Hydrierung zu den Amidochromanen der Formel XVI reduzieren. Geeignete Katalysatoren für diese Hydrierung sind z. B. Metalle wie Pt, Pd, Rh, Ru oder Raneynickel, wobei die vier erstgenannten auch als Metalloxide vorliegen können. Bevorzugt sind Pd, Pt und Raneynickel. Als Lösungsmittel für die Hydrierung eignen sich z. B. Alkohole wie Methanol, Ethanol, Propanol, Ether wie Dioxan, Tetrahydrofuran, oder Säuren, wobei Essigsäure bevorzugt ist. Zur Reaktionsbeschleunigung kann bei der Hydrierung eine katalytische Menge einer starken Säure wie konz. Schwefelsäure, Salzsäure, Perchlorsäure oder Trifluoressigsäure zugesetzt werden. Es wird im allgemeinen bei 10 bis 50 °C, vorzugsweise bei 15 bis 30 °C, und bei 0 bis 100 atü, vorzugsweise bei 0 bis 5 atü, Wasserstoffdruck (d.h. bei einem Wasserstoffüberdruck von 0 bis 100 atm, vorzugsweise 0 bis 5 atm) hydriert (J. Med. Chem. 15 (1972), 863-865). Wird Essigsäure als Lösungsmittel verwendet, so lassen sich die Ausbeuten durch Zusatz von Anhydriden von (C₁-C₄)-Alkylcarbonsäuren wie z.B. Essigsäureanhydrid erhöhen. Die Chromanole der Formel XV lassen sich auch durch weitere Reduktionsmethoden, wie sie z. B. in Larock, Comprehensive Organic Transformations, VCH, 1989, Seite 27 - 28, beschrieben sind, in die Amidochromane der Formel XVI überführen.

Die folgenden Schritte in der Synthese der Verbindungen der Formel I sind im Schema II dargestellt. 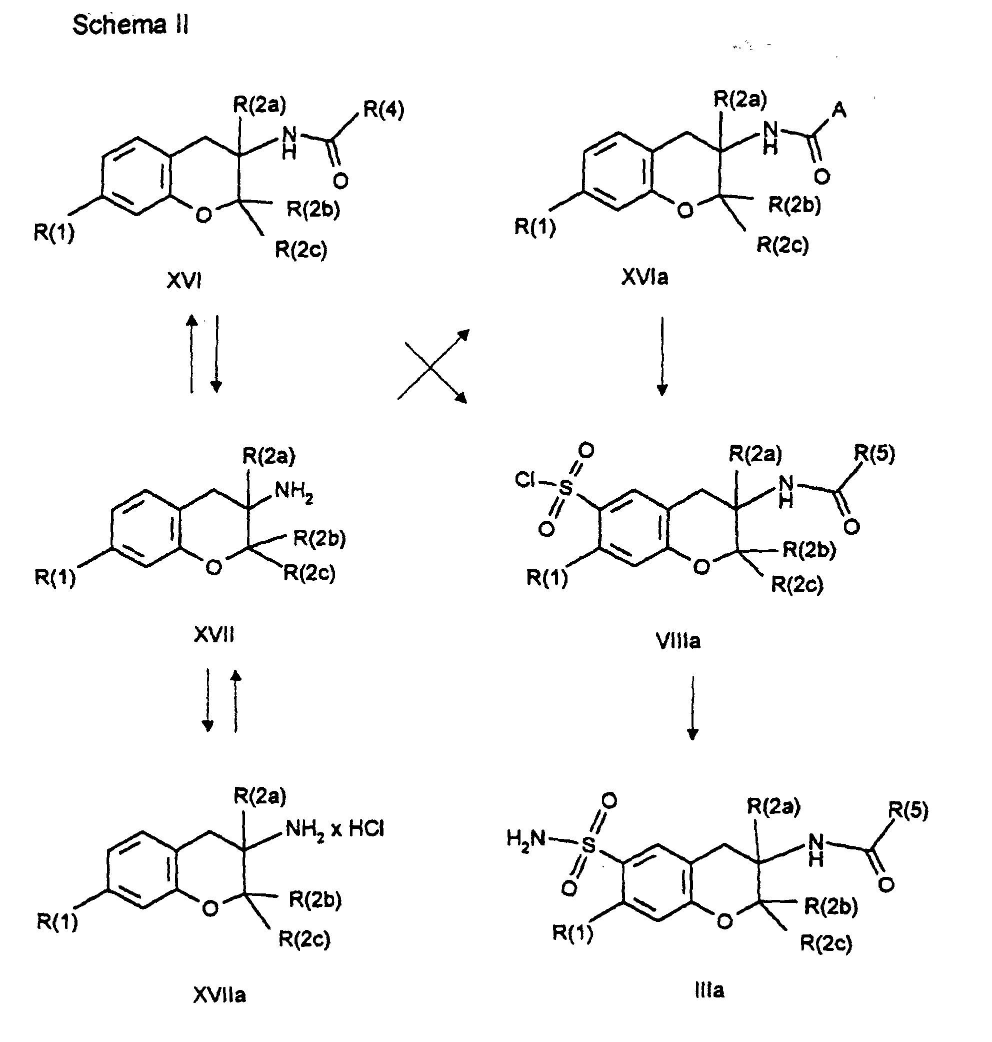 R(4) bedeutet Phenyl, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist, oder Alkyl mit 1 bis 4 C-Atomen, oder Trihalogenmethyl,
R(5) steht für alle eingangs angegebenen Bedeutungen von A oder für Alkyl mit 1 bis 4 C-Atomen oder Trihalogenmethyl

Fungiert die Acylgruppe R(4)-C(=O) in den Verbindungen der Formel XVI als Schutzgruppe, so läßt sich diese durch Basen oder Säuren wieder abspalten, wobei die Aminochromane der Formel XVII entstehen. Durch Spaltung mit Säuren, z. B. mit wäßrigen Säuren oder mit Säuren in inerten organischen Lösungsmitteln, kann dabei das zugehörige Säureadditionssalz, z.B. das Hydrochlorid der Formel XVIIa, entstehen. Für die Spaltung eignen sich z. B. Schwefelsäure, Halogenwasserstoffsäuren, wie Chlorwasserstoffsäure oder Bromwasserstoffsäure, Phosphorsäuren wie Orthophosphorsäure oder Polyphosphorsäure, oder andere übliche Säuren, mit denen Amide gespalten werden können, z. B. organische Carbon-, Sulfon- oder Schwefelsäuren, wie zum Beispiel Essigsäure, Salicyclsäure, Methan- oder Ethansulfonsäure, Benzolsulfonsäure, p-Toluolsulfonsäure, Laurylschwefelsäure. Die Spaltung des acylierten Amins der Formel XVI mit Basen kann ebenfalls in wäßrigen oder inerten organischen Lösungsmitteln erfolgen. Als Basen eignen sich z. B. Alkalimetall- oder Erdalkalimetallhydroxide oder auch - alkoholate wie Natriumhydroxid, Kaliumhydroxid, Calciumhydroxid, Natriummethylat, Natriumethylat, Kaliummethylat oder Kaliumethylat.

Die Amine der Formel XVII weisen an den Ring-C-Atomen ein oder zwei chirale Zentren auf. Wenn sie als Gemische von stereoisomeren Formen vorliegen, z. B. als Racemate, und wenn stereochemisch einheitliche Verbindungen der Formel I hergestellt werden sollen, dann kann beispielsweise auf der Stufe der Amine der Formel XVII eine Trennung der Stereoisomeren erfolgen. Weisen die Amine der Formel XVII zwei oder mehr chirale Zentren auf, dann können sie bei der Synthese als Gemische von Racematen anfallen, aus denen man einzelne Racemate beispielsweise durch Umkristallisieren aus inerten Lösungsmitteln in reiner Form isolieren kann. Erhaltene Racemate können, falls erwünscht, nach an sich bekannten Methoden mechanisch oder chemisch in ihre Enantiomeren getrennt werden. So können aus dem Racemat durch Umsetzung mit einem optisch aktiven Trennmittel Diastereomere gebildet werden. Als Trennmittel für basische Verbindungen eignen sich zum Beispiel optisch aktive Säuren, wie die R- bzw. R,Rund S- bzw. S,S-Formen von Weinsäure, Dibenzoylweinsäure, Diacetylweinsäure, Camphersulfonsäure, Mandelsäure, Äpfelsäure oder Milchsäure. Die verschiedenen Formen der Diastereomeren können in an sich bekannter Weise, zum Beispiel durch fraktionierte Kristallisation, getrennt werden, und die Enantiomeren können in an sich bekannter Weise aus den Diastereomeren in Freiheit gesetzt werden. Enantiomerentrennungen gelingen ferner durch Chromatographie an optisch aktiven Trägermaterialien. Ein besonders einfaches Verfahren zur Herstellung von optisch einheitlichen Verbindungen besteht bei entsprechender Substitution beispielsweise darin, daß man die Amine der Formel XVII durch Kristallisation bzw. Umkristallisation der Salze mit optisch aktiven Säuren wie beispielsweise (+)- oder (-)-Mandelsäure in die Enantiomeren spaltet und diese in die Endverbindungen der Formel I umwandelt, die dann ihrerseits enantiomerenrein sind. Zur Herstellung von stereochemisch einheitlichen Verbindungen der Formel I, z. B. reinen Enantiomeren, können Auftrennungen nach den genannten oder anderen üblichen Methoden aber auch auf anderen Stufen der Synthese erfolgen.

Die Verbindungen der Formel XVII, d. h. auch stereochemisch einheitliche Formen, lassen sich zu den Amiden der Formeln XVIa bzw. XVI acylieren. Als acylierende Mittel für die Einführung der Gruppe A-C(=O), in der A für Phenyl steht, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist, und die im Molekül verbleiben kann, eignen sich dabei z. B. die Alkylester, die Halogenide (wie z.B. Chloride oder Bromide) oder die Anhydride von Benzoesäuren. Insbesondere kann die Acylierung mit Verbindungen der Formel A-C(=O)-Y durchgeführt werden, in der A für Phenyl steht, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist, und Y für eine Fluchtgruppe steht wie z. B. Halogen, (C₁-C₄)-Alkoxy, Trihalogenacetoxy oder (C₁-C₄)-Alkylcarbonyloxy. Diese Acylierung kann wiederum unter Zusatz von tertiären Basen wie zum Beispiel Pyridin oder Trialkylaminen und in Gegenwart oder Abwesenheit eines inerten Lösungsmittels durchgeführt werden, wobei auch ein Katalysator wie zum Beispiel Dimethylaminopyridin zugegen sein kann. Die Umsetzung wird im allgemeinen bei Temperaturen von etwa 0 bis 160 °C, vorzugsweise von 20 bis 100 °C, durchgeführt. Als inerte Lösungsmittel eignen sich z. B. Ether wie Tetrahydrofuran, Dioxan, Glykolether wie Ethylenglykolmonomethyloder Ethylenglykolmonoethylether (Methylglykol oder Ethylglykol), Ethylenglykoldimethylether oder Diglyme, Ketone wie Aceton oder Butanon, Nitrile wie Acetonitril, Amide wie Dimethylformamid oder N-Methylpyrrolidon, Hexamethylphosphorsäuretriamid, Sulfoxide wie DMSO, chlorierte Kohlenwasserstoffe wie Dichlormethan, Chloroform, Trichlorethylen, 1,2-Dichlorethan oder Kohlenstofftetrachlorid, Kohlenwasserstoffe wie Benzol, Toluol, Xylole. Weiterhin eignen sich auch Gemische dieser Lösungsmittel untereinander.

Die Herstellung von solchen Verbindungen der Formel XVIa, in der A für einen Rest der Formel 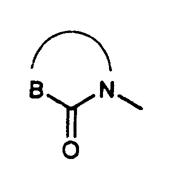 oder einen Rest der Formeln 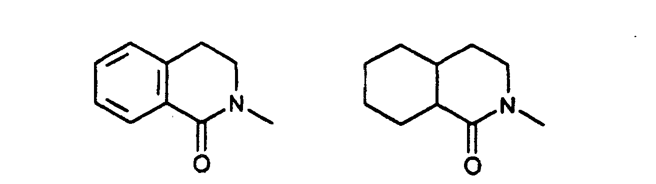 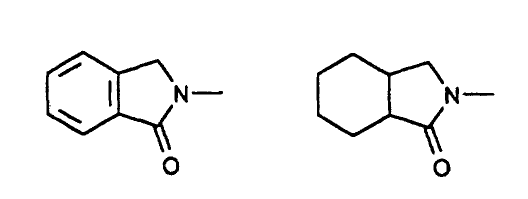 steht, kann z. B. auf den folgenden Wegen erfolgen. Auf einem Weg wird das Amin der Formel XVII zunächst in an sich bekannter Weise durch Reaktion mit Kohlensäurehalogeniden wie Phosgen oder Triphosgen in Gegenwart tertiärer Alkylamine oder Pyridin und inerter Lösungsmittel in ein Isocyanat der Formel XVIII 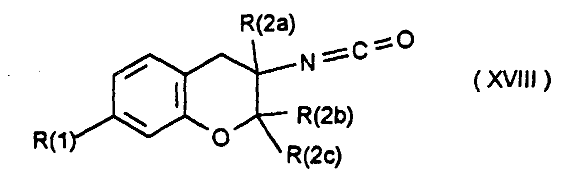 überführt, in der R(1), R(2a), R(2b) und R(2c) die eingangs angegebenen Bedeutungen haben. Als inerte Lösungsmittel eignen sich z. B. Ether wie Tetrahydrofuran, Dioxan, Ethylenglykoldimethylether oder Diglyme, Ketone wie Aceton oder Butanon, Nitrile wie Acetonitril, Nitroverbindungen wie Nitromethan, Ester wie Ethylacetat, Amide wie Dimethylformamid oder N-Methylpyrrolidon, Hexamethylphosphorsäuretriamid, Sulfoxide wie DMSO, Sulfone wie Sulfolan, Kohlenwasserstoffe wie Benzol, Toluol, Xylole. Weiterhin eignen sich auch Gemische dieser Lösungsmittel untereinander. Auf einem anderen Wege werden die Amine der Formel XVII zunächst in an sich bekannter Weise in reaktive Kohlensäurederivate überführt, z. B. in Kohlensäureester (Urethane), wie sie sich aus Chlorameisensäurealkylestem und Aminen der Formel XVII in Gegenwart geeigneter tertiärer Alkylamine oder Pyridin synthetisieren lassen. Weiterhin können auch N,N'-Carbonyldiimidazol und analoge reaktive Derivate als Isocyanatäquivalente eingesetzt werden (H. A. Staab, Synthesen mit heterocyclischen Amiden (Azoliden), Angewandte Chemie 74 (1962), 407 - 423).

Die Isocyanate der Formel XVIII oder die Urethane oder die aus Aminen der Formel XVII und z. B. N,N'-Carbonyldiimidazol erhaltenen Zwischenstufen können dann mit einer Verbindung der Formel, 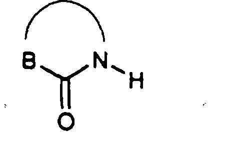 in der B die eingangs angegebene Bedeutung hat, oder einer Verbindung der Formeln 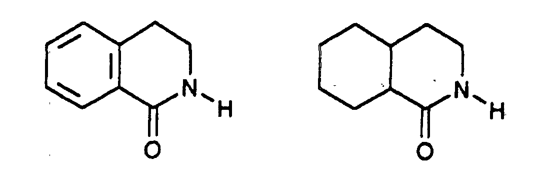 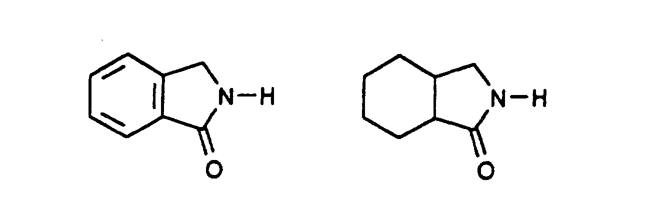 in Anwesenheit oder Abwesenheit inerter Lösungsmittel bei Temperaturen von 100 bis 170 °C gekoppelt werden (Justus Liebigs Ann. Chem. 598 (1956), 203) und liefern die entsprechenden Verbindungen der Formel XVIa, in der A für einen der heterocyclischen Reste steht.

Aus den acylierten Aminen der Formeln XVI bzw. XVIa können in an sich bekannter Weise unter geeigneten, an sich bekannten Reaktionsbedingungen die Sulfonamide der Formel IIIa hergestellt werden (vgl. Schema II). Dabei kann man auch von an sich bekannten, hier aber nicht erwähnten Varianten Gebrauch machen. Die Synthesen der Sulfonamide können in einem, zwei oder mehreren Schritten vollzogen werden. Insbesondere sind Verfahren bevorzugt, in denen die acylierten Amine der Formel XVI bzw. XVIa durch elektrophile Reagenzien in Anwesenheit oder Abwesenheit von inerten Lösungsmitteln bei Temperaturen von -10 °C bis 120 °C, vorzugsweise von 0 bis 100 °C, in die 6-Chromanylsulfonsäuren oder Derivate davon, zum Beispiel Sulfonsäurehalogenide der Formel VIIIa, überführt werden. Dafür können beispielsweise Sulfonierungen mit Schwefelsäuren oder Oleum, Halogensulfonierungen mit Halogensulfonsäuren wie Chlorsulfonsäure, Reaktionen mit Sulfurylhalogeniden in Gegenwart von wasserfreien Metallhalogeniden oder Reaktionen mit Thionylhalogeniden in Gegenwart von wasserfreien Metallhalogeniden mit anschließenden, in bekannter Weise durchgeführten Oxidationen zu Sulfonsäurechloriden, durchgeführt werden. Sind Sulfonsäuren die primären Reaktionsprodukte, so können diese entweder direkt oder nach Behandlung mit tertiären Aminen, wie zum Beispiel Pyridin oder Trialkylaminen, oder mit Alkalimetall- oder Erdalkalimetallhydroxiden oder Reagenzien, die diese basischen Verbindungen in situ bilden, in an sich bekannter Weise durch Säurehalogenide wie zum Beispiel Phosphortrihalogenide, Phosphorpentahalogenide, Phosphoroxychloride, Thionylhalogenide oder Oxalylhalogenide, in Sulfonsäurehalogenide z. B. der Formel VIIIa überführt werden. Die Überführung der Sulfonsäurederivate in Sulfonamide der Formel IIIa erfolgt in literaturbekannter Weise, vorzugsweise werden Sulfonsäurechloride in inerten Lösungsmitteln bei Temperaturen von 0 bis 100 °C mit wäßrigem Ammoniak umgesetzt.

Zur Synthese der Verbindungen der Formel I können aus den acylierten Aminen der Formel XVI auch entsprechende Sulfonamide der Formel IIIa hergestellt werden, in denen die Gruppe R(5)-C(=O) die Funktion einer Schutzgruppe hat. Wie im Schema II angegeben, kann die in den Formeln VIIIa und IIIa enthaltene Gruppe R(5) für die eingangs angegebenen Bedeutungen von A stehen, aber - genauso wie R(4) - auch beispielsweise für Alkyl mit 1 bis 4 C-Atomen oder für Trihalogenmethyl. Hat R(5) in der Formel IIIa die Bedeutungen von A, so liegen die Verbindungen der Formel III vor. Hat R(5) in der Formel VIIIa die Bedeutungen von A, so liegen die Verbindungen der Formel VIII vor.

Von den Verbindungen der Formel IIIa, die eine Schutzgruppe enthalten, kann nach der Einführung der Sulfonamidgruppe die Schutzgruppe mit Säuren oder Basen abgespalten werden, wie es oben für die Abspaltung der Schutzgruppe aus den Verbindungen der Formel XVI erläutert ist. Aus den so hergestellten Sulfonamidsubstituierten Aminen können dann, wie oben für die Einführung der Gruppe A-C(=O) in die Verbindungen der Formeln XIII oder XVII erläutert, die Sulfamoylchromane der Formel III hergestellt werden. Je nach der Natur der Reste R(1), R(2a), R(2b), R(2c), R(3), A und Z kann für die Synthese der Verbindungen der Formel I das eine oder das andere der genannten Verfahren und deren Ausführungsarten weniger gut geeignet sein oder zumindest Vorkehrungen zum Schutz reaktiver Gruppen erforderlich machen. Derartige, verhältnismäßig selten auftretende Fälle können aber vom Fachmann unschwer erkannt werden, und es bereitet keine Schwierigkeiten, in solchen Fällen einen anderen der beschriebenen Synthesewege erfolgreich anzuwenden.

Die Verbindungen der Formel I beeinflussen das Aktionspotential von Zellen, insbesondere von Herzmuskelzellen. Sie haben eine normalisierende Wirkung auf ein gestörtes Aktionspotential, wie es beispielsweise bei Ischämien vorliegt, und eignen sich somit z. B. zur Behandlung und Prophylaxe von Störungen des cardiovaskulären Systems, insbesondere von Arrhythmien und ihren Folgen. Die Wirksamkeit der Verbindungen der Formel I läßt sich beispielsweise in dem unten beschriebenen Modell nachweisen, in dem die Dauer des Aktionspotentials am Papillarmuskel des Meerschweinchens bestimmt wird.

Die Verbindungen der Formel I und ihre physiologisch unbedenklichen Salze können daher am Tier, bevorzugt am Säugetier, und insbesondere am Menschen a!s Arzneimittel für sich allein, in Mischungen untereinander oder in Form von pharmazeutischen Zubereitungen verwendet werden. Säugetiere, an denen die Verbindungen der Formel I angewandt oder geprüft werden können, sind zum Beispiel Affen, Hunde, Mäuse, Ratten, Kaninchen, Meerschweinchen, Katzen und größere Nutztiere wie z. B. Rinder und Schweine. Gegenstand der vorliegenden Erfindung sind auch die Verbindungen der Formel I und/oder ihre physiologisch unbedenklichen Salze zur Verwendung als Arzneimittel sowie pharmazeutische Zubereitungen, die als aktiven Bestandteil eine wirksame Dosis mindestens einer Verbindung der Formel I und/oder eines physiologisch unbedenklichen Salzes davon neben üblichen, pharmazeutisch einwandfreien Träger- und Hilfsstoffen enthalten. Die pharmazeutischen Zubereitungen können für eine enterale oder eine parenterale Anwendung bestimmt sein und enthalten normalerweise 0.5 bis 90 Gewichtsprozent der Verbindungen der Formel I und/oder ihrer physiologisch unbedenklichen Salze.

Die Herstellung der erfindungsgemäßen pharmazeutischen Zubereitungen kann in an sich bekannter Weise erfolgen. Dazu werden die Verbindungen der Formel I und/oder ihre physiologisch unbedenklichen Salze zusammen mit einem oder mehreren festen oder flüssigen Trägerstoffen und/oder Hilfsstoffen und, wenn gewünscht, in Kombination mit anderen Arzneimitteln, z.B. Herz-Kreislauf-aktiven Arzneimitteln wie etwa Calcium-Antagonisten oder ACE-Hemmern, in eine geeignete Dosierungsform und Darreichungsform gebracht, die dann als Arzneimittel in der Human- oder Veterinärmedizin verwendet werden kann.

Als Trägerstoffe kommen organische oder anorganische Substanzen in Frage, die sich z. B. für die enterale (zum Beispiel die orale) Applikation oder für die parenterale Applikation (zum Beispiel die intravenöse Injektion oder Infusion) oder für topische Anwendungen eignen und mit den Verbindungen der Formel I nicht reagieren, beispielsweise Wasser, pflanzliche Öle, Wachse, Alkohole wie Ethanol, Propandiol oder Benzylalkohole, Glycerin, Polyole, Polyethylenglykole, Polypropylenglykole, Glycerintriacetat, Gelatine, Kohlenhydrate wie Lactose oder Stärke, Stearinsäure und deren Salze wie Magnesiumstearat, Talk, Lanolin, Vaseline. Zur oralen und rektalen Anwendung dienen insbesondere Arzneiformen wie Tabletten, Dragees, Kapseln, Suppositorien, Lösungen, vorzugsweise ölige oder wäßrige Lösungen, Sirupe, Säfte oder Tropfen, ferner Suspensionen oder Emulsionen. Für die topische Anwendung dienen insbesondere Salben, Cremes, Pasten, Lotionen, Gele, Sprays, Schäume, Aerosole, Lösungen oder Puder. Als Lösungsmittel für Lösungen können zum Beispiel Wasser oder Alkohole wie Ethanol, Isopropanol oder 1,2-Propandiol oder deren Gemische untereinander oder mit Wasser dienen. Als weitere Arzneiformen kommen z.B. auch Implantate in Betracht. Die Verbindungen der Formel I können auch lyophilisiert und die erhaltenen Lyophilisate zum Beispiel zur Herstellung von Injektionspräparaten verwendet werden. Insbesondere für die topische Anwendung kommen auch liposomale Zubereitungen in Betracht. Die pharmazeutischen Zubereitungen können Hilfsstoffe wie Gleit-, Konservierungs-, Spreng-, Dickungs-, Stabilisierungsund/oder Netzmittel, Mittel zur Erzielung eines Depoteffekts, Emulgatoren, Salze (z.B. zur Beeinflussung des osmotischen Druckes), Puffersubstanzen, Farb-, Geschmacks- und/oder Aromastoffe enthalten. Sie können, falls erwünscht, auch einen oder mehrere weitere Wirkstoffe enthalten und/oder zum Beispiel ein oder mehrere Vitamine.

Die Verbindungen der Formel I und ihre physiologisch unbedenklichen Salze sind wertvolle Therapeutika, die sich nicht nur als Antiarrhythmika, sondern auch zur Behandlung und Prophylaxe bei anderen Störungen des cardiovaskulären Systems, bei Herzinsuffizienz, Ischämien, Herztransplantationen, oder bei cerebralen Gefäßerkrankungen zum Einsatz an Menschen oder Säugetieren eignen. Insbesondere finden sie Verwendung als Antiarrhythmika zur Behandlung von Herzrhythmusstörungen unterschiedlichster Genese und zur Verhinderung des arrhythmisch bedingten plötzlichen Herztodes. Beispiele arrhythmischer Störungen des Herzens sind supraventrikuläre Rhythmusstörungen wie etwa Vorhof-Tachycardien, Vorhof-Flattern oder paroxysmale supraventrikuläre Rhythmusstörungen, oder ventrikuläre Rhythmusstörungen wie ventrikuläre Extrasystolen, insbesondere aber lebensbedrohende ventrikuläre Tachycardien oder das besonders gefährliche Kammerflimmern. Sie eignen sich insbesondere für solche Fälle, wo Arrhythmien Folge einer Verengung eines Koronargefäßes sind, wie sie beispielsweise bei Angina pectoris oder während eines akuten Herzinfarkts oder als chronische Folge eines Herzinfarkts auftreten. Sie sind daher insbesondere bei Postinfarktpatienten zur Verhinderung des plötzlichen Herztodes geeignet. Weitere Krankheitsbilder, wo derartige Rhythmusstörungen und/oder der plötzliche, arrhythmisch bedingte Herztod eine Rolle spielen, sind beispielsweise die Herzinsuffizienz oder die Herzhypertrophie als Folge eines chronisch erhöhten Blutdrucks.

Darüberhinaus sind die Verbindungen der vorliegenden Erfindung in der Lage, eine verminderte Kontraktilität des Herzens und eine geschwächte Herzkraft positiv zu beeinflussen. Hierbei kann es sich um ein krankheitsbedingtes Nachlassen der Herzkontraktilität handeln, wie beispielsweise bei Herzinsuffizienz, aber auch um akute Fälle wie Herzversagen bei Schockeinwirkungen. Ebenso kann unter dem Einfluß der Verbindungen der Formel I bei einer Herztransplantation das Herz nach erfolgter Operation seine Leistungsfähigkeit rascher und zuverlässiger wieder aufnehmen. Gleiches gilt für Operationen am Herz, die eine vorübergehende Still-Legung der Herzaktivität durch cardioplegische Lösungen erforderlich machen.

Gegenstand der vorliegenden Erfindung ist auch die Verwendung der Verbindungen der Formel I und/oder ihrer physiologisch unbedenklichen Salze zur Behandlung und Prophylaxe der genannten Krankheitsbilder sowie die Verwendung zur Herstellung von Arzneimitteln zur Anwendung bei diesen Krankheiten.

Die Dosierungen, die z. B. zur Behandlung von Herzrhythmusstörungen mit den Verbindungen der Formel I notwendig sind, hängen davon ab, ob akut oder prophylaktisch therapiert wird, und richten sich nach dem jeweiligen Einzelfall. Normalerweise kommt man mit einer Dosis aus, die im Bereich von etwa mindestens 0.01 mg, vorzugsweise 0.1 mg, insbesondere 1 mg, bis höchstens 100 mg, vorzugsweise 10 mg (jeweils pro kg Körpergewicht und Tag) liegt, wenn Prophylaxe betrieben wird. Besonders geeignet ist ein Dosisbereich von 1 bis 10 mg pro kg und Tag. Die Dosis kann dabei in Form einer oralen oder parenteralen Einzeldosis verabreicht werden oder in mehrere, insbesondere z.B. zwei, drei oder vier, Einzeldosen aufgeteilt werden. Werden akute Fälle von Herzrhythmusstörungen behandelt, beispielsweise auf einer Intensivstation, kann die parenterale Verabreichung, z.B. durch Injektion oder Infusion, vorteilhaft sein. Ein bevorzugter Dosisbereich in kritischen Situationen kann dann 10 bis 100 mg pro kg und Tag betragen und beispielsweise als intravenöse Dauerinfusion verabreicht werden.

Die Verbindungen der Formel I inhibieren ATP-sensitive Kaliumkanäle von Zellen. Außer als Arzneimittelwirkstoffe in der Human- und Veterinärmedizin können die Verbindungen der Formel I auch als wissenschaftliches Tool oder als Hilfsmittel für biochemische Untersuchungen eingesetzt werden, bei denen eine derartige Beeinflussung von Ionenkanälen beabsichtigt ist, sowie für diagnostische Zwecke. Ferner können die Verbindungen der Formel I und ihre Salze als Zwischenprodukte zur Herstellung weiterer Arzneimittelwirkstoffe verwendet werden.

Analog zu den in den untenstehenden Ausführungsbeispielen beschriebenen Verbindungen können beispielsweise auch die folgenden Verbindungen der Formel I erhalten werden:
3-(5-Chlor-2-methoxy-benzamido)-6-(methylaminocarbonylaminosulfonyl)-7-ethylchroman
3-(5-Chlor-2-methoxy-benzamido)-6-(methylaminothiocarbonylaminosulfonyl)-7-ethoxy-chroman
3-(5-Brom-2-methoxy-benzamido)-6-(methylaminocarbonylaminosulfonyl)-7-ethylchroman
3-(5-Brom-2-methoxy-benzamido)-6-(methylaminothiocarbonylaminosulfonyl)-7-methoxy-chroman
3-(5-Chlor-2-methoxy-benzamido)-6-(ethylaminocarbonylaminosulfonyl)-7-ethylchroman
3-(5-Brom-2-methoxy-benzamido)-6-(ethylaminocarbonylaminosulfonyl)-7-ethylchroman
3-(5-Chlor-2-methoxy-benzamido)-6-(ethylaminothiocarbonylaminosulfonyl)-7-ethoxy-chroman
3-(5-Brom-2-methoxy-benzamido)-6-(ethylaminothiocarbonylaminosulfonyl)-7-methoxy-chroman
3-(3-Ethyl-4-methyl-2-oxo-3-pyrrolin-1-carboxamido)-6-(methylaminothiocarbonylaminosulfonyl)-7-ethoxy-chroman
3-(3-Ethyl-4-methyl-2-oxo-3-pyrrolin-1-carboxamido)-6-(methylaminocarbonylaminosulfonyl)-7-ethoxy-chroman
3-(3-Ethyl-4-methyl-2-oxo-3-pyrrolin-1-carboxamido)-6-(methylaminocarbonylaminosulfonyl)-7-ethyl-chroman
3-(3-Ethyl-4-methyl-2-oxo-3-pyrrolin-1-carboxamido)-6-(methylaminocarbonylaminosutfonyl)-7-ethyl-chroman
3-(2-Oxo-3-pyrrolin-1-carboxamido)-6-(methylaminothiocarbonylaminosulfonyl)-7-ethoxy-chroman
3-(2-Oxo-3-pyrrolin-1-carboxamido)-6-(methylaminocarbonylaminosulfonyl)-7-ethoxy-chroman
3-(2-Oxo-3-pyrrolin-1-carboxamido)-6-(methylaminothiocarbonylaminosulfonyl)-7-ethyl-chroman
3-(2-Oxo-3-pyrrolin-1-carboxamido)-6-(methylaminocarbonylaminosulfonyl)-7-ethylchroman
3-(2-Oxo-3-pyrrolin-1-carboxamido)-6-(methylaminothiocarbonylaminosulfonyl)-7-ethoxy-chroman
3-(2-Oxo-3-pyrrolin-1-carboxamido)-6-(methylaminocarbonylaminosulfonyl)-7-ethoxy-chroman

### Beispiele

### Beispiel 1:

### 3-(5-Chlor-2-methoxy-benzamido)-6-(methylaminocarbonylaminosulfonyl)-7-methoxy-chroman

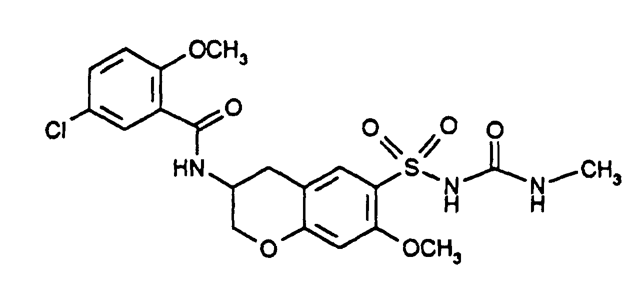

1.71 g (4 mmol) 3-(5-Chlor-2-methoxy-benzamido)-6-sulfamoyl-7-methoxy-chroman wurden in 10 ml trockenem DMSO nach Zugabe von 0.4 g (10 mmol) frisch pulverisierten Natriumhydroxid und 1.05 g (6 mmol) N-Methyltrichloracetamid 30 Minuten auf 80 °C erhitzt. Der abgekühlte Reaktionsansatz wurde in Eiswasser eingetragen, mit Aktivkohle geklärt und auf pH 1 angesäuert. Der Niederschlag wurde abgesaugt, getrocknet und zweimal aus Ethanol umkristallisiert. Das Produkt hatte einen Schmelzpunkt von 256 -257 °C.

Herstellung der Ausgangsverbindung 3-(5-Chlor-2-methoxy-benzamido)-6-sulfamoyl-7-methoxy-chroman

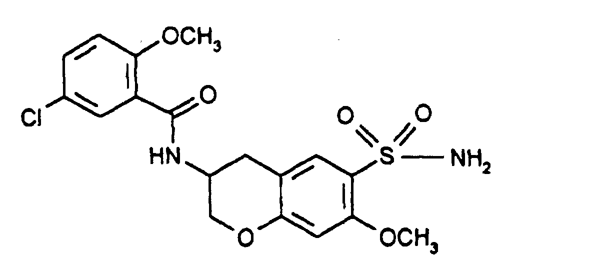

15.1 g (70 mmol) 3-Amino-7-methoxy-chroman-hydrochlorid (Eur. J. Med. Chem. 11 (1976), 251 - 256) wurden in 80 ml Pyridin gelöst und bei 0 °C mit 14.8 g 2-Methoxy-5-chlor-benzoesäurechlorid versetzt. Man rührte 1.5 Stunden bei Zimmertemperatur und 1 Stunde bei 60 °C. Die abgekühlte Reaktionsmischung wurde zwischen Wasser und Methylenchlorid verteilt. Die wäßrige Phase wurde dreimal mit Methylenchlorid extrahiert. Die vereinigten organischen Phasen wurden mit 2 N Salzsäure, Wasser und Bikarbonatlösung gewaschen. Nach dem Trocknen und Eindampfen der organischen Phase wurde der Rückstand in wenig Toluol gelöst und mit überschüssigem Diethylether gefällt. Man erhielt 3-(5-Chlor-2-methoxy-benzamido)-7-methoxy-chroman vom Schmelzpunkt 92-93 °C.

20 g 3-(5-Chlor-2-methoxy-benzamido)-7-methoxy-chroman wurden portionsweise unter Rühren in 35 ml auf -10 °C gekühlte Chlorsulfonsäure eingetragen. Man ließ auf Zimmertemperatur kommen und gab weitere 5 ml Chlorsulfonsäure zu. Nach 1 Stunde wurde vorsichtig in Eiswasser eingerührt. Der erhaltene Niederschlag wurde abgesaugt und nach Waschen mit Wasser in eine auf -20 °C gekühlte Mischung aus 200 ml Aceton und 120 ml konzentriertem wäßrigem Ammoniak eingetragen. Man ließ auf Raumtemperatur erwärmen. Nach Stehen über Nacht wurde die Lösung bei 30 °C im Vakuum eingeengt. Unter Eiskühlung wurde der Rückstand mit konzentrierter Salzsäure versetzt. Der erhaltene Niederschlag wurde abgesaugt und aus Eisessig/Methanol umkristallisiert. Das erhaltene 3-(5-Chlor-2-methoxybenzamido)-6-sulfamoyl-7-methoxy-chroman hatte einen Schmelzpunkt von 210-212°C.

### Beispiel 2:

### 3-(5-Chlor-2-methoxy-benzamido)-6-(ethylaminocarbonylaminosulfonyl)-7-methoxychroman

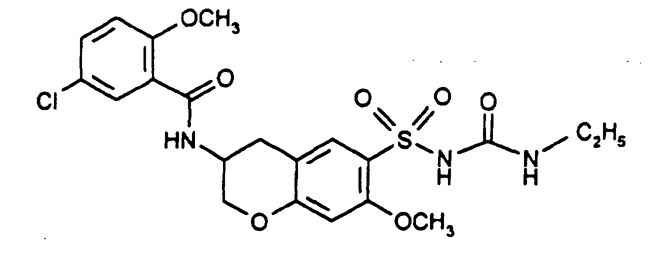

Die Substanz wurde analog Beispiel 1 mit 1.15 g (6 mmol) N-Ethyltrichloracetamid an Stelle des N-Methyltrichloracetamids hergestellt und hatte nach Umkristallisation aus Ethanol einen Schmelzpunkt von 233-234 °C.

### Beispiel 3:

### 3-(5-Chlor-2-methoxy-benzamido)-6-(n-propylaminocarbonylaminosulfonyl)-7-methoxy-chroman

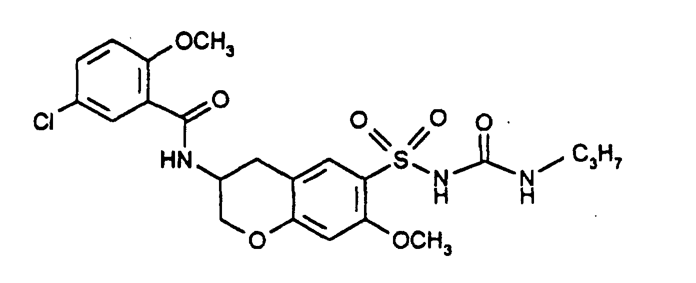

Die Substanz wurde analog Beispiel 1 mit 1.23 g (6 mmol) N-n-Propyltrichloracetamid an Stelle des N-Methyltrichloracetamids hergestellt und hatte nach Umkristallisation aus Essigester einen Schmelzpunkt von 203-205 °C.

### Beispiel 4:

### 3-(5-Chlor-2-methoxy-benzamido)-6-(isopropylaminocarbonylaminosulfonyl)-7-methoxy-chroman

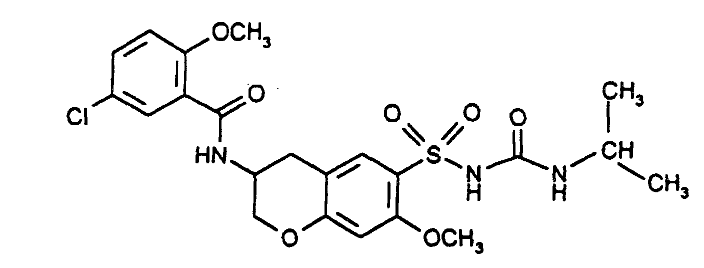

Die Substanz wurde analog Beispiel 1 mit 1.23 g (6 mmol) N-Isopropyltrichloracetamid an Stelle des N-Methyltrichloracetamids hergestellt und hatte nach Umkristallisation aus Methanol einen Schmelzpunkt von 181-183 °C.

### Beispiel 5:

### 3-(5-Chlor-2-methoxy-benzamido)-6-(n-butylaminocarbonylaminosulfonyl)-7-methoxy-chroman

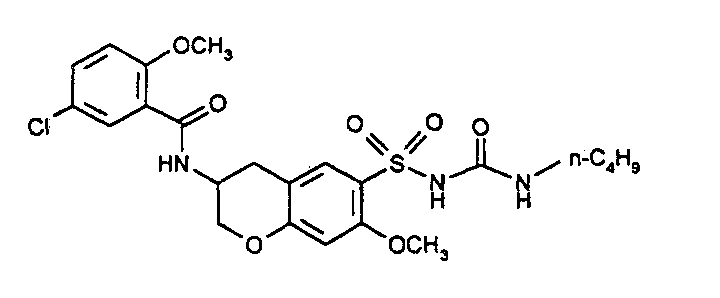

Die Substanz wurde analog Beispiel 1 mit 1.31 g (6 mmol) N-n-Butyltrichloracetamid an Stelle des N-Methyltrichloracetamids hergestellt und hatte nach Umkristallisation aus Methanol einen Schmelzpunkt von 185-186 °C.

### Beispiel 6:

### 3-(5-Chlor-2-methoxy-benzamido)-6-(methylaminothiocarbonylaminosulfonyl)-7-methoxy-chroman

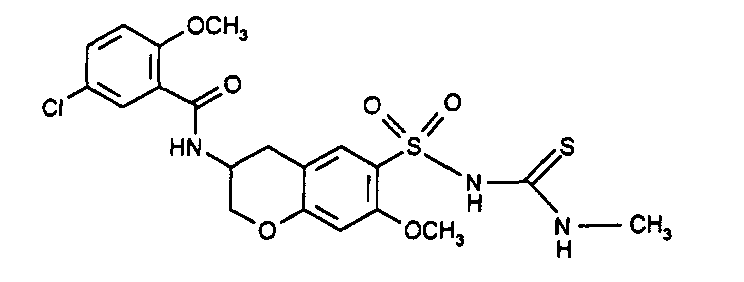

1.71 g (4 mmol) 3-(5-Chlor-2-methoxy-benzamido)-6-sulfamoyl-7-methoxy-chroman (Beispiel 1) wurden in 10 ml trockenem DMSO gelöst und mit 1.65 g (12 mmol) fein gepulvertem Kaliumkarbonat sowie 0.35 g (4.8 mmol) Methylisothiocyanat versetzt. Nach 25minütigem Rühren bei 80 °C wurde abgekühlt, in Eiswasser eingetragen, mit Kohle geklärt und auf pH 1 angesäuert. Der Niederschlag wurde abgesaugt, getrocknet und aus Ethanol/DMF umkristallisiert. Schmelzpunkt: 219-220 °C.

### Beispiel 7:

### 3-(5-Chlor-2-methoxy-benzamido)-6-(ethylaminothiocarbonylaminosulfonyl)-7-methoxy-chroman

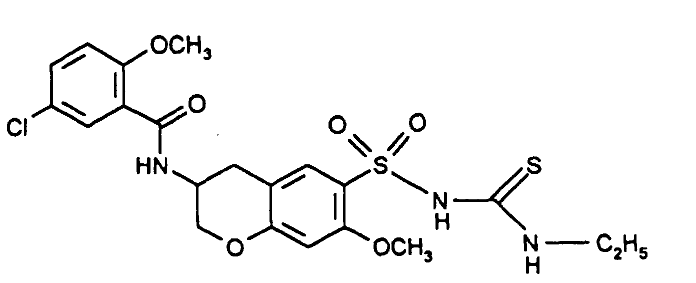

Die Substanz wurde analog Beispiel 6 mit 0.41 g (4.8 mmol) Ethylisothiocyanat an Stelle des Methylisothiocyanats hergestellt und hatte nach Umkristallisation aus Methanol/DMF einen Schmelzpunkt von 194-195 °C.

### Beispiel 8:

### 3-(5-Chlor-2-methoxy-benzamido)-6-(n-propylaminothiocarbonylaminosulfonyl)-7-methoxy-chroman

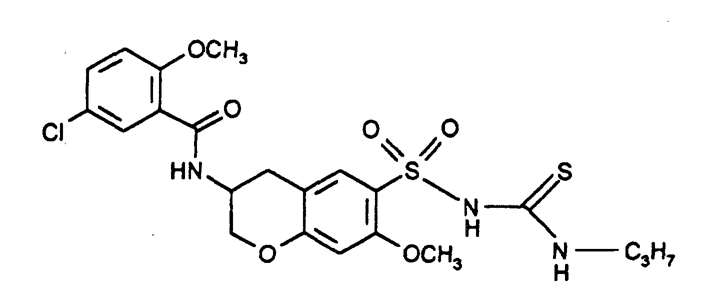

Die Substanz wurde analog Beispiel 6 mit 0.5 ml (4.8 mmol) n-Propylisothiocyanat an Stelle des Methylisothiocyanats hergestellt und hatte nach Umkristallisation aus Ethanol/DMF einen Schmelzpunkt von 182 °C.

### Beispiel 9:

### 3-(5-Fluor-2-methoxy-benzamido)-6-(methylaminocarbonylaminosulfonyl)-7-methoxy-chroman

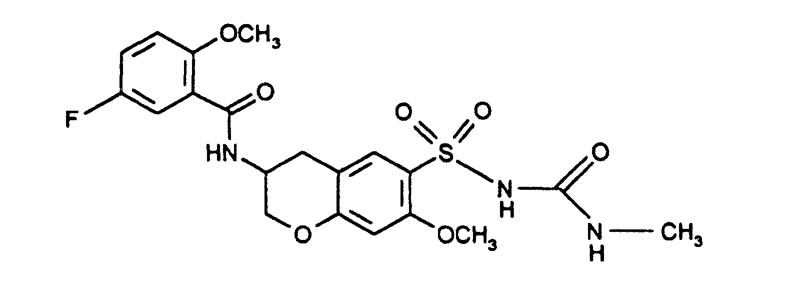

1.64 g (4 mmol) 3-(5-Fluor-2-methoxy-benzamido)-6-sulfamoyl-7-methoxy-chroman wurden in 10 ml trockenem DMSO nach Zugabe von 0.4 g (10 mmol) frisch pulverisiertem Natriumhydroxid und 1.05 g (6 mmol) N-Methyltrichloracetamid 30 Minuten auf 80 °C erhitzt. Der abgekühlte Reaktionsansatz wurde in Eiswasser eingetragen, mit Aktivkohle geklärt und auf pH 1 angesäuert. Der Niederschlag wurde abgesaugt, getrocknet und aus Ethanol umkristallisiert. Das Produkt hatte einen Schmelzpunkt von 260 °C.

Herstellung der Ausgangsverbindung 3-(5-Fluor-2-methoxy-benzamido)-6-sulfamoyl-7-methoxy-chroman

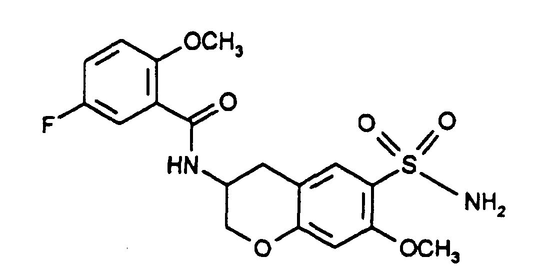

Zu 15.1 g (70 mmol) 3-Amino-7-methoxy-chroman-hydrochlorid in 80 ml auf 0 °C gekühltem Pyridin wurden 13.6 g (72 mmol) 2 Methoxy-5-fluor-benzoesäurechlorid zugesetzt. Die Aufarbeitung erfolgte analog Beispiel 1. Das erhaltene 3-(5-Fluor-2-methoxy-benzamido)-7-methoxy-chroman hatte nach Umkristallisation aus Ethanol einen Schmelzpunkt von 107-108 °C. Die weitere Umsetzung mit Chlorsulfonsäure und Ammoniak erfolgte analog Beispiel 1. Das erhaltene 3-(5-Fluor-2-methoxybenzamido)-6-sulfamoyl-7-methoxy-chroman hatte nach Umkristallisation aus DMF/Methanol einen Schmelzpunkt von 209-210 °C.

### Beispiel 10:

### 3-(5-Fluor-2-methoxy-benzamido)-6-(methylaminothiocarbonylaminosulfonyl)-7-methoxy-chroman

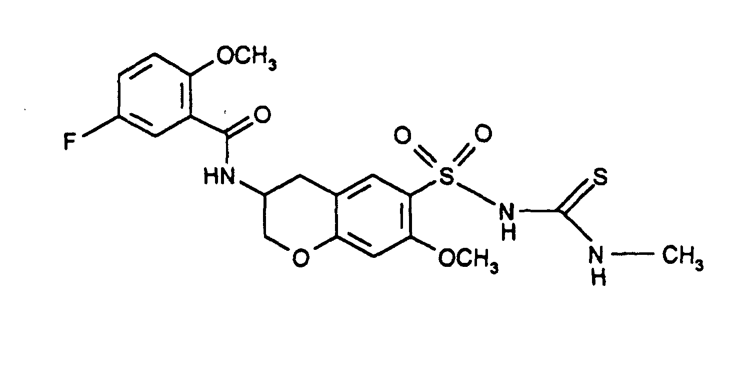

1.64 g (4 mmol) 3-(5-Fluor-2-methoxy-benzamido)-6-sulfamoyl-7-methoxy-chromar (Beispiel 9) wurden in 10 ml trockenem DMSO mit 1.65 g (12 mmol) gepulvertem Kaliumkarbonat sowie 0.35 g (4.8 mmol) Methylisothiocyanat versetzt. Nach 25minütigem Rühren bei 80 °C wurde abgekühlt, in Eiswasser eingetragen, mit Kohle geklärt und auf pH 1 angesäuert. Der Niederschlag wurde abgesaugt, getrocknet und aus Ethanol umkristallisiert. Das Produkt hatte einen Schmelzpunkt von 221-222 °C.

### Beispiel 11:

### 3-(5-Fluor-2-methoxy-benzamido)-6-(ethylaminothiocarbonylaminosulfonyl)-7-methoxy-chroman

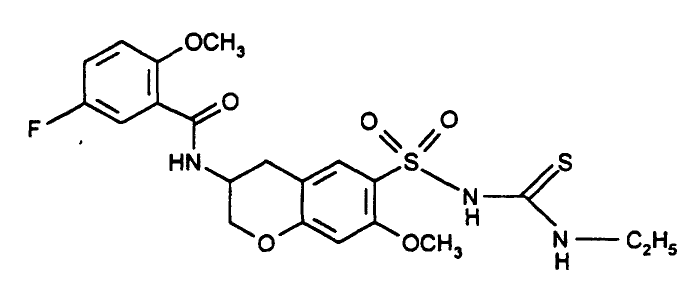

Die Substanz wurde analog Beispiel 10 mit 0.41 g (4.8 mmol) Ethylisothiocyanat an Stelle des Methylisothiocyanats hergestellt und hatte einen Schmelzpunkt von 186-187 °C.

### Beispiel 12:

### 3-(5-Fluor-2-methoxy-benzamido)-6-(n-propylaminothiocarbonylaminosulfonyl)-7-methoxy-chroman

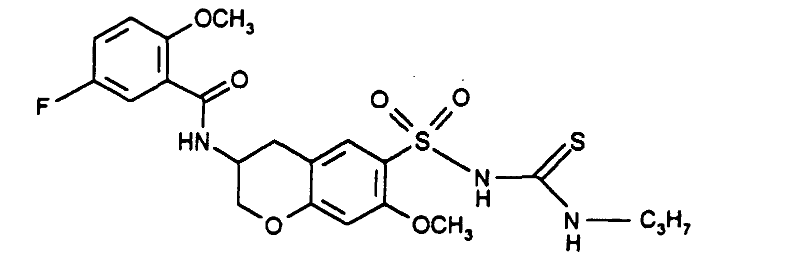

Die Substanz wurde analog Beispiel 10 mit 0.5 ml (4.8 mmol) n-Propylisothiocyanat an Stelle des Methylisothiocyanats hergestellt und hatte nach Umkristallisation aus Ethanol einen Schmelzpunkt von 172-173 °C.

### Beispiel 13:

### 3-(5-Fluor-2-methoxy-benzamido)-6-(isopropylaminothiocarbonylaminosulfonyl)-7-methoxy-chroman

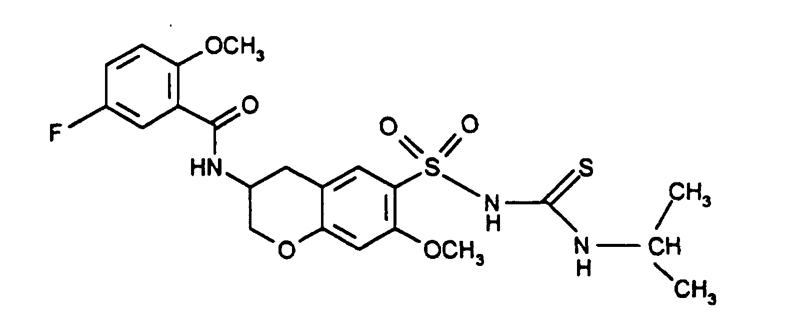

Die Substanz wurde analog Beispiel 10 mit 0.48 ml Isopropylisothiocyanat an Stelle des Methylisothiocyanats hergestellt und hatte nach Umkristallisation aus Ethanol einen Schmelzpunkt von 179-180 °C.

### Beispiel 14:

### 3-(5-Chlor-2-methoxy-benzamido)-6-(methylaminothiocarbonylaminosulfonyl)-7-methyl-chroman

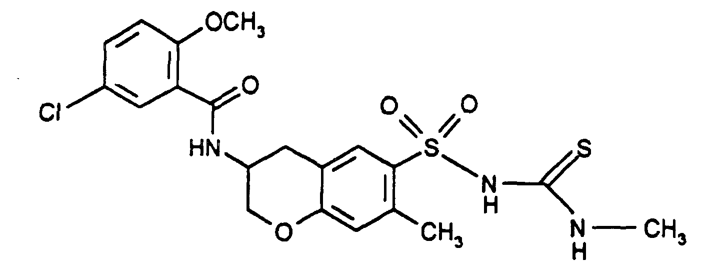

1.64 g (4 mmol) 3-(5-Chlor-2-methoxy-benzamido)-6-sulfamoyl-7-methyl-chroman wurden in 10 ml trockenem DMSO gelöst und mit 1.65 g (12 mmol) fein gepulvertem Kaliumkarbonat sowie 0.35 g (4.8 mmol) Methylisothiocyanat versetzt. Nach 25minütigem Rühren bei 80 °C wurde abgekühlt, in Eiswasser eingetragen, mit Kohle geklärt und auf pH 1 angesäuert. Der Niederschlag wurde abgesaugt, getrocknet, über eine Kieselgelsäule mit Essigsäureethylester/Toluol 2:1 gereinigt und aus Ethanol umkristallisiert. Schmelzpunkt: 207-208 °C.

Herstellung der Ausgangsverbindung 3-(5-Chlor-2-methoxy-benzamido)-6-sulfamoyl-7-methyl-chroman

### a) 3-(5-Chlor-2-methoxy-benzamido)-7-methyl-4-chromanon

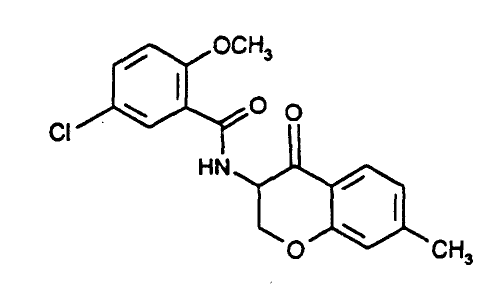

Zu einer Lösung von 18.2 g (85 mmol) 3-Amino-7-methyl-4-chromanon-hydrochlorid (Hebd. Seances Acad. Sci. Ser. C. 279, 281-284) in 90 ml Pyridin wurden bei Zimmertemperatur 17.5 g (85 mmol) 5-Chlor-2-methoxybenzoylchlorid zugegeben. Nach zweistündigem Rühren (DC-Kontrolle: Kieselgelplatte mit Petrolether/Essigsäureethylester/Toluol 2:2:1) wurde der Ansatz in Eis/Wasser eingetragen. Der Niederschlag wurde abgesaugt, mehrfach mit Wasser gewaschen und getrocknet. Das 3-(5-Chlor-2-methoxy-benzamido)-7-methyl-4-chromanon hatte einen Schmelzpunkt von 177-178 °C.

### b) 3-(5-Chlor-2-methoxy-benzamido)-7-methyl-chroman-4-ol

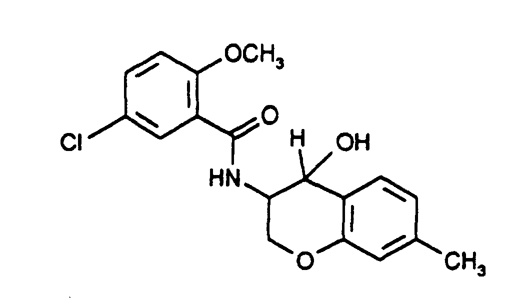

In eine Suspension von 8.65 g (25 mmol) 3-(5-Chlor-2-methoxy-benzamido)-7-methyl-4-chromanon in 40 ml Ethanol wurden 0.5 g (12.5 mmol) Natriumborhydrid eingetragen. Während zweistündigen Rührens bei 30-40 °C ging der Feststoff in Lösung. Die Lösung wurde anschließend abgekühlt, in Eis/Wasser eingetragen und mit verdünnter Salzsäure auf pH 1-2 angesäuert. Der Niederschlag wurde abgesaugt, mit Wasser gewaschen, getrocknet und aus Ethanol umkristallisiert. Das erhaltene 3-(5-Chlor-2-methoxy-benzamido)-7-methyl-chroman-4-ol (Diastereomerengemisch) hatte einen Schmelzpunkt von 151-152 °C.

### c) 3-(5-Chlor-2-methoxy-benzamido)-7-methyl-chroman

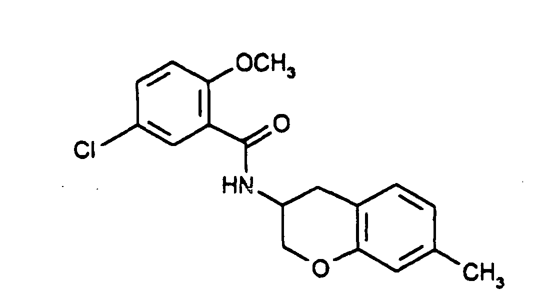

7.7 g (22 mmol) 3-(5-Chlor-2-methoxy-benzamido)-7-methyl-chroman-4-ol wurden in einer Mischung von 80 ml Eisessig, 7.5 ml Essigsäureanhydrid, 0.5 ml Trifluoressigsäure mit 0.5 g Pd/C (10 %ig) ca. 3 Stunden bei 25 °C bei Atmosphärendruck hydriert. Der Katalysator wurde abfiltriert und das Filtrat im Vakuum auf ein kleines Volumen eingeengt. Der Rückstand wurde in Eis/Wasser eingetragen und mehrfach mit Methylenchlorid extrahiert. Die vereinigten Methylenchloridextrakte wurden mit Natriumbikarbonatlösung und Wasser gewaschen, getrocknet, eingeengt und der Rückstand wurde aus Diisopropylether umkristallisiert. Das 3-(5-Chlor-2-methoxy-benzamido)-7-methyl-chroman hatte einen Schmelzpunkt von 97 °C.

### d) 3-(5-Chlor-2-methoxy-benzamido)-6-sulfamoyl-7-methyl-chroman

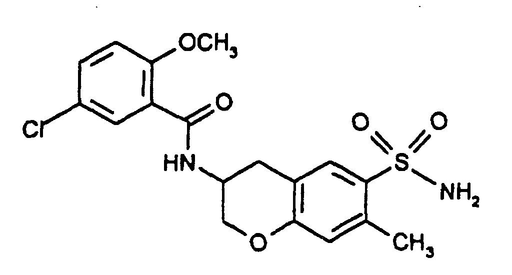

5.8 g 3-(5-Chlor-2-methoxy-benzamido)-7-methyl-chroman wurden unter leichter Kühlung in 10 ml Chlorsulfonsäure eingerührt. Nach ca. 45minütigem Rühren bei Zimmertemperatur wurde vorsichtig in Eis/Wasser eingetropft. Der Niederschlag wurde abgesaugt und in eine auf ca. -10 °C gekühlte Mischung aus 50 ml Aceton und 30 ml konzentriertem wäßrigem Ammoniak eingetragen. Man ließ auf Raumtemperatur kommen, rührte 3 Stunden nach und engte die Lösung bei 30°C im Vakuum ein. Unter Eiskühlung wurde der Rückstand mit konz. Salzsäure angesäuert. Der Niederschlag wurde abgesaugt, mit Wasser neutral gewaschen und aus Eisessig/Methanol umkristallisiert. Das 3-(5-Chlor-2-methoxy-benzamido)-6-sulfamoyl-7-methyl-chroman hatte einen Schmelzpunkt bei 218-219 °C.

### Beispiel 15:

### 3-(5-Chlor-2-methoxy-benzamido)-2,2-dimethyl-6-(methylaminocarbonylaminosulfonyl)-7-methoxy-chroman

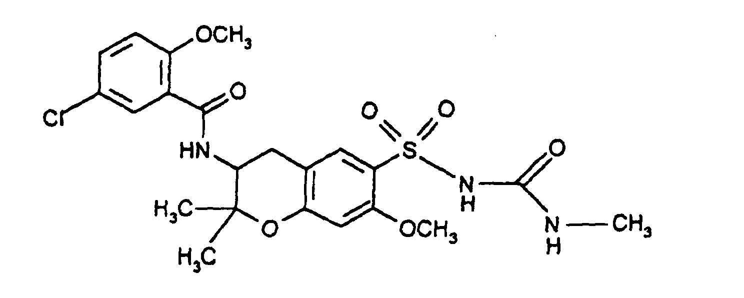

Zu 2.27 g (5 mmol) 3-(5-Chlor-2-methoxy-benzamido)-2,2-dimethyl-6-sulfamoyl-7-methoxy-chroman und 0.5 g (12.5 mmol) fein gepulvertem Natriumhydroxid wurden 1.23 g (7.5 mmol) N-Methyltrichloracetamid zugesetzt. Nach halbstündigem Rühren bei 80 °C wurde in Eis/Wasser eingetragen, mit Kohle geklärt und auf pH 1 angesäuert. Der Niederschlag wurde abgesaugt, getrocknet und aus Ethanol/DMF umkristallisiert. Das Produkt hatte einen Schmelzpunkt von 248 °C.

Herstellung der Ausgangsverbindung 3-(5-Chlor-2-methoxy-benzamido)-2,2-dimethyl-6-suffamoyl-7-methoxy-chroman

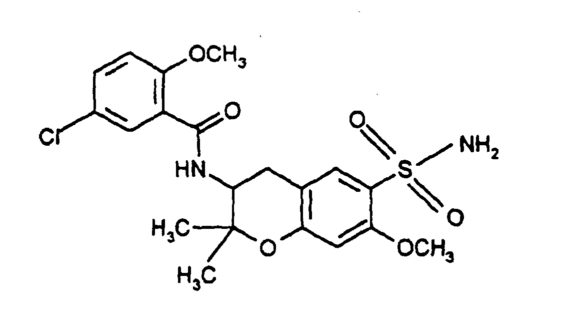

### a) 2,2-Dimethyl-7-methoxy-4-chromanon-oxim-tosylat

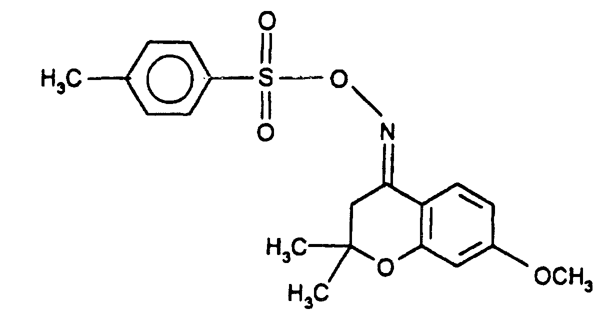

In eine Lösung von 88.5 g (0.4 mol) 2,2-Dimethyl-7-methoxy-4-chromanon-oxim (Heterocycles 38 (1994), 305-318) in 550 ml Pyridin wurden bei 0 °C 85.8 g (0.45 mol) p-Toluolsulfonsäurechlorid eingetragen. Man ließ auf Zimmertemperatur kommen, rührte mehrere Stunden nach, rührte in Eis/Wasser ein und extrahierte mit Dichlormethan. Die organische Lösung wurde zweimal mit 2 N Salzsäure und anschließend mehrfach mit Wasser gewaschen, getrocknet, eingedampft und der Rückstand aus Ethanol umkristallisiert. Das 2,2-Dimethyl-7-methoxy-4-chromanonoxim-tosylat hatte einen Schmelzpunkt von 113 °C.

### b) 3-Amino-2,2-dimethyl-7-methoxy-4-chromanon-hydrochlorid

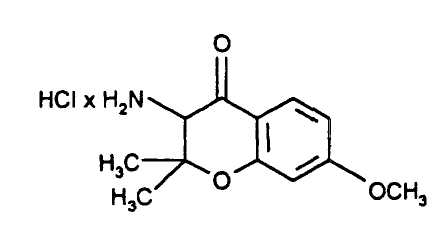

6.9 g (0.3 mol) Natrium wurden unter Stickstoff und leichtem Kühlen in 250 ml Ethanol gelöst. Zu dieser Natriumethylatlösung gab man eine Suspension von 105 g (0.28 mol) 2,2-Dimethyl-7-methoxy-4-chromanon-oxim-tosylat in 900 ml Ethanol zu. Man erwärmte auf 50 °C, hielt 3 Stunden bei dieser Temperatur, erwärmte 1 Stunde auf 60 °C, kühlte ab, saugte vom ausgefallenen Natriumsulfonat ab, engte ein, goß in salzsaures Eiswasser, extrahierte zweimal mit Dichlormethan und klärte die wäßrige Lösung mit Kohle. Beim Einengen fiel 3-Amino-2,2-dimethyl-7-methoxy-4-chromanon-hydrochlorid aus. Schmelzpunkt: 224-226 °C.

### c) 3-(5-Chlor-2-methoxy-benzamido)-2,2-dimethyl-7-methoxy-4-chromanon

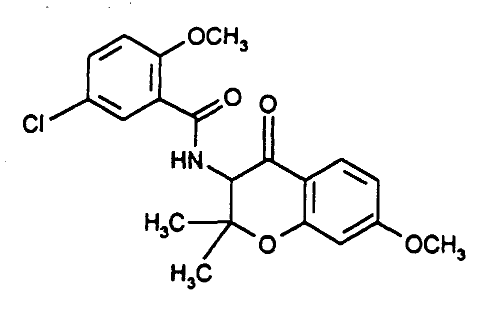

In eine Lösung von 33.5 g (0.13 mol) 3-Amino-2,2-dimethyl-7-methoxy-4-chromanon-hydrochlorid in 150 ml Pyridin wurden bei 10 °C 28.7 g (0.14 mol) 5-Chlor-2-methoxybenzoylchlorid eingetragen. Nach dreistündigem Rühren bei ca. 27°C wurde in Eis/Wasser eingetragen und zweimal mit Dichlormethan extrahiert. Die vereinigten Dichlormethanextrakte wurden zweimal mit 2 N Salzsäure und mit Wasser gewaschen, anschließend getrocknet, eingedampft und der Rückstand aus Ethanol/DMF umkristallisiert. Das 3-(5-Chlor-2-methoxy-benzamido)-2,2-dimethyl-7-methoxy-4-chromanon hatte einen Schmelzpunkt von 174 °C.

### d) 3-(5-Chlor-2-methoxy-benzamido)-2,2-dimethyl-7-methoxy-4-chromanol

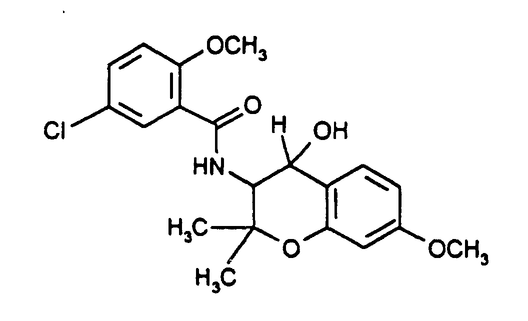

Eine Suspension von 25 g (64 mmol) 3-(5-Chlor-2-methoxy-benzamido)-2,2-dimethyl-7-methoxy-4-chromanon und 1.24 g (32 mmol) gepulvertem Natriumborhydrid in 100 ml Ethanol wurde 3 Stunden bei 50 °C gerührt, wobei der Feststoff sich auflöste. Anschließend wurde nach dem Abkühlen in salzsaures Eiswasser gegossen und mit Dichlormethan extrahiert. Die organische Lösung wurde mit Wasser gewaschen, getrocknet und eingedampft. Das erhaltene 3-(5-Chlor-2-methoxy-benzamido)-2,2-dimethyl-7-methoxy-4-chromanol (Diastereomerengemisch) schmolz ab 165 °C.

### e) 3-(5-Chlor-2-methoxy-benzamido)-2,2-dimethyl-7-methoxy-chroman

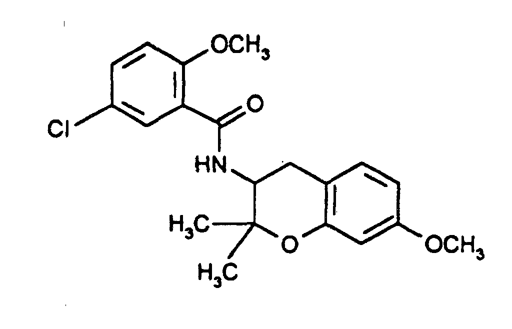

Zu 19.6 g (50 mmol) 3-(5-Chlor-2-methoxy-benzamido)-2,2-dimethyl-7-methoxy-4-chromanol in 120 ml Acetonitril wurden 44 g (300 mmol) Natriumiodid und 38 ml (300 mmol) Chlortrimethylsilan zugesetzt. Die Temperatur stieg vorübergehend auf 32 °C. Nach dreistündigem Rühren bei ca. 25 °C wurde in Eis/Wasser gegossen, mit konzentrierter Natriumbisulfitlösung entfärbt und mehrfach mit Dichlormethan extrahiert. Die vereinigten organischen Lösungen wurden mit Wasser gewaschen, getrocknet, eingedampft und der Rückstand über eine Kieselgelsäule mit Dichlormethan/Essigsäureethylester 95:5 chromatographiert. Das 3-(5-Chlor-2-methoxy-benzamido)-2,2-dimethyl-7-methoxy-chroman wurde als Öl enthalten.

### f) 3-(5-Chlor-2-methoxy-benzamido)-2,2-dimethyl-7-methoxy-6-sulfamoyl-chroman

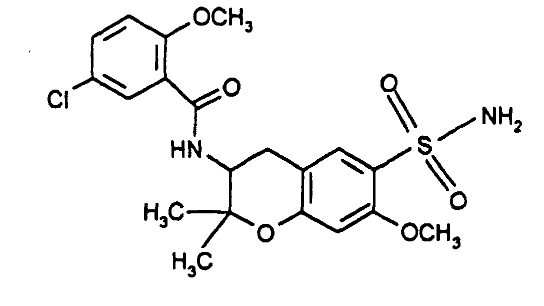

18 g (47.9 mmol) 3-(5-Chlor-2-methoxy-benzamido)-2,2-dimethyl-7-methoxychroman wurden auf unter 0 °C abgekühlt und mit 25 ml auf -15 °C gekühlter Chlorsulfonsäure versetzt. Beim Erwärmen auf 10 °C stieg die Temperatur schnell auf 35 °C an. Man kühlte auf 0 °C ab und rührte anschließend 2 Stunden bei 15 °C, trug in Eiswasser ein, saugte das Sulfochlorid ab und trug es in eine auf -10 °C gekühlte Mischung aus 350 ml Aceton und 75 ml konz. wäßrigen Ammoniak ein. Man ließ auf Zimmertemperatur kommen, rührte mehrere Stunden nach und engte die Lösung bei 30 °C im Vakuum ein. Unter Eiskühlung wurde der Rückstand mit konz. Salzsäure angesäuert. Der Niederschlag wurde abgesaugt, mit Wasser neutral gewaschen, getrocknet und aus Ethanol umkristallisiert. Das 3-(5-Chlor-2-methoxy-benzamido)-2,2-dimethyl-7-methoxy-6-sulfamoyl-chroman hatte einen Schmelzpunkt von 228 °C.

### Beispiel 16:

### 3-(5-Chlor-2-methoxy-benzamido)-2,2-dimethyl-6-(methylaminothiocarbonylaminosulfonyl)-7-methoxy-chroman

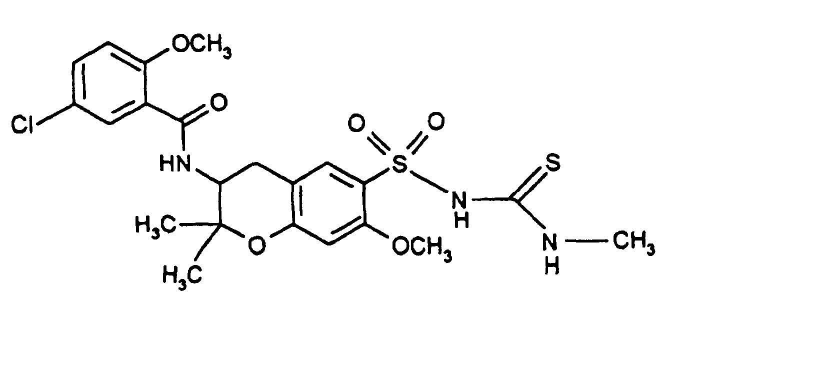

Zu einer Suspension von 1.14 g (2.5 mmol) 3-(5-Chlor-2-methoxy-benzamido)-2,2-dimethyl-6-sulfamoyl-7-methoxy-chroman und 1.04 g (7.5 mmol) fein gepulvertem Kaliumkarbonat in 10 ml DMSO wurden 0.26 g (3.5 mmol) Methylisothiocyanat zugefügt. Nach 25minütigem Rühren bei 80 °C wurde abgekühlt, in Eis/Wasser eingetragen, mit Kohle geklärt und auf pH 1 angesäuert. Der Niederschlag wurde abgesaugt und aus Methanol/DMF umkristallisiert. Das Produkt hatte einen Schmelzpunkt von 234-235 °C.

### Beispiel 17:

### 3-(5-Chlor-2-methoxy-benzamido)-2,2-dimethyl-6-(npropylaminothiocarbonylaminosulfonyl)-7-methoxy-chroman

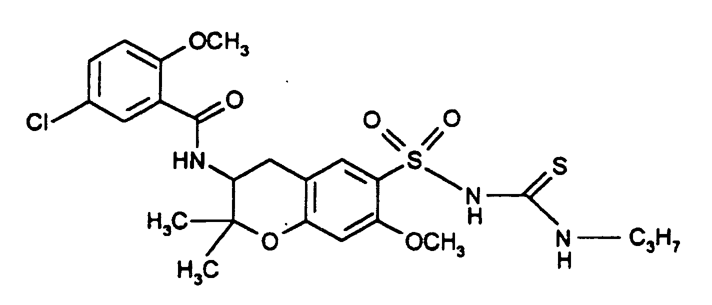

Die Substanz wurde analog Beispiel 16 mit 0.36 ml (3.5 mmol) n-Propylisothiocyanat an Stelle des Methylisothiocyanats hergestellt und hatte nach Umkristallisation aus Methanol/DMF einen Schmelzpunkt von 210-211 °C.

### Beispiel 18:

### 3-(5-Chlor-2-methoxy-benzamido)-2,2-dimethyl-6-(isopropylaminothiocarbonylaminosulfonyl)-7-methoxy-chroman

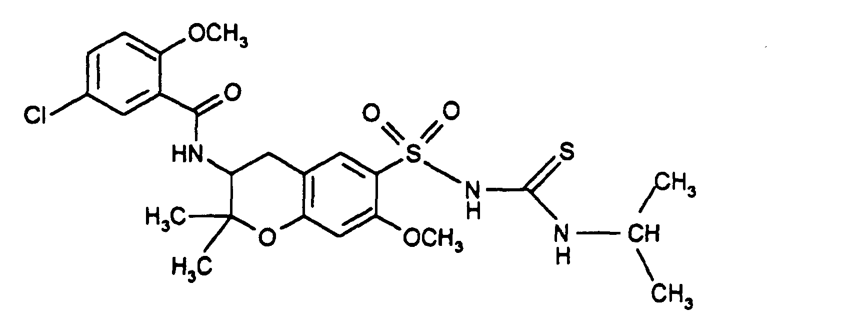

Die Substanz wurde analog Beispiel 16 mit 0.35 ml (3.5 mmol) Isopropylisothiocyanat an Stelle des Methylisothiocyanats hergestellt und hatte nach Umkristallisation aus Methanol/DMF einen Schmelzpunkt von 201-202 °C.

### Beispiel 19:

### 3-(5-Fluor-2-methoxy-benzamido)-2,2-dimethyl-6-(methylaminothiocarbonylaminosulfonyl)-7-methoxy-chroman

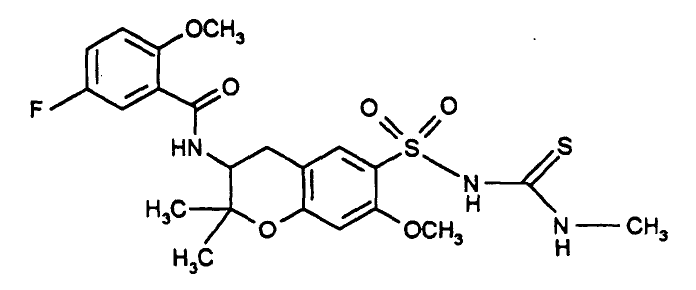

Zu einer Suspension von 1.1 g (2.5 mmol) 3-(5-Fluor-2-methoxy-benzamido)-2,2-dimethyl-7-methoxy-6-sulfamoyl-chroman und 1.04 g (7.5 mmol) fein gepulvertem Kaliumkarbonat in 10 ml DMSO wurden 0.26 g (3.5 mmol) Methylisothiocyanat zugefügt. Nach 25minütigem Rühren bei 80 °C wurde abgekühlt, in Eis/Wasser eingetragen, mit Kohle geklärt und auf pH 1 angesäuert. Der Niederschlag wurde abgesaugt und aus Ethanol/DMF umkristallisiert. Das Produkt hatte einen Schmelzpunkt von 222 °C.

Herstellung der Ausgangsverbindung 3-(5-Fluor-2-methoxy-benzamido)-2,2-dimethyl-6-sulfamoyl-7-methoxy-chroman

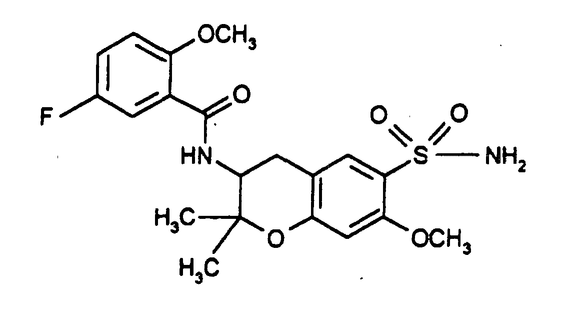

### a) 3-(5-Fluor-2-methoxy-benzamido)-2,2-dimethyl-7-methoxy-4-chromanon

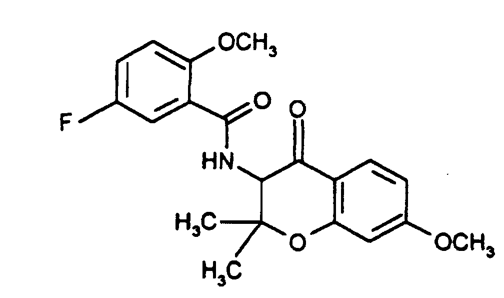

Die Substanz wurde analog Beispiel 15 c mit 26.4 g (0.14 mol) 5-Fluor-2-methoxybenzoylchlorid an Stelle des 5-Chlor-2-methoxybenzoylchlorids hergestellt und hatte nach Umkristallisation aus Ethanol/DMF einen Schmelzpunkt von 143-144°C.

### b) 3-(5-Fluor-2-methoxy-benzamido)-2,2-dimethyl-7-methoxy-4-chromanol

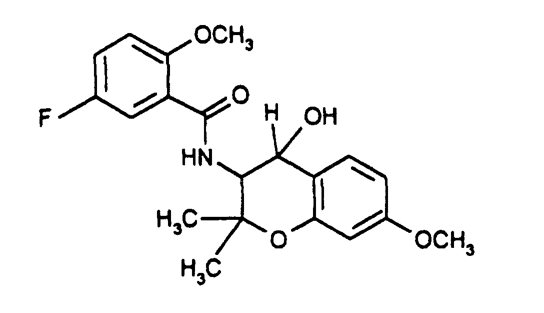

Die Substanz wurde analog Beispiel 15 d unter Verwendung von 23.9 g (64 mmol) 3-(5-Fluor-2-methoxy-benzamido)-2,2-dimethyl-7-methoxy-4-chromanon hergestellt. Nach der Reduktion mit Natriumborhydrid erhielt man 3-(5-Fluor-2-methoxybenzamido)-2,2-dimethyl-7-methoxy-4-chromanol als Diastereomerengemisch vom Schmelzpunkt 156-157 °C.

### c) 3-(5-Fluor-2-methoxy-benzamido)-2,2-dimethyl-7-methoxy-4-chroman

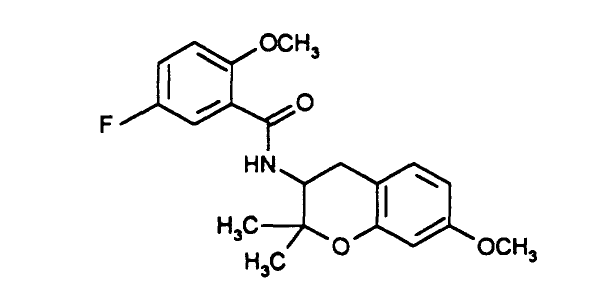

Es wurde eine Reduktion analog Beispiel 15 e unter Verwendung von 18.8 g (50 mmol) 3-(5-Fluor-2-methoxy-benzamido)-2,2-dimethyl-7-methoxy-4-chromanol durchgeführt. Nach Chromatographie an einer Kieselgelsäule mit Dichlormethan/Essigsäureethylester 95:5 erhielt man das 3-(5-Fluor-2-methoxybenzamido)-2,2-dimethyl-7-methoxy-chroman als Öl.

### d) 3-(5-Fluor-2-methoxy-benzamido)-2,2-dimethyl-7-methoxy-6-sulfamoyl-chroman

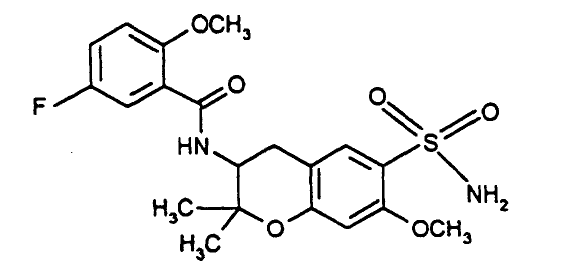

Die Substanz wurde analog Beispiel 15 f unter Verwendung von 17.2 g (47.9 mmol) 3-(5-Fluor-2-methoxy-benzamido)-2,2-dimethyl-7-methoxy-4-chroman hergestellt und hatte nach Umkristallisation aus Ethanol einen Schmelzpunkt von 159-160 °C.

### Beispiel 20:

### 3-(5-Fluor-2-methoxy-benzamido)-2,2-dimethyl-6-(ethylaminothiocarbonylaminosulfonyl)-7-methoxy-chroman

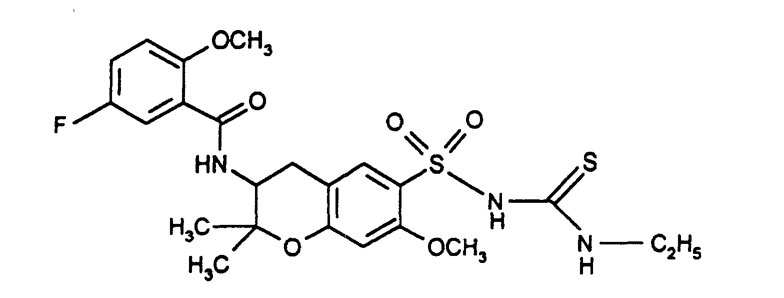

Die Substanz wurde analog Beispiel 19 mit 0.31 ml (35 mmol) Ethylisothiocyanat an Stelle des Methylisothiocyanats hergestellt und hatte einen Schmelzpunkt von 211 °C.

### Beispiel 21:

### 3-(5-Fluor-2-methoxy-benzamido)-2,2-dimethyl-6-(isopropylaminothiocarbonylaminosulfonyl)-7-methoxy-chroman

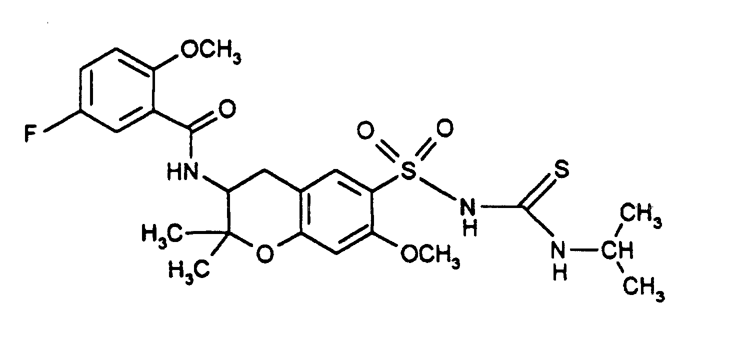

Die Substanz wurde analog Beispiel 19 mit 0.35 ml (35 mmol) Isopropylisothiocyanat an Stelle des Methylisothiocyanats hergestellt und hatte einen Schmelzpunkt von 156-157 °C.

### Beispiel 22:

### 3-(3-Ethyl-4-methyl-2-oxo-3-pyrrolin-1-carboxamido)-6-(methylaminothiocarbonylaminosulfonyl)-7-methoxy-chroman

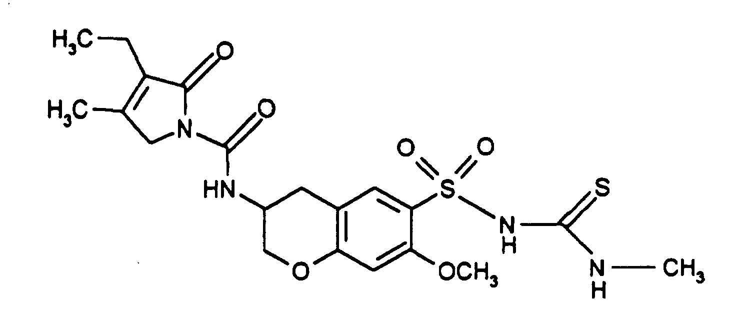

2.05 g (5 mmol) 3-(3-Ethyl-4-methyl-2-oxo-3-pyrrolin-1-carboxamido)-6-sulfamoyl-7-methoxy-chroman, 2.07 g (15 mmol) fein gepulvertes Kaliumkarbonat und 0.44 g (6 mmol) Methylisothiocyanat wurden in 20 ml DMSO suspendiert bzw. gelöst. Das Reaktionsgemisch wurde eine Stunde bei 80 °C gerührt. Der Ansatz wurde auf Eiswasser gegossen und das Produkt durch Ansäuern mit Salzsäure gefällt. Nach Absaugen und Trocknen wurde das Rohprodukt durch Chromatographie an Kieselgel (Elutionsmittel Methylenchlorid/Eisessig 19:1) gereinigt. Das Produkt hatte einen Schmelzpunkt von 205 °C.

Herstellung der Ausgangsverbindung 3-(3-Ethyl-4-methyl-2-oxo-3-pyrrolin-1-carboxamido)-6-sulfamoyl-7-methoxy-chroman

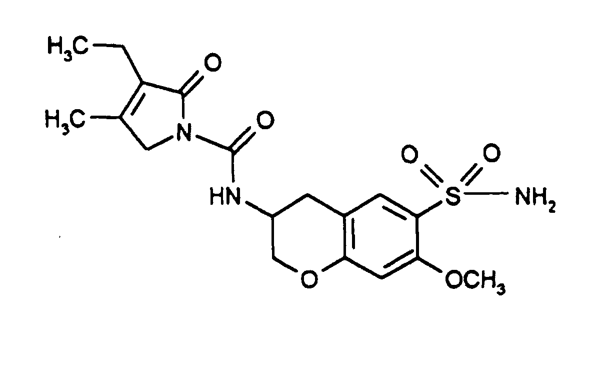

Zu einer Lösung von 8.2 g (46 mmol) 3-Amino-7-methoxy-chroman in 60 ml THF wurden 8.43 g (52 mmol) N,N'-Carbonyldiimidazol zugesetzt. Die Lösung erwärmte sich dabei. Nach einstündigem Rühren bei Zimmertemperatur wurde im Vakuum eingedampft. Der Rückstand wurde mit 6.51 g (52 mmol) 3-Ethyl-4-methyl-3-pyrrolin-2-on bei 160-170 °C 1.5 bis 2 Stunden zusammengeschmolzen und anschließend an Kieselgel mit dem Elutionsmittel Essigester/Petrolether 3:1 chromatographiert. Die Hauptfraktion wurde eingedampft und der Rückstand wurde aus Methanol umkristallisiert. Man erhielt 3-(3-Ethyl-4-methyl-2-oxo-3-pyrrolin-1-carboxamido)-7-methoxy-chroman von Schmelzpunkt 118-119°C. Dieses Produkt wurde nach der üblichen Vorgehensweise in auf -15 °C gekühlte Chlorsulfonsäure eingetragen. Man ließ auf Zimmertemperatur kommen und rührte 1 Stunde nach. Nach üblicher Aufarbeitung wurde das Sulfochlorid wie in Beispiel 1 beschrieben in das Sulfonamid umgewandelt. 3-(3-Ethyl-4-methyl-2-oxo-3-pyrrolin-1-carboxamido)-6-sulfamoyl-7-methoxy-chroman hatte einen Schmelzpunkt von 225-227 °C.

### Beispiel 23:

### 3-(3-Ethyl-4-methyl-2-oxo-3-pyrrolin-1-carboxamido)-6-(methylaminocarbonylaminosulfonyl)-7-methoxy-chroman

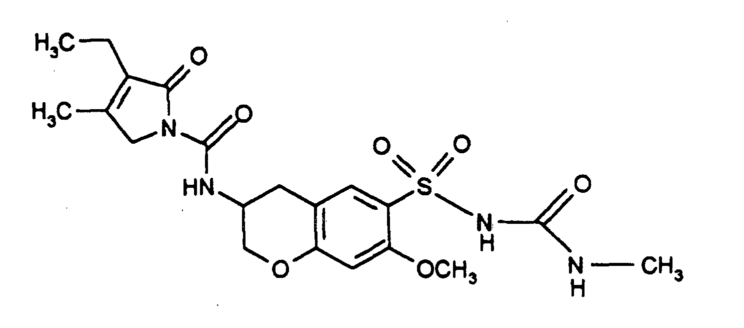

1 g 3-(3-Ethyl-4-methyl-2-oxo-3-pyrrolin-1-carboxamido)-6-(methylaminothiocarbonylaminosulfonyl)-7-methoxy-chroman (Beispiel 22) wurden in 20 ml kalter 0.5 N Natronlauge suspendiert bzw. gelöst. In der Kälte (-4 bis 0 °C) wurde 1 ml 37 %ige Wasserstoffperoxidlösung zugegeben. Der Ansatz wurde 1.5 Stunden bei 0 °C gerührt. Das Rohprodukt wurde durch Zugabe von 2 N HCl gefällt und anschließend an Kieselgel (Elutionsmittel Methylenchlorid/Eisessig 9:1) gereinigt. Das Produkt hatte einen Schmelzpunkt von 245-246 °C.

### Pharmakologische Daten

In den folgenden Modellen wurden die therapeutischen Eigenschaften der Verbindungen der Formel I nachgewiesen:

### Test 1: Aktionspotentialdauer am Papillarmuskel des Meerschweinchens

### (a) Einleitung

ATP-Mangelzustände, wie sie während einer Ischämie in der Herzmuskelzelle beobachtet werden, führen zu einer Verkürzung der Aktionspotentialdauer. Sie gelten als eine der Ursachen für sogenannte Reentry-Arrhythmien, die den plötzlichen Herztod verursachen können. Die Öffnung von ATP-sensitiven Kaliumkanälen durch das Absinken von ATP gilt hierfür als ursächlich.

### (b) Methode

Zur Messung des Aktionspotentials wurde eine Standard-Mikroelektroden-Technik eingesetzt. Hierfür wurden Meerschweinchen beiderlei Geschlechts durch Schlag auf den Kopf getötet, die Herzen entnommen, die Papillarmuskeln herausgetrennt und in einem Organbad aufgehängt. Das Organbad wurde mit Ringerlösung (0.9 % NaCl, 0.048 % KCl, 0.024 % CaCl₂, 0.02 % NaHCO₃, und 0.1 % Glucose) durchspült und mit einer Mischung aus 95 % Sauerstoff und 5 % Kohlendioxid bei einer Temperatur von 36 °C begast. Der Muskel wurde über eine Elektrode mit Rechteck-Impulsen von 1 V und 1 ms Dauer und einer Frequenz von 2 Hz angeregt. Das Aktionspotential wurde durch eine intrazellulär eingestochene Glas-Mikroelektrode, die mit 3 M KCl-Lösung gefüllt war, abgeleitet und registriert. Die zu prüfenden Substanzen wurden der Ringerlösung in einer Konzentration von 2 × 10⁻⁶ mol pro Liter oder 2 × 10⁻⁵ mol pro Liter zugesetzt. Das Aktionspotential wurde mit einem Amplifier von Hugo Sachs verstärkt auf einem Oszilloskop dargestellt. Die Dauer des Aktionspotentials in Millisekunden (ms) wurde bei einem Repolarisierungsgrad von 95 % (APD₉₅) bestimmt. Aktionspotentialverkürzungen wurden durch Zugabe einer Lösung des Kaliumkanalöffners HOE 234 (Rilmakalim) (W. Linz, E. Klaus, U. Albus, R.H.A. Becker, D. Mania, H.C. Englert, B.A. Schölkens, Arzneimittelforschung/Drug Research, Band 42 (II), 1992, 1180-1185) ausgelöst, wobei eine Konzentration an HOE 234 in der Badlösung von 1 µg/ml eingestellt wurde. Die Testsubstanzen wurden als Stammlösungen in Propandiol der Badlösung zugesetzt. Die angegebenen Werte beziehen sich auf Messungen 30 Minuten nach der Zugabe. Als Kontrolle wurde der APD₉₅-Wert in Gegenwart von HOE 234 und in Abwesenheit der Testsubstanz bestimmt.

### (c) Resultate

Folgende Werte wurden gemessen:

| Verbindung | Konzentration | APD₉₅ - HOE 234 (ms) |
|---|---|---|
| Kontrolle | | < 40 |
| Beispiel 1 | 20 µmol/l | 157 ± 36 (158 ± 12) (n = 3) |
| Beispiel 3 | 20 µmol/l | 134 ± 9 (178 ± 8) (n = 3) |
| Beispiel 6 | 2 µmol/l | 145 ± 19.1 (187 ± 10.2) (n = 3) |
| Beispiel 7 | 2 µmol/l | 130 ± 28.1 (173 ± 13.1) (n = 3) |
| Beispiel 21 | 2 µmol/l | 67 ± 10 (149 ± 3) (n = 3) |

Den Meßwerten (Mittelwerten aus n Versuchen) sind die korrespondierenden Leerwerte in Klammern nachgestellt. Die Leerwerte sind die APD₉₅-Werte, die zu Versuchsbeginn ohne HOE 234 und Testsubstanz in der Ringerlösung gemessen wurden. Die gefundenen Werte zeigen die normalisierende Wirkung der erfindungsgemäßen Substanzen auf eine verkürzte Aktionspotentialdauer.

### Test 2: Membranpotential an isolierten β-Zellen

### (a) Einleitung

Der Wirkmechanismus der blutzuckersenkenden Sulfonylharnstoffe ist in groben Zügen aufgeklärt. Zielorgan der Sulfonylharnstoffe sind die β-Zellen des Pankreas, wo sie durch durch eine Beeinflussung des elektrischen Potentials der Zellmembran eine Freisetzung des blutzuckersenkenden Hormons Insulin herbeiführen. Ein hypoglykämischer Sulfonylharnstoff, z. B. Glibenclamid, bewirkt eine Depolarisation der Zellmembran, die zu einem vermehrten Einstrom von Calciumionen und im Gefolge davon zu einer Insulinfreisetzung führt. Das Ausmaß ΔU dieser Depolarisation der Zellmembran, das durch die erfindungsgemäßen Substanzen verursacht wird, wurde an insulinsezemierenden RINm5F-Zellen, einer Pankreastumorzellinie, bestimmt. Die Wirkstärke einer Verbindung in diesem Modell sagt das Ausmaß des blutzuckersenkenden Potentials dieser Verbindung voraus.

### (b) Methode

Zellkultur von RINm5F-Zellen: RINm5F-Zellen wurden in RPMI 1640-Kulturmedium (Flow), dem 11 mM Glukose, 10% (vol/vol) fötales Kälberserum, 2 mM Glutamin und 50 µg/ml Gentamycin zugesetzt wurden, bei 37°C kultiviert. Die Zellen wurden alle 2 bis 3 Tage auf Petrischalen ausgesät und in einer befeuchteten Atmosphäre von 95 % O₂ und 5 % CO₂ bei einer Temperatur von 37 °C gehalten. Für die Untersuchungen wurden die Zellen durch Inkubation (ca. 3 min) in einem Ca²⁺freien Medium, das 0.25% Trypsin enthielt, isoliert.

Meßmethode: Isolierte RINm5F-Zellen wurden in eine Plexiglaskammer auf einem inversen Mikroskop, das mit Differential-Interferenz-Kontrast-Optik ausgerüstet war, gebracht. Unter optischer Kontrolle (400-fache Vergrößerung) wurde eine feuerpolierte Mikropipette mit einem Öffnungsdurchmessser von etwa 1 µm mit Hilfe eines Mikromanipulators auf die Zelle aufgesetzt. Durch Anlegen eines leichten Unterdruckes an das Innere der Patch-Pipette wurde zunächst eine hohe elektrische Abdichtung zwischen Glas und Zellmembran hergestellt. Anschließend wurde durch Erhöhung des Unterdruckes der Membranfleck unter der Meßpipette aufgerissen. In dieser Ganzzell-Konfiguration wurde mit Hilfe eines Patch-Clamp-Verstärkers (UM EPC 7, List, Darmstadt) das Zellpotential registriert und durch Anlegen einer Spannungsrampe der Ganzzell-Strom gemessen. Die Patch-Pipette war mit KCl-Lösung gefüllt, die (in mmol pro Liter) enthielt: 140 KCl, 10 NaCl, 1.1 MgCl₂, 0.5 EGTA, 1 Mg-ATP, 10 HEPES, und die einen pH-Wert von 7.2 hatte. Im Bad befand sich NaCl-Lösung, die (in mmol pro Liter) enthielt: 140 NaCl, 4.7 KCl, 1.1 MgCl₂, 2 CaCl₂, 10 HEPES, und die pH-Wert von 7.4 hatte. Von den Testsubstanzen wurden Stammlösungen (Konzentration 100 mmol/l) in Dimethylsulfoxid (DMSO) und entsprechende Verdünnungen in NaCl-Lösung hergestellt. DMSO alleine hatte keinen Effekt auf das Zellpotential. Um das Zellpotential zu stabilisieren, wurde Diazoxid (100 µmol/l), ein Öffner für ATP-sensitive K⁺-Kanäle, bei allen Versuchen der Badlösung zugesetzt. Alle Experimente wurden bei 34 ± 1°C durchgeführt.

### (c) Resultate

Folgende Werte ΔU (durch den Zusatz der Testsubstanzen bewirkten Änderungen der Zellpotentiale) wurden gemessen. Die Kontrollwerte in Klammern sind die Zellpotentiale U vor der Zugabe der Testsubstanzen. Zum Vergleich sind die Werte angegeben, die in diesem Test mit Glibenclamid, einem typischen hypoglykämischen Benzolsulfonylharnstoff, erhalten wurden. Die gefundenen Werte zeigen, daß die erfindungsgemäßen Substanzen keine oder nur eine geringe hypoglykämische Wirkung haben.

| Verbindung | Konzentration | ΔU (mV) |
|---|---|---|
| Beispiel 1 | 1 µmol/l | 6 (Kontrolle: -74 mV) |
| Beispiel 1 | 10 µmol/l | 24 (Kontrolle: -74 mV) |
| Beispiel 3 | 1 µmol/l | 18 (Kontrolle: -69 mV) |
| Beispiel 3 | 10 µmol/l | 23 (Kontrolle: -69 mV) |
| Beispiel 6 | 1 µmol/l | 3 (Kontrolle: -76 mV) |
| Beispiel 7 | 1 µmol/l | 29 (Kontrolle: -78 mV) |
| Glibenclamid | 1 µmol/l | 47 (Kontrolle: -73 mV) |
| Glibenclamid | 10 µmol/l | 46 (Kontrolle: -73 mV) |

## Patentansprüche

1. Verbindungen der Formel I, 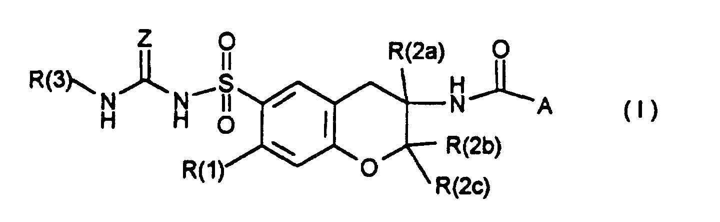 in der
R(1) Wasserstoff, Alkyl mit 1, 2, 3 oder 4 C-Atomen, Alkoxy mit 1, 2, 3 oder 4 C-Atomen, Alkoxyalkoxy mit unabhängig voneinander 1, 2, 3 oder 4 C-Atomen in jeder der beiden Alkoxyeinheiten, Alkylmercapto mit 1, 2, 3 oder 4 C-Atomen, Fluor, Chlor, Brom, Iod oder Trifluormethyl bedeutet;
R(2a), R(2b) und R(2c), die gleich oder verschieden sind, Wasserstoff oder Alkyl mit 1 oder 2 C-Atomen bedeuten;
R(3) Wasserstoff oder Alkyl mit 1, 2, 3 oder 4 C-Atomen bedeutet;
Z Schwefel oder Sauerstoff bedeutet;
A Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist,
oder
A den Rest eines gesättigten oder ungesättigten Lactams der Formel 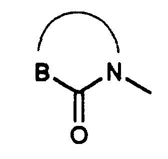 bedeutet, in der B Alkenylen oder Alkylen mit 3, 4, 5 oder 6 C-Atomen bedeutet, das unsubstituiert ist oder durch bis zu drei gleiche oder verschiedene Alkylgruppen mit 1, 2, 3 oder 4 C-Atomen substituiert ist,
oder
A den Rest eines bicyclisches Systems der Formeln 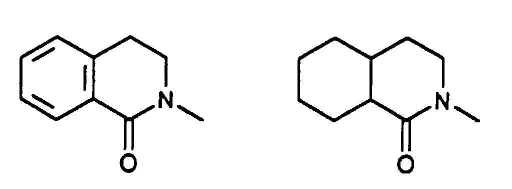 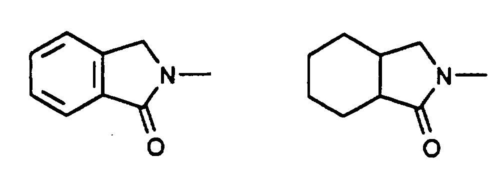 bedeutet;
in allen ihren stereoisomeren Formen und Gemische davon in allen Verhältnissen; sowie ihre physiologisch unbedenklichen Salze.

2. Verbindungen der Formel I nach Anspruch 1, in der
R(1) Wasserstoff, Alkyl mit 1 oder 2 C-Atomen, Alkoxy mit 1, 2, 3 oder 4 C-Atomen, Alkylmercapto mit 1, 2, 3 oder 4 C-Atomen, Fluor, Chlor, Brom, Iod oder Trifluormethyl bedeutet;
R(2a), R(2b) und R(2c), die gleich oder verschieden sind, Wasserstoff oder Alkyl mit 1 oder 2 C-Atomen bedeuten;
R(3) Wasserstoff oder Alkyl mit 1, 2, 3 oder 4 C-Atomen bedeutet;
Z Schwefel oder Sauerstoff bedeutet;
A Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist,
oder
A den Rest eines gesättigten oder ungesättigten Lactams der Formel 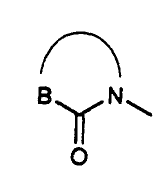 bedeutet, in der B Alkenylen oder Alkylen mit 3, 4, 5 oder 6 C-Atomen bedeutet, das unsubstituiert ist oder durch bis zu drei gleiche oder verschiedene Alkylgruppen mit 1, 2, 3 oder 4 C-Atomen substituiert ist,
oder
A den Rest eines bicyclischen Systems der Formeln 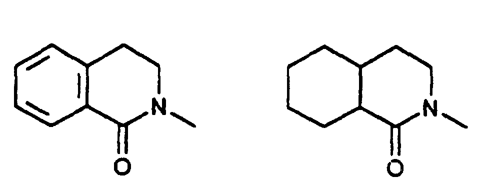 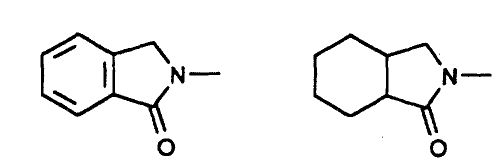 bedeutet;
in allen ihren stereoisomeren Formen und Gemische davon in allen Verhältnissen; sowie ihre physiologisch unbedenklichen Salze.

3. Verbindungen der Formel I nach Anspruch 1 und/oder 2, in der
R(1) Wasserstoff, Alkyl mit 1 oder 2 C-Atomen, Alkoxy mit 1 oder 2 C-Atomen, Alkylmercapto mit 1 oder 2 C-Atomen, Fluor, Chlor, Brom, Iod oder Trifluormethyl bedeutet;
R(2a) Wasserstoff bedeutet und R(2b) und R(2c) Wasserstoff oder Methyl bedeuten;
R(3) Wasserstoff oder Alkyl mit 1, 2, 3 oder 4 C-Atomen bedeutet;
Z Schwefel oder Sauerstoff bedeutet;
A Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist,
oder
A den Rest eines gesättigten oder ungesättigten Lactams der Formel 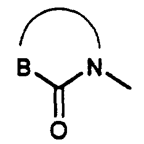 bedeutet, in der B Alkenylen oder Alkylen mit 3, 4, 5 oder 6 C-Atomen bedeutet, das unsubstituiert ist oder durch bis zu drei gleiche oder verschiedene Alkylgruppen mit 1, 2, 3 oder 4 C-Atomen substituiert ist,
oder
A den Rest eines bicyclischen Systems der Formeln 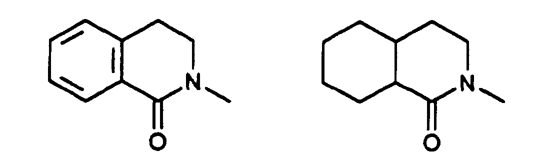 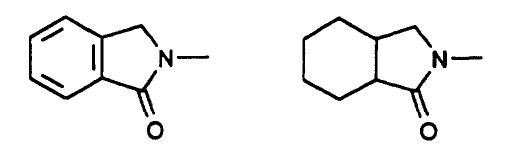 bedeutet;
in allen ihren stereoisomeren Formen und Gemische davon in allen Verhältnissen; sowie ihre physiologisch unbedenklichen Salze.

4. Verbindungen der Formel I nach einem oder mehreren der Ansprüche 1 bis 3, in der
R(1) Wasserstoff, Alkyl mit 1 oder 2 C-Atomen, Alkoxy mit 1 oder 2 C-Atomen oder Alkylmercapto mit 1 oder 2 C-Atomen bedeutet;
R(2a), R(2b) und R(2c) Wasserstoff bedeuten;
R(3) Wasserstoff oder Alkyl mit 1, 2, 3 oder 4 C-Atomen bedeutet;
Z Schwefel bedeutet;
A Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist,
oder
A den Rest eines gesättigten oder ungesättigten Lactams der Formel 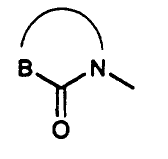 bedeutet, in der B Alkenylen oder Alkylen mit 3, 4, 5 oder 6 C-Atomen bedeutet, das unsubstituiert ist oder durch bis zu drei gleiche oder verschiedene Alkylgruppen mit 1, 2, 3 oder 4 C-Atomen substituiert ist,
oder
A den Rest eines bicyclischen Systems der Formeln 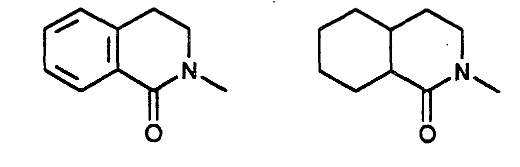 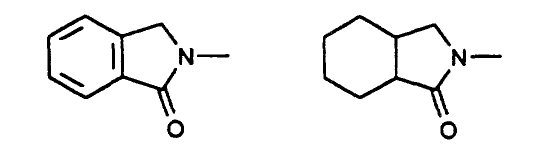 bedeutet;
in allen ihren stereoisomeren Formen und Gemische davon in allen Verhältnissen; sowie ihre physiologisch unbedenklichen Salze.

5. Verbindungen der Formel I nach einem oder mehreren der Ansprüche 1 bis 4, in der
R(1) Wasserstoff, Alkyl mit 1 oder 2 C-Atomen, Alkoxy mit 1 oder 2 C-Atomen oder Alkylmercapto mit 1 oder 2 C-Atomen bedeutet;
R(2a), R(2b) und R(2c) Wasserstoff bedeuten;
R(3) Wasserstoff, Methyl oder Ethyl bedeutet;
Z Schwefel bedeutet;
A Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist,
oder
A den Rest eines gesättigten oder ungesättigten Lactams der Formel 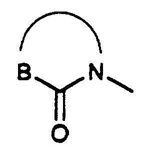 bedeutet, in der B Alkenylen oder Alkylen mit 3, 4, 5 oder 6 C-Atomen bedeutet, das unsubstituiert ist oder durch bis zu drei gleiche oder verschiedene Alkylgruppen mit 1, 2, 3 oder 4 C-Atomen substituiert ist;
in allen ihren stereoisomeren Formen und Gemische davon in allen Verhältnissen; sowie ihre physiologisch unbedenklichen Salze.

6. Verbindungen der Formel I nach einem oder mehreren der Ansprüche 1 bis 5, in der
R(1) Wasserstoff, Alkyl mit 1 oder 2 C-Atomen, Alkoxy mit 1 oder 2 C-Atomen oder Alkylmercapto mit 1 oder 2 C-Atomen bedeutet;
R(2a), R(2b) und R(2c) Wasserstoff bedeuten;
R(3) Wasserstoff, Methyl oder Ethyl bedeutet;
Z Schwefel bedeutet;
A Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist:
in allen ihren stereoisomeren Formen und Gemische davon in allen Verhältnissen; sowie ihre physiologisch unbedenklichen Salze.

7. Verbindungen der Formel I nach einem oder mehreren der
R(1) Wasserstoff, Alkyl mit 1 oder 2 C-Atomen, Alkoxy mit 1 oder 2 C-Atomen oder Alkylmercapto mit 1 oder 2 C-Atomen bedeutet;
R(2a), R(2b) und R(2c) Wasserstoff bedeuten;
R(3) Wasserstoff oder Alkyl mit 1, 2, 3 oder 4 C-Atomen bedeutet;
Z Sauerstoff bedeutet;
A Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist,
oder
A den Rest eines gesättigten oder ungesättigten Lactams der Formel 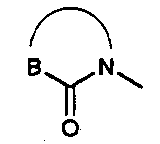 bedeutet, in der B Alkenylen oder Alkylen mit 3, 4, 5 oder 6 C-Atomen bedeutet, das unsubstituiert ist oder durch bis zu drei gleiche oder verschiedene Alkylgruppen mit 1, 2, 3 oder 4 C-Atomen substituiert ist,
oder
A den Rest eines bicyclischen Systems der Formeln 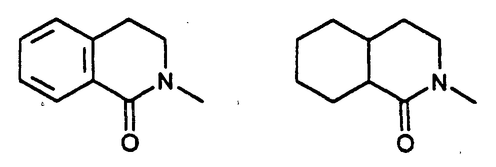 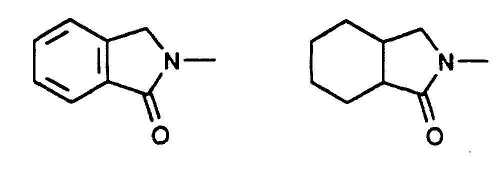 bedeutet;
in allen ihren stereoisomeren Formen und Gemische davon in allen Verhältnissen; sowie ihre physiologisch unbedenklichen Salze.

8. Verbindungen der Formel I nach einem oder mehreren der Ansprüche 1, 2, 3 und 7, in der
R(1) Wasserstoff, Alkyl mit 1 oder 2 C-Atomen, Alkoxy mit 1 oder 2 C-Atomen oder Alkylmercapto mit 1 oder 2 C-Atomen bedeutet;
R(2a), R(2b) und R(2c) Wasserstoff bedeuten;
R(3) Wasserstoff, Methyl oder Ethyl bedeutet;
Z Sauerstoff bedeutet;
A Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist,
oder
A den Rest eines gesättigten oder ungesättigten Lactams der Formel 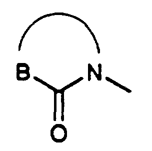 bedeutet, in der B Alkenylen oder Alkylen mit 3, 4, 5 oder 6 C-Atomen bedeutet, das unsubstituiert ist oder durch bis zu drei gleiche oder verschiedene Alkylgruppen mit 1, 2, 3 oder 4 C-Atomen substituiert ist;
in allen ihren stereoisomeren Formen und Gemische davon in allen Verhältnissen; sowie ihre physiologisch unbedenklichen Salze.

9. Verbindungen der Formel I nach einem oder mehreren der Ansprüche 1, 2, 3, 7 und 8, in der
R(1) Wasserstoff, Alkyl mit 1 oder 2 C-Atomen, Alkoxy mit 1 oder 2 C-Atomen oder Alkylmercapto mit 1 oder 2 C-Atomen bedeutet;
R(2a), R(2b) und R(2c) Wasserstoff bedeuten;
R(3) Wasserstoff, Methyl oder Ethyl bedeutet;
Z Sauerstoff bedeutet;
A Phenyl bedeutet, das unsubstituiert ist oder durch bis zu drei gleiche oder verschiedene Substituenten, ausgewählt aus der Gruppe bestehend aus Halogen, Alkyl mit 1 oder 2 C-Atomen und Alkoxy mit 1 oder 2 C-Atomen, substituiert ist;
in allen ihren stereoisomeren Formen und Gemische davon in allen Verhältnissen; sowie ihre physiologisch unbedenklichen Salze.

10. Verfahren zur Herstellung von Verbindungen der Formel I nach einem oder mehreren der Ansprüche 1 bis 9, **dadurch gekennzeichnet, daß** man Chromanylsulfonamide der Formel III 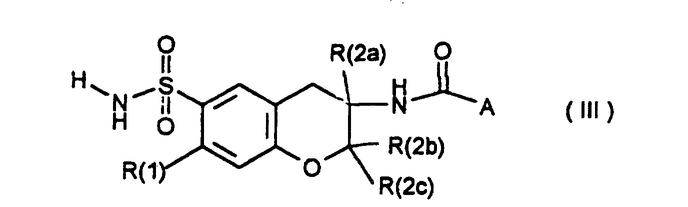 oder deren Salze der Formel IV, 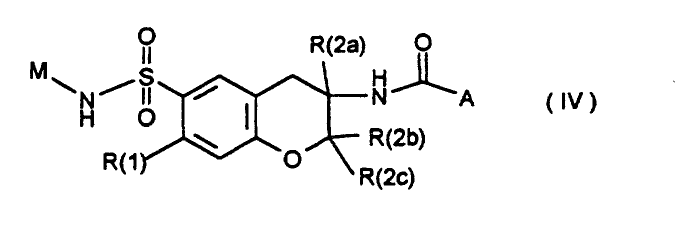 in denen die Reste die in den Ansprüchen 1 bis 9 angegebenen Bedeutungen haben und das Kation M für ein Alkalimetall- oder Erdalkalimetallion oder ein Ammoniumion steht, mit einem R(3)-substituierten Isocyanat oder R(3)-substituierten Isothiocyanat, mit einem R(3)-substituierten Kohlensäurederivat oder, zur Herstellung von Verbindungen der Formel I, in der Z für Sauerstoff steht, mit einem am Stickstoff R(3)-substituierten Trichloracetamid umsetzt;
oder, zur Herstellung von Verbindungen der Formel I, in der R(3) für Wasserstoff steht, Verbindungen der Formeln III oder IV mit einem Trialkylsilyliso(thio)cyanat oder Siliciumtetraiso(thio)cyanat umsetzt und die primären Silicium-substituierten Chromanylsulfonyl(thio)harnstoffe spaltet;
oder, zur Herstellung von Verbindungen der Formel I, in der Z für Sauerstoff steht, Verbindungen der Formel VIII, 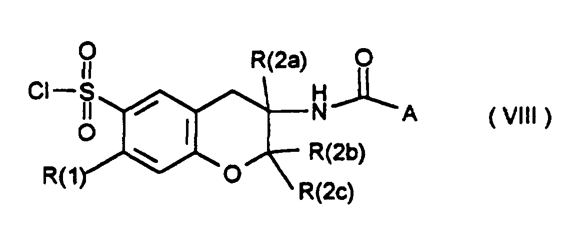 in der die Reste die in den Ansprüchen 1 bis 9 angegebenen Bedeutungen haben, mit einem R(3)-substituierten Harnstoff oder einem R(3)-substituierten Bis(trialkylsilyl)harnstoff umsetzt;
oder Verbindungen der Formeln IX oder X, 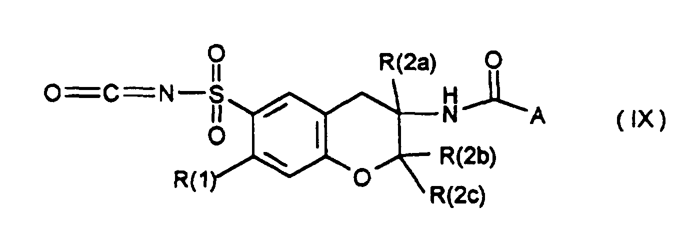 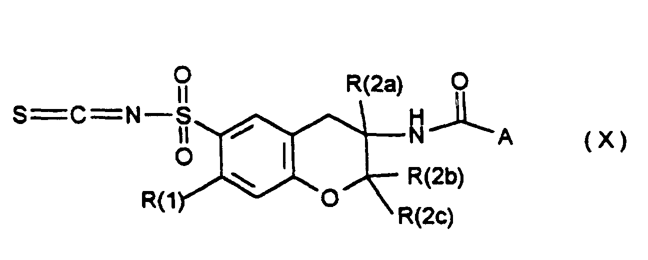 in denen die Reste die in den Ansprüchen 1 bis 9 angegebenen Bedeutungen haben, mit einem Amin der Formel R(3)-NH₂ umsetzt, in der R(3) die in den Ansprüchen 1 bis 9 angegebenen Bedeutungen hat;
oder, zur Herstellung von Verbindungen der Formel I, in der Z für Sauerstoff steht, Verbindungen der Formel I, in der Z für Schwefel steht, entschwefelt.

11. Verbindung der Formel I nach einem oder mehreren der Ansprüche 1 bis 9 und/oder ihre physiologisch unbedenklichen Salze zur Verwendung als Arzneimittel.

12. Verwendung von Verbindungen der Formel I nach einem oder mehreren der Ansprüche 1 bis 9 und/oder ihrer physiologisch unbedenklichen Salze zur Herstellung eines Medikaments zur Behandlung oder Prophylaxe von Störungen des cardiovaskulären Systems, von cerebralen Gefäßerkrankungen, von ischämischen Zuständen des Herzens, von einer geschwächten Herzkraft, oder zur Verbesserung der Herzfunktion nach Herztransplantationen.

13. Verwendung von Verbindungen der Formel I nach einem oder mehreren der Ansprüche 1 bis 9 und/oder ihrer physiologisch unbedenklichen Salze zur Herstellung eines Medikaments zur Behandlung oder Prophylaxe von Herzrhythmusstörungen oder zur Verhinderung des plötzlichen Herztodes.

14. Pharmazeutische Zubereitung, **dadurch gekennzeichnet, daß** sie eine wirksame Menge mindestens einer Verbindung der Formel I nach einem oder mehreren der Ansprüche 1 bis 9 und/oder eines physiologisch unbedenklichen Salzes davon neben pharmazeutisch einwandfreien Trägerstoffen und/oder Hilfsstoffen enthält.

## Claims

1. A compound of the formula I 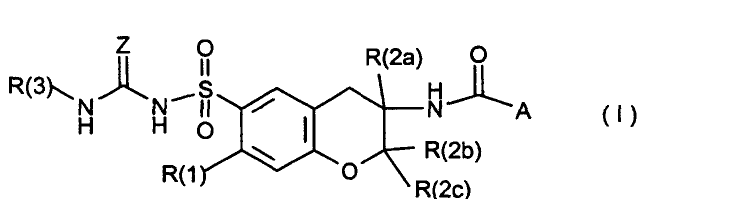 in which
R(1) is hydrogen, alkyl having 1, 2, 3 or 4 carbon atoms, alkoxy having 1, 2, 3 or 4 carbon atoms, alkoxyalkoxy having, independently of one another, 1, 2, 3 or 4 carbon atoms in each of the two alkoxy units, alkylmercapto having 1, 2, 3 or 4 carbon atoms, fluorine, chlorine, bromine, iodine or trifluoromethyl;
R(2a), R(2b) and R(2c), which are identical or different, are hydrogen or alkyl having 1 or 2 carbon atoms;
R(3) is hydrogen or alkyl having 1, 2, 3 or 4 carbon atoms;
Z is sulfur or oxygen;
A is phenyl which is unsubstituted or substituted by up to three identical or different substituents selected from the group consisting of halogen, alkyl having 1 or 2 carbon atoms and alkoxy having 1 or 2 carbon atoms,
or
A is the radical of a saturated or unsaturated lactam of the formula 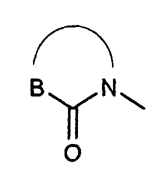 in which B is alkenylene or alkylene having 3, 4, 5 or 6 carbon atoms, which is unsubstituted or substituted by up to three identical or different alkyl groups having 1, 2, 3 or 4 carbon atoms,
or
A is the radical of a bicyclic system of the formulae 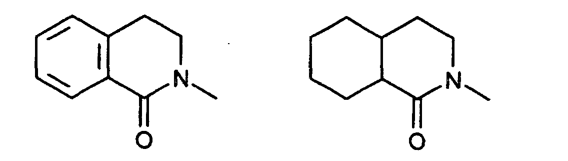 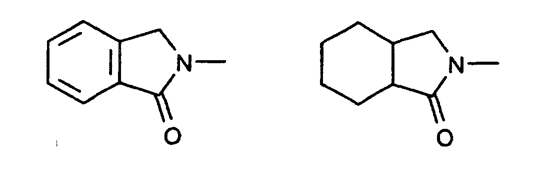 in any of its stereoisomeric forms or a mixture thereof in any ratio; or one of its physioliogically acceptable salts.

2. A compound of the formula I as claimed in claim 1, in which
R(1) is hydrogen, alkyl having 1 or 2 carbon atoms, alkoxy having 1, 2, 3 or 4 carbon atoms, alkylmercapto having 1, 2, 3 or 4 carbon atoms, fluorine, chlorine, bromine, iodine or trifluoromethyl;
R(2a), R(2b) and R(2c), which are identical or different, are hydrogen or alkyl having 1 or 2 carbon atoms;
R(3) is hydrogen or alkyl having 1, 2, 3 or 4 carbon atoms;
Z is sulfur or oxygen;
A is phenyl which is unsubstituted or substituted by up to three identical or different substituents selected from the group consisting of halogen, alkyl having 1 or 2 carbon atoms and alkoxy having 1 or 2 carbon atoms,
or
A is the radial of a saturated or unsaturated lactam of the formula 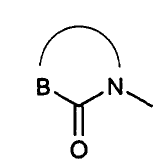 in which B is alkenylene or alkylene having 3, 4, 5 or 6 carbon atoms, which is unsubstituted or substituted by up to three identical or different alkyl groups having 1, 2, 3 or 4 carbon atoms,
or
A is the radical of a bicyclic system of the formulae 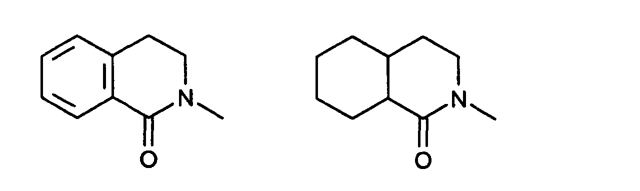 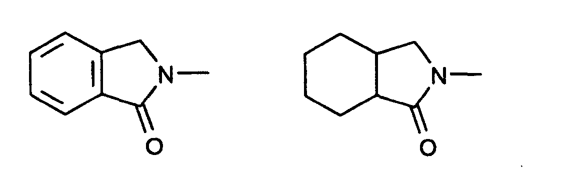 in any of its stereoisomeric forms or a mixture thereof in any ratio; or one of its physiologically acceptable salts.

3. A compound of the formula I as claimed in claim 1 and/or 2, in which
R(1) is hydrogen, alkyl having 1 or 2 carbon atoms, alkoxy having 1 or 2 carbon atoms, alkylmercapto having 1 or 2 carbon atoms, fluorine, chlorine, bromine, iodine or trifluoromethyl;
R(2a) is hydrogen and R(2b) and R(2c) are hydrogen or methyl;
R(3) is hydrogen or alkyl having 1, 2, 3 or 4 carbon atoms;
Z is sulfur or oxygen;
A is phenyl which is unsubstituted or substituted by up to three identical or different substituents selected from the group consisting of halogen, alkyl having 1 or 2 carbon atoms and alkoxy having 1 Or 2 carbon atoms,
or
A is the radical of a saturated or unsaturated lactam of the formula 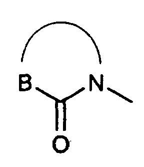 in which B is alkenylene or alkylene having 3, 4, 5 or 6 carbon atoms, which is unsubstituted or substituted by up to three identical or different alkyl groups having 1, 2, 3 or 4 carbon atoms,
or
A is the radical of a bicyclic system of the formulae 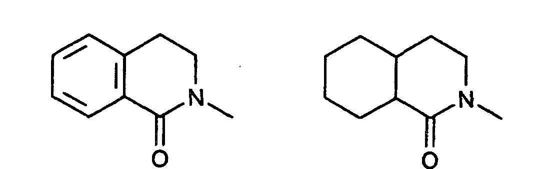 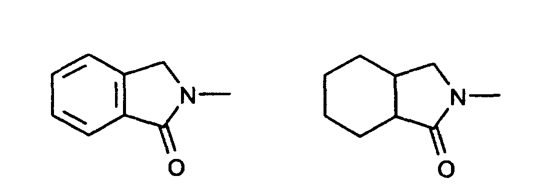 in any of its stereoisomeric forms or a mixture thereof in any ratio; or one of its physiologically acceptable salts.

4. A compound of the formula I as claimed in one or more of claims 1 to 3, in which
R(1) is hydrogen, alkyl having 1 or 2 carbon atoms, alkoxy having 1 or 2 carbon atoms or alkylmercapto having 1 or 2 carbon atoms;
R(2a), R(2b) and R(2c) are hydrogen;
R(3) is hydrogen or alkyl having 1, 2, 3 or 4 carbon atoms;
Z is sulfur;
A is phenyl which is unsubstituted or substituted by up to three identical or different substituents selected from the group consisti of halogen, alkyl having 1 or 2 carbon atoms and alkoxy having 1 2 carbon atoms,
or
A is the radical of a saturated or unsaturated lactam of the formula 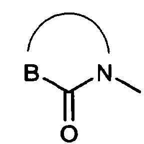 in which B is alkenylene or alkylene having 3, 4, 5 or 6 carbon atoms, which is unsubstituted or substituted by up to three identical or different alkyl groups having 1, 2, 3 or 4 carbon atoms,
or
A is the radical of a bicyclic system of the formulae 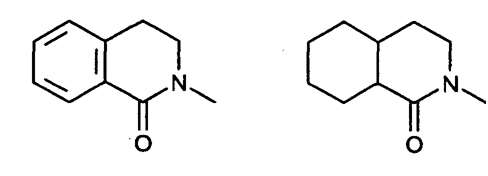 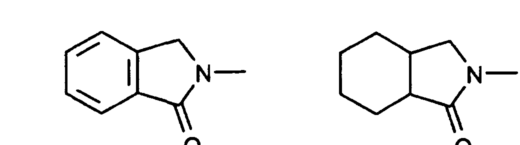 in any of its stereoisomeric forms or a mixture thereof in any ratio; or one of its physiologically acceptable salts.

5. A compound of the formula I as claimed in one or more of claims 1 to 4, in which
R(1) is hydrogen, alkyl having 1 or 2 carbon atoms, alkoxy having 1 or 2 carbon atoms or alkylmercapto having 1 or 2 carbon atoms;
R(2a), R(2b) and R(2c) are hydrogen;
R(3) is hydrogen, methyl or ethyl;
Z is sulfur;
A is phenyl which is unsubstituted or substituted by up to three identical or different substituents selected from the group consisting of halogen, alkyl having 1 or 2 carbon atoms and alkoxy having 1 or 2 carbon atoms,
or
A is the radical of a saturated or unsaturated lactam of the formula 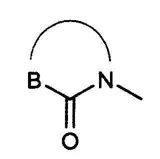 in which B is alkenylene or alkylene having 3, 4, 5 or 6 carbon atoms, which is unsubstituted or substituted by up to three identical or different alkyl groups having 1, 2, 3 or 4 carbon atoms;
in any of its stereoisomeric forms or a mixture thereof in any ratio; or one of its physiologically acceptable salts.

6. A compound of the formula I as claimed in one or more of claims 1 to 5, in which
R(1) is hydrogen, alkyl having 1 or 2 carbon atoms, alkoxy having 1 or 2 carbon atoms or alkylmercapto having 1 or 2 carbon atoms;
R(2a), R(2b) and R(2c) are hydrogen;
R(3) is hydrogen, methyl or ethyl;
Z is sulfur;
A is phenyl which is unsubstituted or substituted by up to three identical or different substituents selected from the group consisting of halogen, alkyl having 1 or 2 carbon atoms and alkoxy having 1 or 2 carbon atoms;
in any of its stereoisomeric forms or a mixture thereof in any ratio; or one of its physiologically acceptable salts.

7. A compound of the formula I as claimed in one or more of claims 1 to 3, in which
R(1) is hydrogen, alkyl having 1 or 2 carbon atoms, alkoxy having 1 or 2 carbon atoms or alkylmercapto having 1 or 2 carbon atoms;
R(2a), R(2b) and R(2c) are hydrogen;
R(3) is hydrogen or alkyl having 1, 2, 3 or 4 carbon atoms;
Z is oxygen;
A is phenyl which is unsubstituted or substituted by up to three identical or different substituents selected from the group consisting of halogen, alkyl having 1 or 2 carbon atoms and alkoxy having 1 or 2 carbon atoms,
or
A is the radical of a saturated or unsaturated lactam of the formula 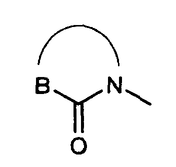 in which B is alkenylene or alkylene having 3, 4, 5 or 6 carbon atoms, which is unsubstituted or substituted by up to three identical or different alkyl groups having 1, 2, 3 or 4 carbon atoms,
or
A is the radical of a bicyclic system of the formulae 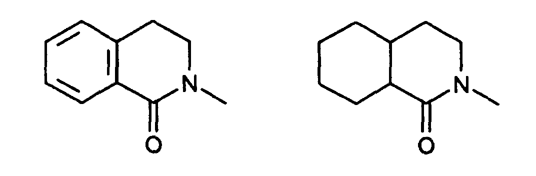 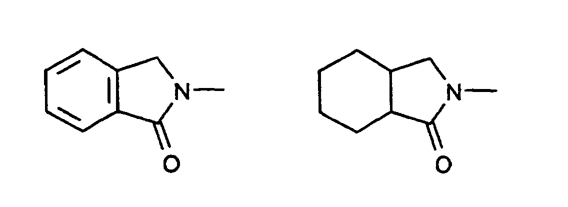 in any of its stereoisomeric forms or a mixture thereof in any ratio; or one of its physiologically acceptable salts.

8. A compound of the formula I as claimed in one or more of claims 1, 2, 3 and 7, in which
R(1) is hydrogen, alkyl having 1 or 2 carbon atoms, alkoxy having 1 or 2 carbon atoms or alkylmercapto having 1 or 2 carbon atoms;
R(2a), R(2b) and R(2c) are hydrogen;
R(3) is hydrogen, methyl or ethyl;
Z is oxygen;
A is phenyl which is unsubstituted or substituted by up to three identical or different substituents selected from the group consisting of halogen, alkyl having 1 or 2 carbon atoms and alkoxy having 1 or 2 carbon atoms,
or
A is the radical of a saturated or unsaturated lactam of the formula 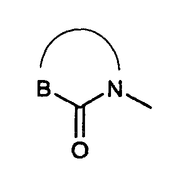 in which B is alkenylene or alkylene having 3, 4, 5 or 6 carbon atoms, which is unsubstituted or substituted by up to three identical or different alkyl groups having 1, 2, 3 or 4 carbon atoms;
in any of its stereoisomeric forms or a mixture thereof in any ratio; or one of its physiologically acceptable salts.

9. A compound of the formula I as claimed in one or more of claims 1, 2, 3, 7 and 8, in which
R(1) is hydrogen, alkyl having 1 or 2 carbon atoms, alkoxy having 1 or 2 carbon atoms or alkylmercapto having 1 or 2 carbon atoms;
R(2a), R(2b) and R(2c) are hydrogen;
R(3) is hydrogen, methyl or ethyl;
Z is oxygen;
A is phenyl which is unsubstituted or substituted by up to three identical or different substituents selected from the group consisting of halogen, alkyl having 1 or 2 carbon atoms and alkoxy having 1 or 2 carbon atoms;
in any of its stereoisomeric forms or a mixture thereof in any ratio; or one of its physiologically acceptable salts.

10. A process for the preparation of a compound of the formula I as claimed in one or more of claims 1 to 9, which comprises reacting a chromanylsulfonamide of the formula III 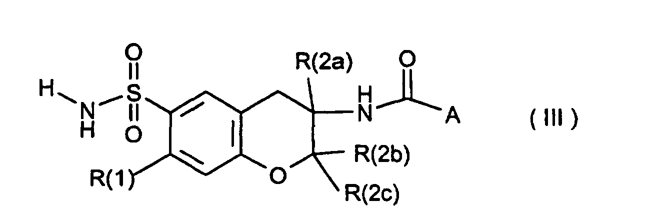 or a salt thereof of the formula IV 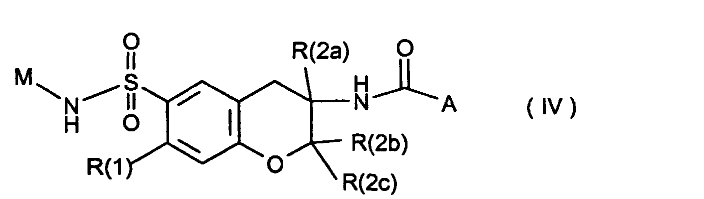 in which the radicals have the meanings given in claims 1 to 9 and the cation M is an alkali metal or alkaline earth metal ion or an ammonium ion, with an R(3)-substituted isocyanate or R(3)-substituted isothiocyanate, with an R(3)-substituted carbonic acid derivative or, to prepare a compound of the formula I in which Z is oxygen, with a trichloroacetamide substituted by R(3) on the nitrogen;
or, to prepare a compound of the formula I in which R(3) is hydrogen, reacting a compound of the formulae III or IV with a trialkylsilyl iso(thio)cyanate or silicon tetraiso(thio)cyanate and cleaving the primary silicon-substituted chromanylsulfonyl(thio)urea;
or, to prepare a compound of the formula I in which Z is oxygen, reacting a compound of the formula VIII 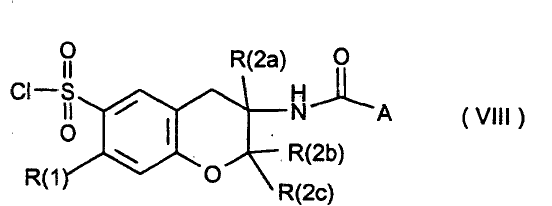 in which the radicals have the meanings given in claims 1 to 9, with an R(3)-substituted urea or an R(3)-substituted bis(trialkylsilyl)urea;
or reacting a compound of the formulae IX or X 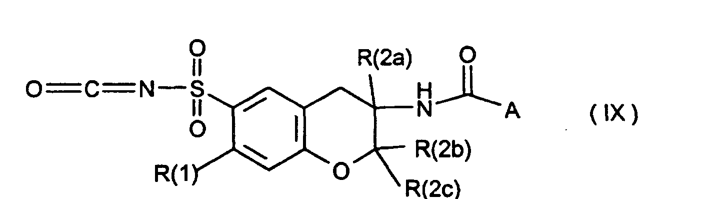 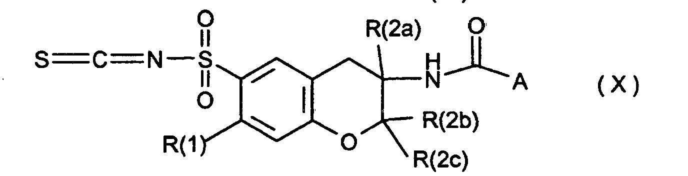 in which the radicals have the meanings given in claims 1 to 9, with an amine of the formula R(3)-NH₂, in which R(3) has the meanings given in claims 1 to 9;
or, to prepare a compound of the formula I in which Z is oxygen, desulfurizing a compound of the formula I in which Z is sulfur.

11. A compound of the formula I as claimed in one or more of claims 1 to 9 and/or one of its physiologically acceptable salts for use as a medicament.

12. The use of a compound of the formula I as claimed in one or more of claims 1 to 9 and/or of one of its physiologically acceptable salts for the preparation of a medicament for the treatment or prophylaxis of disturbances of the cardiovascular system, of cerebral vascular diseases, of ischemic states of the heart, of a weakened cardiac power, or for improving cardiac function after heart transplants.

13. The use of the compound of the formula I as claimed in one or more of claims 1 to 9 and/or of one of its physiologically acceptable salts for the preparation of a medicament for the treatment or prophylaxis of disturbances in cardiac rhythm or for preventing sudden cardiac death.

14. A pharmaceutical formulation which comprises an effective amount of at least one compound of the formula I as claimed in one or more of claims 1 to 9 and/or a physiologically acceptable salt thereof, in addition to pharmaceutically acceptable carriers and/or auxiliaries.

## Revendications

1. Composés de la formule I : 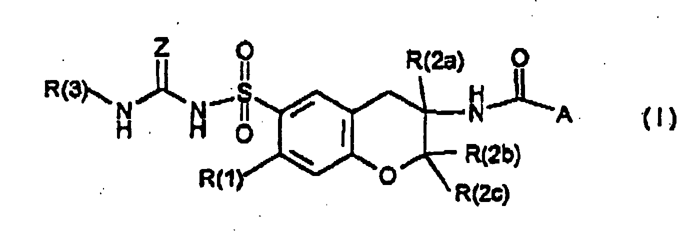 dans laquelle :
R(1) représente hydrogène, alkyle ayant 1, 2, 3 ou 4 atomes C, alcoxy ayant 1, 2, 3 ou 4 atomes C, alcoxyalcoxy ayant indépendamment l'un de l'autre, 1, 2, 3 ou 4 atomes C dans chacune des deux unités alcoxy, alkylmarcapto ayant 1, 2, 3 ou 4 atomes C, fluor, chlore, brome, iode ou trifluorométhyle ;
R(2a), R(2b) et R(2c), qui sont identiques ou différents, représentent hydrogène, alkyle ayant 1 ou 2 atomes C ;
R(3) représente hydrogène, alkyle ayant 1, 2, 3 ou 4 atomes C ;
Z représente soufre ou oxygène ;
A représente phényle, qui est non substitué ou substitué par jusqu'à trois substituants identiques ou différents, choisis parmi le groupe consistant en halogène, alkyle ayant 1 ou 2 atomes C ou alcoxy ayant 1 ou 2 atomes C, ou
A représente le reste d'un lactame saturé ou insaturé de la formule : 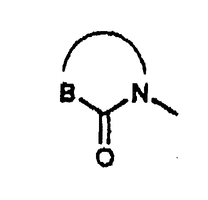
dans laquelle B représente un alcénylène ou alcylène ayant 3, 4, 5 ou 6 atomes C, qui est non substitué ou substitué par jusqu'à trois alkyle ayant 1, 2, 3 ou 4 atomes C identiques ou différents, ou
A représente le reste d'un système bicyclique des formules : 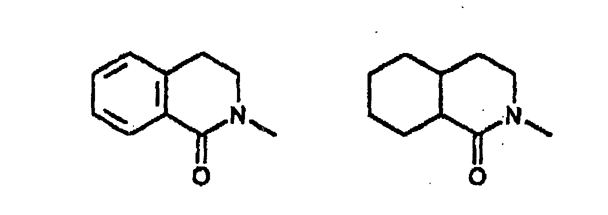 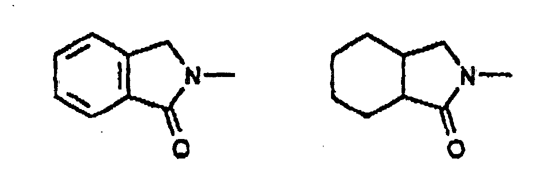
sous toutes leurs formes stéréoisomères et mélanges de ceux-ci en tout rapport, ainsi que leur sels physiologiquement compatibles.

2. Composés de la formule I selon la revendication 1, dans laquelle :
R(1) représente hydrogène, alkyle ayant 1 ou 2 atomes C, alcoxy ayant 1, 2, 3 ou 4 atomes C, alkylmarcapto ayant 1, 2, 3 ou 4 atomes C, fluor, chlore, brome, iode ou trifluorométhyle ;
R(2a), R(2b) et R(2c), qui sont identiques ou différents, représentent hydrogène, alkyle ayant 1 ou 2 atomes C ;
R(3) représente hydrogène, alkyle ayant 1, 2, 3 ou 4 atomes C ;
Z représente soufre ou oxygène ;
A représente phényle, qui est non substitué ou substitué par jusqu'à trois substituants identiques ou différents, choisis parmi le groupe consistant en halogène, alkyle ayant 1 ou 2 atomes C ou alcoxy ayant 1 ou 2 atomes C, ou
A représente le reste d'un lactame saturé ou insaturé de la formule : 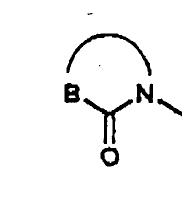
dans laquelle B représente un alcénylène ou alcylène ayant 3, 4, 5 ou 6 atomes C, qui est non substitué ou substitué par jusqu'à trois alkyle ayant 1, 2, 3 ou 4 atomes C identiques ou différents, ou
A représente le reste d'un système bicyclique des formules : 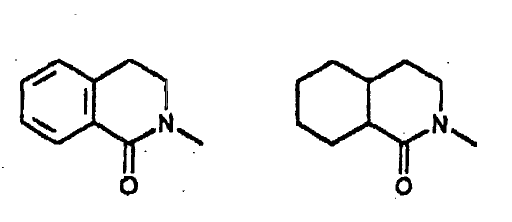 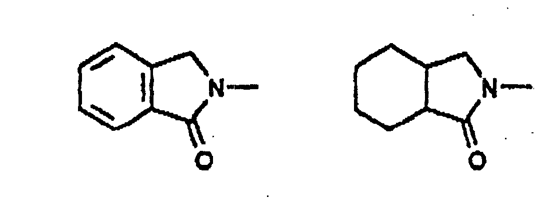
sous toutes leurs formes stéréoisomères et mélanges de ceux-ci en tout rapport, ainsi que leur sels physiologiquement compatibles.

3. Composés de la formule I selon la revendication 1 et/ou 2, dans laquelle :
R(1) représente hydrogène, alkyle ayant 1 ou 2 atomes C, alcoxy ayant 1 ou 2 atomes C, alkylmarcapto ayant 1 ou 2 atomes C, fluor, chlore, brome, iode ou trifluorométhyle ;
R(2a) représente hydrogène et R(2b) et R(2c), représentent hydrogène ou méthyle ;
R(3) représente hydrogène, alkyle ayant 1, 2, 3 ou 4 atomes C ;
Z représente soufre ou oxygène ;
A représente phényle, qui est non substitué ou substitué par jusqu'à trois substituants identiques ou différents, choisis parmi le groupe consistant en halogène, alkyle ayant 1 ou 2 atomes C ou alcoxy ayant 1 ou 2 atomes C, ou
A représente le reste d'un lactame saturé insaturé de la formule : 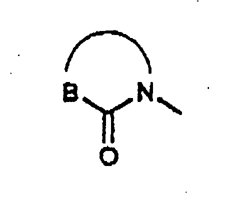
dans laquelle B représente un alcénylène ou alcylène ayant 3, 4, 5 ou 6 atomes C, qui est non substitué ou substitué par jusqu'à trois alkyle ayant 1, 2, 3 ou 4 atomes C identiques ou différents, ou
A représente le reste d'un système bicyclique des formules : 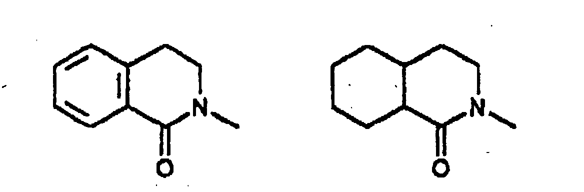 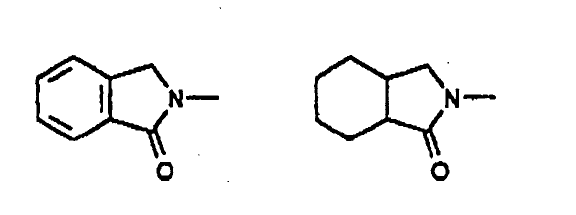
sous toutes leurs formes stéréoisomères et mélanges de ceux-ci en tout rapport, ainsi que leur sels physiologiquement compatibles.

4. Composés de la formule I selon l'une ou plusieurs des revendications 1 à 3, dans laquelle :
R(1) représente hydrogène, alkyle ayant 1 ou 2 atomes C, alcoxy ayant 1 ou 2 atomes C, alkylmarcapto ayant 1 ou 2 atomes C ;
R(2a), R(2b) et R(2c) représentent hydrogène ;
R(3) représente hydrogène ou alkyle ayant 1, 2, 3 ou 4 atomes C ;
Z représente soufre ;
A représente phényle, qui est non substitué ou substitué par jusqu'à trois substituants identiques ou différents, choisis parmi le groupe consistant en halogène, alkyle ayant 1 ou 2 atomes C ou alcoxy ayant 1 ou 2 atomes C, ou
A représente le reste d'un lactame saturé ou insaturé de la formule : 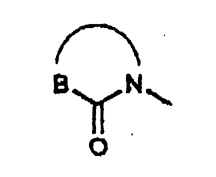
dans laquelle B représente un alcénylène ou alcylène ayant 3, 4, 5 ou 6 atomes C, qui est non substitué ou substitué par jusqu'à trois alkyle ayant 1, 2, 3 ou 4 atomes C identiques ou différents, ou
A représente le reste d'un système bicyclique des formules : 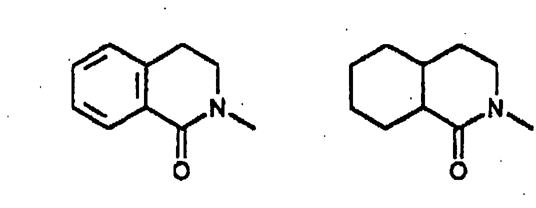 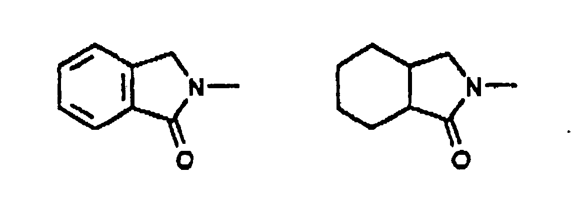
sous toutes leurs formes stéréoisomères et mélanges de ceux-ci en tout rapport, ainsi que leur sels physiologiquement compatibles.

5. Composés de la formule I selon l'une ou plusieurs des revendications 1 à 4, dans laquelle :
R(1) représente hydrogène, alkyle ayant 1 ou 2 atomes C, alcoxy ayant 1 ou 2 atomes C, alkylmarcapto ayant 1 ou 2 atomes C ;
R(2a), R(2b) et R(2c) représentent hydrogène ;
R(3) représente hydrogène, méthyle ou éthyle ;
Z représente soufre ;
A représente phényle, qui est non substitué ou substitué par jusqu'à trois substituants identiques ou différents, choisis parmi le groupe consistant en halogène, alkyle ayant 1 ou 2 atomes C ou alcoxy ayant 1 ou 2 atomes C, ou
A représente le reste d'un lactame saturé ou insaturé de la formule : 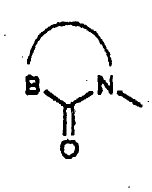
dans laquelle B représente un alcénylène ou alcylène ayant 3, 4, 5 ou 6 atomes C, qui est non substitué ou substitué par jusqu'à trois alkyle ayant 1, 2, 3 ou 4 atomes C identiques ou différents ;
sous toutes leurs formes stéréoisomères et mélanges de ceux-ci en tout rapport, ainsi que leur sels physiologiquement compatibles.

6. Composés de la formule I selon l'une ou plusieurs des revendications 1 à 5, dans laquelle :
R(1) représente hydrogène, alkyle ayant 1 ou 2 atomes C, alcoxy ayant 1 ou 2 atomes C, alkylmarcapto ayant 1 ou 2 atomes C ;
R(2a), R(2b) et R(2c) représentent hydrogène ;
R(3) représente hydrogène, méthyle ou éthyle ;
Z représente soufre ;
A représente phényle, qui est non substitué ou substitué par jusqu'à trois substituants identiques ou différents, choisis parmi le groupe consistant en halogène, alkyle ayant 1 ou 2 atomes C ou alcoxy ayant 1 ou 2 atomes C ;
sous toutes leurs formes stéréoisomères et mélanges de ceux-ci en tout rapport, ainsi que leur sels physiologiquement compatibles.

7. Composés de la formule I selon l'une ou plusieurs des revendications 1 à 3, dans laquelle :
R(1) représente hydrogène, alkyle ayant 1 ou 2 atomes C, alcoxy ayant 1 ou 2 atomes C, alkylmarcapto ayant 1 ou 2 atomes C ;
R(2a), R(2b) et R(2c) représentent hydrogène ;
R(3) représente hydrogène ou alkyle ayant 1, 2, 3 ou 4 atomes C ;
Z représente oxygène ;
A représente phényle, qui est non substitué ou substitué par jusqu'à trois substituants identiques ou différents, choisis parmi le groupe consistant en halogène, alkyle ayant 1 ou 2 atomes C ou alcoxy ayant 1 ou 2 atomes C, ou
A représente le reste d'un lactame saturé ou insaturé de la formule : 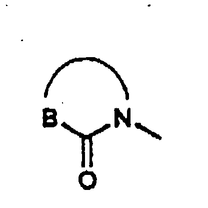
dans laquelle B représente un alcénylène ou alcylène ayant 3, 4, 5 ou 6 atomes C, qui est non substitué ou substitué par jusqu'à trois alkyle ayant 1, 2, 3 ou 4 atomes C identiques ou différents, ou
A représente le reste d'un système bicyclique des formules : 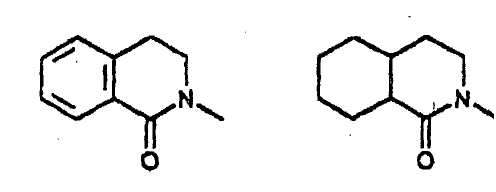 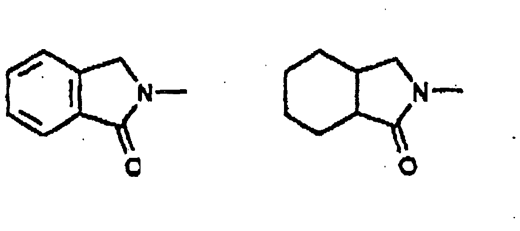
sous toutes leurs formes stéréoisomères et mélanges de ceux-ci en tout rapport, ainsi que leur sels physiologiquement compatibles.

8. Composés de la formule I selon l'une ou plusieurs des revendications 1, 2, 3 et 7, dans laquelle :
R(1) représente hydrogène, alkyle ayant 1 ou 2 atomes C, alcoxy ayant 1 ou 2 atomes C, alkylmarcapto ayant 1 ou 2 atomes C ;
R(2a), R(2b) et R(2c) représentent hydrogène ;
R(3) représente hydrogène, méthyle ou éthyle ;
Z représente oxygène ;
A représente phényle, qui est non substitué ou substitué par jusqu'à trois substituants identiques ou différents, choisis parmi le groupe consistant en halogène, alkyle ayant 1 ou 2 atomes C ou alcoxy ayant 1 ou 2 atomes C, ou
A représente le reste d'un lactame saturé ou insaturé de la formule : 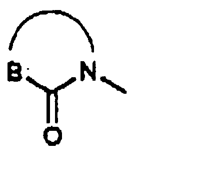
dans laquelle B représente un alcénylène ou alcylène ayant 3, 4, 5 ou 6 atomes C, qui est non substitué ou substitué par jusqu'à trois alkyle ayant 1, 2, 3 ou 4 atomes C identiques ou différents ;
sous toutes leurs formes stéréoisomères et mélanges de ceux-ci en tout rapport, ainsi que leur sels physiologiquement compatibles.

9. Composés de la formule I selon l'une ou plusieurs des revendications 1, 2, 3, 7 et 8, dans laquelle :
R(1) représente hydrogène, alkyle ayant 1 ou 2 atomes C, alcoxy ayant 1 ou 2 atomes C, alkylmarcapto ayant 1 ou 2 atomes C ;
R(2a), R(2b) et R(2c) représentent hydrogène ;
R(3) représente hydrogène, méthyle ou éthyle ;
Z représente oxygène ;
A représente phényle, qui est non substitué ou substitué par jusqu'à trois substituants identiques ou différents, choisis parmi le groupe consistant en halogène, alkyle ayant 1 ou 2 atomes C ou alcoxy ayant 1 ou 2 atomes C ;
sous toutes leurs formes stéréoisomères et mélanges de ceux-ci en tout rapport, ainsi que leur sels physiologiquement compatibles.

10. Procédé de préparation de composés de la formule I, selon une ou plusieurs des revendications 1 à 9, **caractérisé en ce que** l'on fait réagir chromanylsulfonamide de la formule III : 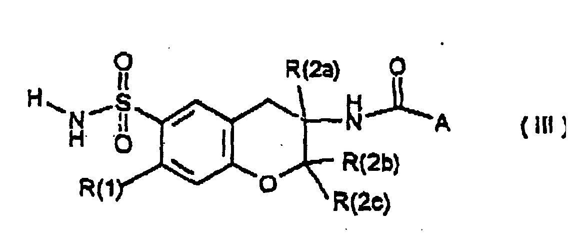 ou ses sels de la formule IV : 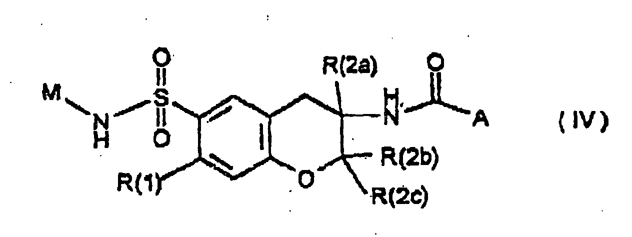 dans lesquelles les restes ont les significations indiquées dans les revendications 1 à 9 et le cation M représente un ion alcalin, alcalino-terreux ou un ion ammonium, avec un isocyanate substitué par R(3) ou isothiocyanate substitué par R(3), avec un dérivé d'acide carbonique substitué par R(3) ou pour la préparation de composés de la formule I, dans laquelle Z représente oxygène; avec un trichloracétamide substitué sur l'azote par R(3) ;
ou pour la préparation de composés de la formule I, dans laquelle R(3) représente hydrogène, on fait réagir les composés des formules III ou IV avec un iso(thio)cyanate de trialkylsilyle ou tétraiso (thio)-cyanate de silicium et on clive la chromanylsulfonyl-(thio)urée substituée par un silicium primaire ;
ou pour la préparation de composés de la formule I, dans laquelle Z représente oxygène, on fait réagir un composé de la formule VIII : 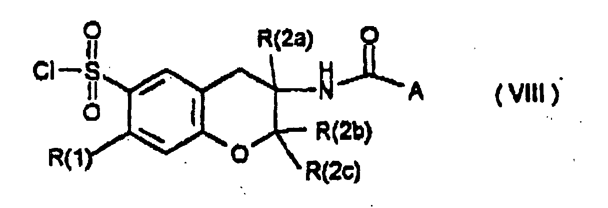 dans lesquelles les restes ont les significations indiquées dans les revendications 1 à 9, avec une urée substituée par R(3) ou une bis(trialkylsilyl)urée substitué par R(3),
ou on fait réagir les composés des formules IX ou X : 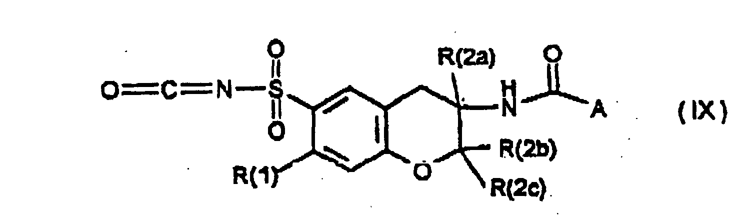 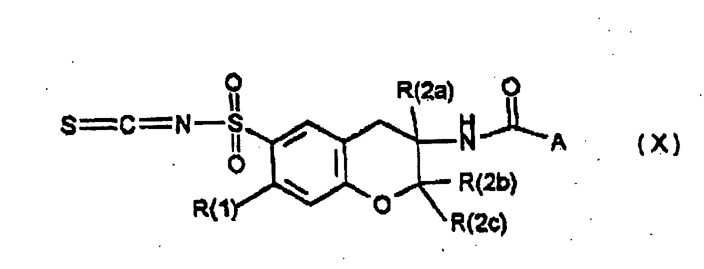 dans lesquelles les restes ont les significations indiquées dans les revendications 1 à 9, avec une amine de la formule R(3)-NH₂, dans laquelle R(3) a les significations indiquées dans les revendications 1 à 9 ;
ou pour la préparation de composés de la formule I, dans laquelle Z représente oxygène, on désulfure des composés de la formule I, dans laquelle Z représente soufre.

11. Composés de la formule I, selon une ou plusieurs des revendications 1 à 9, et/ou leurs sels physiologiquement compatibles à utiliser comme agent pharmaceutique.

12. Utilisation de composés de la formule I, selon une ou plusieurs des revendications 1 à 9, et/ou leurs sels physiologiquement compatibles pour la préparation d'un médicament pour le traitement ou la prophylaxie de troubles du système cardiovasculaire, d'accidents vasculaires cérébraux, d'états ischémiques du coeur, d'une faiblesse du coeur ou pour améliorer la fonction cardiaque après transplantation cardiaque.

13. Utilisation de composés de la formule I, selon une ou plusieurs des revendications 1 à 9, et/ou leurs sels physiologiquement compatibles pour la préparation d'un médicament pour la traitement ou la prophylaxie de troubles du rythme cardiaque ou pour empêcher des arrêts cardiaques soudains.

14. Composition pharmaceutique, **caractérisée en ce qu'**elle contient une quantité active d'au moins un composé de la formule I, selon une ou plusieurs des revendications 1 à 9, et/ou un sel physiologiquement compatible de celui-ci, avec des supports et/ou auxiliaires pharmaceutiquement acceptables.
